# EUROPEAN PATENT APPLICATION

(11) **EP 3 763 737 A1**
(43) Date of publication of application: **13.01.2021**
(21) Application number: 19185969.3
(22) Date of filing: 12.07.2019
(51) Int. Cl.: C07K 16/12

(54) **BINDING COMPOUNDS OF PENICILLIN BINDING PROTEIN 2 A**

(71) Applicant: Eberhard Karls Universität Tübingen, 72074 Tübingen (DE)
(72) Inventor: Götz, Friedrich, 72076 Tübingen (DE); Werner, Rolf, 88400 Biberach (DE); Jittavisutthikul, Surasak, 72070 Tübingen (DE)
(74) Representative: Kuttenkeuler, David

(57) **Abstract**

The invention pertains to protein binding compounds such as antibodies, nanobodies, or antigen-binding fragments thereof, targeting penicillin-binding protein 2a (PBP2a) to increase the susceptibility of resistant pathogens to β-lactam antibiotics. The invention provides protein binding compounds (PBC) specifically binding to the transpeptidase domain of PBP2a, nucleic acids encoding therefor, recombinant cells expressing them, and pharmaceutical compositions comprising such compounds of the invention. The products of the invention are preferably for use in medicine such as for treating or preventing infections of antibiotic resistant-pathogens, such as methicillin-resistant *Staphylococcus aureus* (MRSA). Furthermore, the products of the invention find use in diagnostic applications for the detection of pathogenic organisms such as MRSA. Besides, there is provided a method for the identification of therapeutics based on the binding of such candidate compounds to the transpeptidase domain of PBP2a.

SEQUENCE LISTING

<110>  Eberhard Karls Universität Tübingen

<120>  BINDING COMPOUNDS OF PENICILLIN BINDING PROTEIN 2 A

<130>  U30916EP

<160>  394

<170>  PatentIn version 3.5

<210>  1
<211>  25
<212>  PRT
<213>  Homo sapiens

<400>  1

Gln Val Gln Leu Gln Glu Ser Gly Pro Gly Leu Val Lys Pro Ser Glu
1               5                   10                  15

Thr Leu Ser Leu Thr Cys Thr Val Ser
            20                  25

<210>  2
<211>  10
<212>  PRT
<213>  Homo sapiens

<400>  2

Gly Gly Ser Ile Asn Thr His Ser Tyr Tyr
1               5                   10

<210>  3
<211>  17
<212>  PRT
<213>  Homo sapiens

<400>  3

Trp Leu Trp Ile Arg Gln Pro Pro Gly Lys Gly Leu Glu Trp Val Gly
1               5                   10                  15

Ser

<210>  4
<211>  7
<212>  PRT
<213>  Homo sapiens

<400>  4

Ile Phe His Gly Gly Ser Ala
1               5

<210>  5
<211>  38
<212>  PRT
<213>  Homo sapiens

<400>  5

Phe Tyr Ser Pro Ser Leu Lys Gly Arg Val Thr Ile Ser Val Asp Thr
1               5                   10                  15

Ser Arg Gly Gln Phe Ser Leu Asn Leu Met Ser Val Thr Ala Ala Asp
            20                  25                  30

Thr Ala Val Tyr Tyr Cys
        35

<210>  6
<211>  19
<212>  PRT
<213>  Homo sapiens

<400>  6

Val Arg Gln Arg Gly Leu Ala Pro Ala Gly Thr Tyr Tyr Tyr Tyr Gly
1               5                   10                  15

Leu Asp Ala

<210>  7
<211>  11
<212>  PRT
<213>  Homo sapiens

<400>  7

Trp Gly Gln Gly Thr Thr Val Thr Val Ser Ser
1               5                   10

<210>  8
<211>  127
<212>  PRT
<213>  Homo sapiens

<400>  8

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Asn Thr His
            20                  25                  30

Ser Tyr Tyr Trp Leu Trp Ile Arg Gln Pro Pro Gly Lys Gly Leu Glu
        35                  40                  45

Trp Val Gly Ser Ile Phe His Gly Gly Ser Ala Phe Tyr Ser Pro Ser
    50                  55                  60

Leu Lys Gly Arg Val Thr Ile Ser Val Asp Thr Ser Arg Gly Gln Phe
65                  70                  75                  80

Ser Leu Asn Leu Met Ser Val Thr Ala Ala Asp Thr Ala Val Tyr Tyr
                85                  90                  95

Cys Val Arg Gln Arg Gly Leu Ala Pro Ala Gly Thr Tyr Tyr Tyr Tyr
            100                 105                 110

Gly Leu Asp Ala Trp Gly Gln Gly Thr Thr Val Thr Val Ser Ser
        115                 120                 125

<210>  9
<211>  26
<212>  PRT
<213>  Homo sapiens

<400>  9

Val Ile Trp Met Thr Gln Ser Pro Asp Ser Leu Ala Val Ser Leu Gly
1               5                   10                  15

Glu Arg Ala Thr Ile Asn Cys Lys Ser Ser
            20                  25

<210>  10
<211>  12
<212>  PRT
<213>  Homo sapiens

<400>  10

Gln Ser Leu Leu Tyr Ser Ser Lys Asn Lys Asp Tyr
1               5                   10

<210>  11
<211>  17
<212>  PRT
<213>  Homo sapiens

<400>  11

Leu Thr Trp Tyr Gln Gln Lys Pro Gly Gln Pro Pro Lys Leu Leu Ile
1               5                   10                  15

Tyr

<210>  12
<211>  3
<212>  PRT
<213>  Homo sapiens

<400>  12

Trp Ala Ser
1


<210>  13
<211>  36
<212>  PRT
<213>  Homo sapiens

<400>  13

Thr Arg Ala Ser Gly Val Pro Asp Arg Phe Ser Gly Ser Val Ser Gly
1               5                   10                  15

Thr Ile Phe Thr Leu Thr Ile Ser Ser Leu Gln Ala Glu Asp Val Ala
            20                  25                  30

Val Tyr Tyr Cys
        35


<210>  14
<211>  9
<212>  PRT
<213>  Homo sapiens

<400>  14

Gln Gln Tyr Tyr Ser Thr Pro Tyr Thr
1               5


<210>  15
<211>  10
<212>  PRT
<213>  Homo sapiens

<400>  15

Phe Gly Gln Gly Thr Lys Val Glu Ile Lys
1               5                   10


<210>  16
<211>  113
<212>  PRT
<213>  Homo sapiens

<400>  16

Val Ile Trp Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Tyr Ser
            20                  25                  30

Ser Lys Asn Lys Asp Tyr Leu Thr Trp Tyr Gln Gln Lys Pro Gly Gln
        35                  40                  45

Pro Pro Lys Leu Leu Ile Tyr Trp Ala Ser Thr Arg Ala Ser Gly Val
    50                  55                  60

Pro Asp Arg Phe Ser Gly Ser Val Ser Gly Thr Ile Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Thr Pro Tyr Thr Phe Gly Gln Gly Thr Lys Val Glu Ile
            100                 105                 110

Lys

<210>  17
<211>  25
<212>  PRT
<213>  Homo sapiens

<400>  17

<210>  18
<211>  8
<212>  PRT
<213>  Homo sapiens

<400>  18

Gly Gly Thr Phe Ser Ser Tyr Ala
1               5

<210>  19
<211>  17
<212>  PRT
<213>  Homo sapiens

<400>  19

Ile Ser Trp Val Arg Gln Ala Pro Gly Gln Gly Leu Glu Trp Met Gly
1               5                   10                  15

Arg

<210> 20
<211> 8
<212> PRT
<213> Homo sapiens

<400> 20

Ile Ile Pro Ile Leu Gly Ile Ala
1               5

<210> 21
<211> 38
<212> PRT
<213> Homo sapiens

<400> 21

Asn Tyr Ala Gln Lys Phe Gln Gly Arg Val Thr Ile Thr Ala Asp Lys
1               5                   10                  15

Ser Thr Ser Thr Ala Tyr Met Glu Leu Ser Ser Leu Arg Ser Glu Asp
            20                  25                  30

Thr Ala Val Tyr Tyr Cys
        35

<210> 22
<211> 23
<212> PRT
<213> Homo sapiens

<400> 22

Ala Arg Arg Leu Leu Pro Thr Tyr Ser Ser Ser Trp Ser Asp His Tyr
1               5                   10                  15

Tyr Tyr Tyr Gly Met Asp Val
            20

<210> 23
<211> 11
<212> PRT
<213> Homo sapiens

<400> 23

Trp Gly Gln Gly Thr Thr Val Thr Val Ser Ser
1               5                   10

<210> 24

<211>  130
<212>  PRT
<213>  Homo sapiens

<400>  24

Gln Val Gln Leu Val Gln Ser Gly Ala Glu Val Lys Lys Pro Gly Ser
1               5                   10                  15

Ser Val Lys Val Ser Cys Lys Ala Ser Gly Gly Thr Phe Ser Ser Tyr
            20                  25                  30

Ala Ile Ser Trp Val Arg Gln Ala Pro Gly Gln Gly Leu Glu Trp Met
        35                  40                  45

Gly Arg Ile Ile Pro Ile Leu Gly Ile Ala Asn Tyr Ala Gln Lys Phe
    50                  55                  60

Gln Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Arg Leu Leu Pro Thr Tyr Ser Ser Ser Trp Ser Asp His Tyr
            100                 105                 110

Tyr Tyr Tyr Gly Met Asp Val Trp Gly Gln Gly Thr Thr Val Thr Val
        115                 120                 125

Ser Ser
    130


<210>  25
<211>  26
<212>  PRT
<213>  Homo sapiens

<400>  25

Ala Ile Gln Leu Thr Gln Ser Pro Ser Phe Leu Ser Ala Ser Val Gly
1               5                   10                  15

Asp Arg Val Thr Ile Thr Cys Arg Ala Ser
            20                  25


<210>  26
<211>  6
<212>  PRT
<213>  Homo sapiens

<400>  26

Gln Gly Val Ser Asn Tyr
1               5

<210>  27
<211>  17
<212>  PRT
<213>  Homo sapiens

<400>  27

Leu Val Trp Tyr Gln Gln Lys Pro Gly Lys Ala Pro Lys Leu Leu Ile
1               5                   10                  15

Ser

<210>  28
<211>  3
<212>  PRT
<213>  Homo sapiens

<400>  28

Ala Ala Ser
1

<210>  29
<211>  36
<212>  PRT
<213>  Homo sapiens

<400>  29

Thr Leu Gln Ser Gly Val Pro Ser Arg Phe Ser Gly Ser Gly Ser Gly
1               5                   10                  15

Thr Glu Phe Thr Leu Thr Ile Ser Ser Leu Gln Pro Glu Asp Phe Ala
            20                  25                  30

Thr Tyr Phe Cys
        35

<210>  30
<211>  9
<212>  PRT
<213>  Homo sapiens

<400>  30

Gln Gln Leu Ser Ile Tyr Pro Ile Thr
1               5

<210>  31
<211>  10

<212>   PRT
<213>   Homo sapiens

<400>   31

Phe Gly Pro Gly Thr Lys Leu Asp Ile Lys
1               5                   10


<210>   32
<211>   107
<212>   PRT
<213>   Homo sapiens

<400>   32

Ala Ile Gln Leu Thr Gln Ser Pro Ser Phe Leu Ser Ala Ser Val Gly
1               5                   10                  15


Asp Arg Val Thr Ile Thr Cys Arg Ala Ser Gln Gly Val Ser Asn Tyr
            20                  25                  30


Leu Val Trp Tyr Gln Gln Lys Pro Gly Lys Ala Pro Lys Leu Leu Ile
        35                  40                  45


Ser Ala Ala Ser Thr Leu Gln Ser Gly Val Pro Ser Arg Phe Ser Gly
    50                  55                  60


Ser Gly Ser Gly Thr Glu Phe Thr Leu Thr Ile Ser Ser Leu Gln Pro
65                  70                  75                  80


Glu Asp Phe Ala Thr Tyr Phe Cys Gln Gln Leu Ser Ile Tyr Pro Ile
                85                  90                  95


Thr Phe Gly Pro Gly Thr Lys Leu Asp Ile Lys
            100                 105


<210>   33
<211>   25
<212>   PRT
<213>   Homo sapiens

<400>   33


<210>   34
<211>   8
<212>   PRT
<213>   Homo sapiens

<400>  34

Gly Gly Ser Phe Ser Gly Tyr Tyr
1               5

<210>  35
<211>  17
<212>  PRT
<213>  Homo sapiens

<400>  35

Trp Ser Trp Ile Arg Gln Pro Pro Gly Lys Gly Leu Glu Trp Ile Gly
1               5                   10                  15

Glu

<210>  36
<211>  7
<212>  PRT
<213>  Homo sapiens

<400>  36

Ile Asn His Ser Gly Ser Thr
1               5

<210>  37
<211>  38
<212>  PRT
<213>  Homo sapiens

<400>  37

Asn Tyr Asn Pro Ser Leu Lys Ser Arg Val Thr Ile Ser Val Asp Thr
1               5                   10                  15

Ser Lys Asn Gln Phe Ser Leu Lys Leu Ser Ser Val Thr Ala Ala Asp
            20                  25                  30

Thr Ala Val Tyr Tyr Cys
        35

<210>  38
<211>  22
<212>  PRT
<213>  Homo sapiens

<400>  38

Ala Arg Glu Asp Arg Asp Ile Val Val Val Pro Ala Ala Ile Arg Trp
1               5                   10                  15

Gly Asn Trp Phe Asp Pro
            20

<210>  39
<211>  11
<212>  PRT
<213>  Homo sapiens

<400>  39

Trp Gly Gln Gly Thr Leu Val Thr Val Ser Ser
1               5                   10

<210>  40
<211>  128
<212>  PRT
<213>  Homo sapiens

<400>  40

Gln Val Gln Leu Gln Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asn His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Glu Asp Arg Asp Ile Val Val Val Pro Ala Ala Ile Arg Trp Gly
            100                 105                 110

Asn Trp Phe Asp Pro Trp Gly Gln Gly Thr Leu Val Thr Val Ser Ser
        115                 120                 125

<210>  41
<211>  26
<212>  PRT
<213>  Homo sapiens

<400>  41

Thr Ile Gln Leu Thr Gln Ser Pro Ser Thr Leu Ser Ala Ser Ile Gly
1               5                   10                  15

Asp Arg Val Thr Ile Thr Cys Arg Ala Ser
            20                  25

<210>  42
<211>  6
<212>  PRT
<213>  Homo sapiens

<400>  42

Gln Ser Ile Ser Asp Trp
1               5

<210>  43
<211>  17
<212>  PRT
<213>  Homo sapiens

<400>  43

Leu Ala Trp Tyr Gln Gln Lys Pro Gly Lys Ala Pro Lys Leu Leu Ile
1               5                   10                  15

Gln

<210>  44
<211>  3
<212>  PRT
<213>  Homo sapiens

<400>  44

Lys Ala Ser
1

<210>  45
<211>  36
<212>  PRT
<213>  Homo sapiens

<400>  45

Ser Leu Glu Ser Gly Val Pro Ser Arg Phe Ser Gly Arg Gly Ser Gly
1               5                   10                  15

Thr Glu Phe Thr Leu Thr Ile Ser Ser Leu Gln Pro Asp Asp Phe Ala
            20                  25                  30

Thr Tyr Tyr Cys
        35

<210>  46

<211>  9
<212>  PRT
<213>  Homo sapiens

<400>  46

Gln Gln Tyr Asn Ser Tyr Ser Leu Thr
1               5


<210>  47
<211>  10
<212>  PRT
<213>  Homo sapiens

<400>  47

Phe Gly Gly Gly Thr Lys Leu Asp Ile Lys
1               5                   10


<210>  48
<211>  107
<212>  PRT
<213>  Homo sapiens

<400>  48

Thr Ile Gln Leu Thr Gln Ser Pro Ser Thr Leu Ser Ala Ser Ile Gly
1               5                   10                  15


Asp Arg Val Thr Ile Thr Cys Arg Ala Ser Gln Ser Ile Ser Asp Trp
            20                  25                  30


Leu Ala Trp Tyr Gln Gln Lys Pro Gly Lys Ala Pro Lys Leu Leu Ile
        35                  40                  45


Gln Lys Ala Ser Ser Leu Glu Ser Gly Val Pro Ser Arg Phe Ser Gly
    50                  55                  60


Arg Gly Ser Gly Thr Glu Phe Thr Leu Thr Ile Ser Ser Leu Gln Pro
65                  70                  75                  80


Asp Asp Phe Ala Thr Tyr Tyr Cys Gln Gln Tyr Asn Ser Tyr Ser Leu
                85                  90                  95


Thr Phe Gly Gly Gly Thr Lys Leu Asp Ile Lys
            100                 105


<210>  49
<211>  25
<212>  PRT
<213>  Homo sapiens

<400>  49

Gln Val Gln Leu Gln Glu Ser Gly Pro Gly Leu Val Lys Pro Ser Glu
1               5                   10                  15

Thr Leu Ser Leu Thr Cys Thr Val Ser
            20                  25

<210>  50
<211>  8
<212>  PRT
<213>  Homo sapiens

<400>  50

Gly Gly Ser Ile Ser Ser Tyr Tyr
1               5

<210>  51
<211>  17
<212>  PRT
<213>  Homo sapiens

<400>  51

Trp Ser Trp Ile Arg Gln Pro Ala Gly Lys Gly Leu Glu Trp Ile Gly
1               5                   10                  15

Arg

<210>  52
<211>  7
<212>  PRT
<213>  Homo sapiens

<400>  52

Ile Tyr Thr Ser Gly Ser Thr
1               5

<210>  53
<211>  38
<212>  PRT
<213>  Homo sapiens

<400>  53

Asn Tyr Asn Pro Phe Leu Lys Ser Arg Val Thr Met Ser Val Asp Thr
1               5                   10                  15

Ser Lys Asn Gln Phe Ser Leu Lys Leu Ser Ser Glu Thr Ala Ala Asp
            20                  25                  30

Thr Ala Val Tyr Tyr Cys
        35

<210>  54
<211>  16
<212>  PRT
<213>  Homo sapiens

<400>  54

Ala Cys Thr Gly Tyr Ser Ser Ser Trp Pro Tyr Trp Tyr Phe Asp Leu
1               5                   10                  15


<210>  55
<211>  11
<212>  PRT
<213>  Homo sapiens

<400>  55

Trp Gly Arg Gly Thr Leu Val Thr Val Ser Ser
1               5                   10


<210>  56
<211>  122
<212>  PRT
<213>  Homo sapiens

<400>  56

Gln Val Gln Leu Gln Glu Ser Gly Pro Gly Leu Val Lys Pro Ser Glu
1               5                   10                  15


Thr Leu Ser Leu Thr Cys Thr Val Ser Gly Gly Ser Ile Ser Ser Tyr
            20                  25                  30


Tyr Trp Ser Trp Ile Arg Gln Pro Ala Gly Lys Gly Leu Glu Trp Ile
        35                  40                  45


Gly Arg Ile Tyr Thr Ser Gly Ser Thr Asn Tyr Asn Pro Phe Leu Lys
    50                  55                  60


Ser Arg Val Thr Met Ser Val Asp Thr Ser Lys Asn Gln Phe Ser Leu
65                  70                  75                  80


Lys Leu Ser Ser Glu Thr Ala Ala Asp Thr Ala Val Tyr Tyr Cys Ala
                85                  90                  95


Cys Thr Gly Tyr Ser Ser Ser Trp Pro Tyr Trp Tyr Phe Asp Leu Trp
            100                 105                 110


Gly Arg Gly Thr Leu Val Thr Val Ser Ser
        115                 120


<210>  57

<211>  26
<212>  PRT
<213>  Homo sapiens

<400>  57

Thr Ile Arg Met Thr Gln Ser Pro Ser Ser Leu Ser Ala Ser Val Gly
1               5                   10                  15

Asp Arg Val Thr Ile Thr Cys Arg Ala Ser
            20                  25

<210>  58
<211>  6
<212>  PRT
<213>  Homo sapiens

<400>  58

Gln Ser Ile Ser Ser Tyr
1               5

<210>  59
<211>  17
<212>  PRT
<213>  Homo sapiens

<400>  59

Leu Asn Trp Tyr Gln Gln Lys Pro Gly Lys Ala Pro Lys Leu Leu Ile
1               5                   10                  15

Tyr

<210>  60
<211>  3
<212>  PRT
<213>  Homo sapiens

<400>  60

Gly Ala Ser
1

<210>  61
<211>  36
<212>  PRT
<213>  Homo sapiens

<400>  61

Ser Leu Gln Ser Gly Val Pro Ser Arg Phe Ser Gly Gly Gly Ser Gly
1               5                   10                  15

Thr Asp Phe Thr Leu Thr Ile Ser Ser Leu Gln Pro Glu Asp Phe Ala

20                  25                  30

Thr Tyr Tyr Cys
        35

<210>  62
<211>  9
<212>  PRT
<213>  Homo sapiens

<400>  62

Gln Gln Ser Tyr Ser Thr Pro Ile Thr
1               5

<210>  63
<211>  10
<212>  PRT
<213>  Homo sapiens

<400>  63

Phe Gly Gly Gly Thr Lys Leu Glu Ile Lys
1               5                   10

<210>  64
<211>  107
<212>  PRT
<213>  Homo sapiens

<400>  64

Thr Ile Arg Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Gly Ala Ser Ser Leu Gln Ser Gly Val Pro Ser Arg Phe Ser Gly
    50                  55                  60

Gly Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Ile
                85                  90                  95

Thr Phe Gly Gly Gly Thr Lys Leu Glu Ile Lys
            100                 105

<210>  65
<211>  25
<212>  PRT
<213>  Homo sapiens

<400>  65

Glu Val Gln Leu Val Glu Ser Gly Ala Glu Val Lys Lys Pro Gly Ala
1               5                   10                  15

Ser Val Lys Val Ser Cys Lys Ala Ser
            20                  25

<210>  66
<211>  8
<212>  PRT
<213>  Homo sapiens

<400>  66

Gly Tyr Thr Phe Thr Ser Tyr Asp
1               5

<210>  67
<211>  17
<212>  PRT
<213>  Homo sapiens

<400>  67

Ile Asn Trp Val Arg Gln Ala Thr Gly Gln Gly Leu Glu Trp Met Gly
1               5                   10                  15

Trp

<210>  68
<211>  8
<212>  PRT
<213>  Homo sapiens

<400>  68

Met Asn Pro Asn Ser Gly Asn Thr
1               5

<210>  69
<211>  38
<212>  PRT
<213>  Homo sapiens

<400>  69

Gly Tyr Ala Gln Lys Phe Gln Gly Arg Val Thr Met Thr Arg Asn Thr
1               5                   10                  15

Ser Ile Ser Thr Ala Tyr Met Glu Leu Ser Ser Leu Arg Ser Glu Asp
20                  25                  30

Thr Ala Val Tyr Tyr Cys
        35

<210>  70
<211>  21
<212>  PRT
<213>  Homo sapiens

<400>  70

Ala Arg Glu Pro Tyr Gly Ser Gly Trp Tyr Phe Thr Pro Gln His Tyr
1               5                   10                  15

Tyr Gly Met Asp Val
            20

<210>  71
<211>  11
<212>  PRT
<213>  Homo sapiens

<400>  71

Trp Gly Lys Gly Thr Thr Val Thr Val Ser Ser
1               5                   10

<210>  72
<211>  128
<212>  PRT
<213>  Homo sapiens

<400>  72

Glu Val Gln Leu Val Glu Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Asp Ile Asn Trp Val Arg Gln Ala Thr Gly Gln Gly Leu Glu Trp Met
        35                  40                  45

Gly Trp Met Asn Pro Asn Ser Gly Asn Thr Gly Tyr Ala Gln Lys Phe
    50                  55                  60

Gln Gly Arg Val Thr Met Thr Arg Asn Thr Ser Ile Ser Thr Ala Tyr
65                  70                  75                  80

Met Glu Leu Ser Ser Leu Arg Ser Glu Asp Thr Ala Val Tyr Tyr Cys

85                      90                      95

Ala Arg Glu Pro Tyr Gly Ser Gly Trp Tyr Phe Thr Pro Gln His Tyr
            100                 105                 110

Tyr Gly Met Asp Val Trp Gly Lys Gly Thr Thr Val Thr Val Ser Ser
        115                 120                 125

<210>  73
<211>  26
<212>  PRT
<213>  Homo sapiens

<400>  73

<210>  74
<211>  6
<212>  PRT
<213>  Homo sapiens

<400>  74

Gln Gly Ile Thr Thr Glu
1               5

<210>  75
<211>  17
<212>  PRT
<213>  Homo sapiens

<400>  75

Leu Gly Trp Phe Gln Gln Arg Pro Gly Lys Ala Pro Lys Arg Leu Ile
1               5                   10                  15

Tyr

<210>  76
<211>  3
<212>  PRT
<213>  Homo sapiens

<400>  76

Ala Ala Ser
1

<210>  77
<211>  36
<212>  PRT
<213>  Homo sapiens

<400>  77

Thr Leu Glu Ser Gly Val Pro Ser Arg Phe Ser Gly Ser Gly Ser Gly
1               5                   10                  15

Thr Glu Phe Thr Leu Thr Ile Ser Ser Leu Gln Pro Glu Asp Phe Ala
            20                  25                  30

Thr Tyr Tyr Cys
        35

<210>  78
<211>  9
<212>  PRT
<213>  Homo sapiens

<400>  78

Leu Gln His Asn Ser Tyr Pro Arg Thr
1               5

<210>  79
<211>  10
<212>  PRT
<213>  Homo sapiens

<400>  79

Phe Gly Gln Gly Thr Lys Val Glu Ile Lys
1               5                   10

<210>  80
<211>  107
<212>  PRT
<213>  Homo sapiens

<400>  80

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Gly Ile Thr Thr Glu
            20                  25                  30

Leu Gly Trp Phe Gln Gln Arg Pro Gly Lys Ala Pro Lys Arg Leu Ile
        35                  40                  45

Tyr Ala Ala Ser Thr Leu Glu Ser Gly Val Pro Ser Arg Phe Ser Gly
    50                  55                  60

Ser Gly Ser Gly Thr Glu Phe Thr Leu Thr Ile Ser Ser Leu Gln Pro
65                  70                  75                  80

Glu Asp Phe Ala Thr Tyr Tyr Cys Leu Gln His Asn Ser Tyr Pro Arg
                85                  90                  95

Thr Phe Gly Gln Gly Thr Lys Val Glu Ile Lys
            100                 105

<210>  81
<211>  25
<212>  PRT
<213>  Homo sapiens

<400>  81

Gln Leu Gln Leu Gln Glu Ser Gly Pro Gly Leu Val Lys Pro Ser Gln
1               5                   10                  15

Thr Leu Ser Leu Thr Cys Thr Val Ser
            20                  25

<210>  82
<211>  10
<212>  PRT
<213>  Homo sapiens

<400>  82

Gly Gly Ser Ile Ser Ser Gly Ser Tyr Tyr
1               5                   10

<210>  83
<211>  17
<212>  PRT
<213>  Homo sapiens

<400>  83

Trp Ser Trp Ile Arg Gln Pro Ala Gly Lys Gly Leu Glu Trp Ile Gly
1               5                   10                  15

Arg

<210>  84
<211>  7
<212>  PRT
<213>  Homo sapiens

<400>  84

Ile Tyr Thr Ser Gly Ser Thr
1               5

<210>  85
<211>  38
<212>  PRT
<213>  Homo sapiens

<400>  85

Asn Tyr Asn Pro Ser Leu Lys Ser Arg Val Thr Ile Ser Val Asp Thr
1               5                   10                  15

Ser Lys Asn Gln Phe Ser Leu Lys Leu Ser Ser Val Thr Ala Ala Asp
            20                  25                  30

Thr Ala Val Tyr Tyr Cys
        35

<210>  86
<211>  20
<212>  PRT
<213>  Homo sapiens

<400>  86

Ala Arg Asp Asp Arg Ser Ser Ser Trp Ala Tyr Gly Tyr Tyr Tyr Tyr
1               5                   10                  15

Gly Met Asp Val
            20

<210>  87
<211>  11
<212>  PRT
<213>  Homo sapiens

<400>  87

Trp Gly Gln Gly Thr Thr Val Thr Val Ser Ser
1               5                   10

<210>  88
<211>  128
<212>  PRT
<213>  Homo sapiens

<400>  88

Gln Leu Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Ser Gly
            20                  25                  30

Ser Tyr Tyr Trp Ser Trp Ile Arg Gln Pro Ala Gly Lys Gly Leu Glu

35                  40                  45

Trp Ile Gly Arg Ile Tyr Thr Ser Gly Ser Thr Asn Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Asp Asp Arg Ser Ser Ser Trp Ala Tyr Gly Tyr Tyr Tyr
            100                 105                 110

Tyr Gly Met Asp Val Trp Gly Gln Gly Thr Thr Val Thr Val Ser Ser
        115                 120                 125

<210>  89
<211>  26
<212>  PRT
<213>  Homo sapiens

<400>  89

Glu Ile Val Leu Thr Gln Ser Pro Gly Thr Leu Ser Leu Ser Pro Gly
1               5                   10                  15

Glu Thr Ala Thr Leu Ser Cys Arg Ala Ser
            20                  25

<210>  90
<211>  6
<212>  PRT
<213>  Homo sapiens

<400>  90

Gln Ser Val Gly Thr Tyr
1               5

<210>  91
<211>  17
<212>  PRT
<213>  Homo sapiens

<400>  91

Leu Gly Trp Tyr Gln Gln Lys Pro Gly Gln Val Pro Arg Leu Leu Ile
1               5                   10                  15

Tyr

<210>  92
<211>  3
<212>  PRT
<213>  Homo sapiens

<400>  92

Asp Ala Ser
1


<210>  93
<211>  36
<212>  PRT
<213>  Homo sapiens

<400>  93

Asp Arg Ala Ser Gly Ile Pro Ala Arg Phe Ser Gly Ser Gly Ser Gly
1               5                   10                  15


Thr Gly Phe Thr Leu Thr Ile Ser Ser Leu Glu Ser Glu Asp Phe Ala
            20                  25                  30


Val Tyr Tyr Cys
        35


<210>  94
<211>  9
<212>  PRT
<213>  Homo sapiens

<400>  94

Gln Gln Arg Ser Ala Trp Pro Leu Thr
1               5


<210>  95
<211>  10
<212>  PRT
<213>  Homo sapiens

<400>  95

Phe Gly Gly Gly Thr Lys Leu Glu Ile Lys
1               5                   10


<210>  96
<211>  107
<212>  PRT
<213>  Homo sapiens

<400>  96

Glu Ile Val Leu Thr Gln Ser Pro Gly Thr Leu Ser Leu Ser Pro Gly
1               5                   10                  15

Glu Thr Ala Thr Leu Ser Cys Arg Ala Ser Gln Ser Val Gly Thr Tyr
            20                  25                  30

Leu Gly Trp Tyr Gln Gln Lys Pro Gly Gln Val Pro Arg Leu Leu Ile
        35                  40                  45

Tyr Asp Ala Ser Asp Arg Ala Ser Gly Ile Pro Ala Arg Phe Ser Gly
    50                  55                  60

Ser Gly Ser Gly Thr Gly Phe Thr Leu Thr Ile Ser Ser Leu Glu Ser
65                  70                  75                  80

Glu Asp Phe Ala Val Tyr Tyr Cys Gln Gln Arg Ser Ala Trp Pro Leu
                85                  90                  95

Thr Phe Gly Gly Gly Thr Lys Leu Glu Ile Lys
            100                 105

<210>  97
<211>  25
<212>  PRT
<213>  Homo sapiens

<400>  97

Glu Val Gln Leu Leu Glu Ser Gly Gly Gly Leu Val Gln Pro Gly Gly
1               5                   10                  15

Ser Leu Arg Leu Ser Cys Ala Ala Ser
            20                  25

<210>  98
<211>  8
<212>  PRT
<213>  Homo sapiens

<400>  98

Gly Leu Thr Leu Ser His Tyr Glu
1               5

<210>  99
<211>  17
<212>  PRT
<213>  Homo sapiens

<400>  99

Met Asn Trp Val Arg Gln Ala Pro Gly Lys Gly Leu Glu Trp Val Ser
1               5                   10                  15

Tyr

<210>  100
<211>  8
<212>  PRT
<213>  Homo sapiens

<400>  100

Ile Ser Ser Ser Gly Gly Thr Ile
1               5


<210>  101
<211>  38
<212>  PRT
<213>  Homo sapiens

<400>  101

Arg Tyr Gly Asp Ser Val Lys Gly Arg Phe Thr Ile Ser Arg Asp Asn
1               5                   10                  15


Ala Lys Asn Ser Leu His Leu Gln Met Asn Ser Leu Arg Asp Glu Asp
            20                  25                  30


Thr Ala Val Tyr Tyr Cys
        35


<210>  102
<211>  15
<212>  PRT
<213>  Homo sapiens

<400>  102

Ala Ser Cys Lys Gly Ser Gly Asn Tyr Ala His Ala Phe Asp Ile
1               5                   10                  15


<210>  103
<211>  11
<212>  PRT
<213>  Homo sapiens

<400>  103

Trp Gly Gln Gly Thr Met Val Thr Val Ser Ser
1               5                   10


<210>  104
<211>  122
<212>  PRT
<213>  Homo sapiens

<400>  104

Glu Val Gln Leu Leu Glu Ser Gly Gly Gly Leu Val Gln Pro Gly Gly

1               5                   10                  15

Ser Leu Arg Leu Ser Cys Ala Ala Ser Gly Leu Thr Leu Ser His Tyr
            20                  25                  30

Glu Met Asn Trp Val Arg Gln Ala Pro Gly Lys Gly Leu Glu Trp Val
        35                  40                  45

Ser Tyr Ile Ser Ser Ser Gly Gly Thr Ile Arg Tyr Gly Asp Ser Val
    50                  55                  60

Lys Gly Arg Phe Thr Ile Ser Arg Asp Asn Ala Lys Asn Ser Leu His
65                  70                  75                  80

Leu Gln Met Asn Ser Leu Arg Asp Glu Asp Thr Ala Val Tyr Tyr Cys
                85                  90                  95

Ala Ser Cys Lys Gly Ser Gly Asn Tyr Ala His Ala Phe Asp Ile Trp
            100                 105                 110

Gly Gln Gly Thr Met Val Thr Val Ser Ser
        115                 120


<210>  105
<211>  26
<212>  PRT
<213>  Homo sapiens

<400>  105

Glu Ile Val Met Thr Gln Ser Pro Gly Thr Leu Ser Leu Ser Pro Gly
1               5                   10                  15

Glu Ser Ala Thr Leu Ser Cys Arg Ala Ser
            20                  25


<210>  106
<211>  7
<212>  PRT
<213>  Homo sapiens

<400>  106

Gln Ser Ile Lys Asn Ser Glu
1               5


<210>  107
<211>  17
<212>  PRT
<213>  Homo sapiens

<400>  107

Ile Ala Trp Tyr Gln His Lys Leu Gly Gln Ala Pro Arg Leu Leu Ile
1               5                   10                  15

Tyr

<210>  108
<211>  3
<212>  PRT
<213>  Homo sapiens

<400>  108

Gly Ala Ala
1

<210>  109
<211>  36
<212>  PRT
<213>  Homo sapiens

<400>  109

Ser Arg Ala Ile Gly Val Pro Asp Arg Phe Ser Gly Ser Gly Ser Gly
1               5                   10                  15

Thr Asp Phe Thr Leu Thr Ile Ser Arg Leu Glu Pro Glu Asp Phe Ala
            20                  25                  30

Val Tyr Tyr Cys
        35

<210>  110
<211>  8
<212>  PRT
<213>  Homo sapiens

<400>  110

Gln Gln Tyr Gly Ser Ser Arg Thr
1               5

<210>  111
<211>  10
<212>  PRT
<213>  Homo sapiens

<400>  111

Phe Gly Gln Gly Thr Lys Leu Asp Ile Lys
1               5                   10

<210>  112
<211>  107

<212>  PRT
<213>  Homo sapiens

<400>  112

Glu Ile Val Met Thr Gln Ser Pro Gly Thr Leu Ser Leu Ser Pro Gly
1               5                   10                  15

Glu Ser Ala Thr Leu Ser Cys Arg Ala Ser Gln Ser Ile Lys Asn Ser
            20                  25                  30

Glu Ile Ala Trp Tyr Gln His Lys Leu Gly Gln Ala Pro Arg Leu Leu
        35                  40                  45

Ile Tyr Gly Ala Ala Ser Arg Ala Ile Gly Val Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Ser Ser Arg
                85                  90                  95

Thr Phe Gly Gln Gly Thr Lys Leu Asp Ile Lys
            100                 105

<210>  113
<211>  25
<212>  PRT
<213>  Homo sapiens

<400>  113

Gln Val Gln Leu Val Glu Ser Gly Gly Gly Leu Val Gln Pro Gly Gly
1               5                   10                  15

Ser Leu Arg Leu Ser Cys Ala Ala Ser
            20                  25

<210>  114
<211>  8
<212>  PRT
<213>  Homo sapiens

<400>  114

Gly Phe Gly Val Ser Ser Ser Phe
1               5

<210>  115
<211>  17
<212>  PRT
<213>  Homo sapiens

<400>  115

Met Ser Trp Val Arg Gln Val Pro Gly Lys Gly Leu Glu Trp Leu Ser
1               5                   10                  15

Val

<210>  116
<211>  7
<212>  PRT
<213>  Homo sapiens

<400>  116

Ile Tyr Gly Tyr Gly Ser Thr
1               5

<210>  117
<211>  38
<212>  PRT
<213>  Homo sapiens

<400>  117

Asp Asn Ala Asp Ser Val Lys Gly Arg Phe Thr Ile Ser Arg Asp Asn
1               5                   10                  15

Ser Lys Asn Thr Leu Phe Leu Gln Met Asn Asn Leu Arg Ala Glu Asp
            20                  25                  30

Thr Ala Val Tyr Tyr Cys
        35

<210>  118
<211>  15
<212>  PRT
<213>  Homo sapiens

<400>  118

Ala Arg Gly His Tyr Tyr Asp Ser Thr Tyr Asp Ala Phe Asp Ile
1               5                   10                  15

<210>  119
<211>  11
<212>  PRT
<213>  Homo sapiens

<400>  119

Trp Gly Gln Gly Thr Met Val Thr Val Ser Ser
1               5                   10

<210>  120
<211>  121
<212>  PRT
<213>  Homo sapiens

<400>  120

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Gly Val Ser Ser Ser
            20                  25                  30

Phe Met Ser Trp Val Arg Gln Val Pro Gly Lys Gly Leu Glu Trp Leu
        35                  40                  45

Ser Val Ile Tyr Gly Tyr Gly Ser Thr Asp Asn Ala Asp Ser Val Lys
    50                  55                  60

Gly Arg Phe Thr Ile Ser Arg Asp Asn Ser Lys Asn Thr Leu Phe Leu
65                  70                  75                  80

Gln Met Asn Asn Leu Arg Ala Glu Asp Thr Ala Val Tyr Tyr Cys Ala
                85                  90                  95

Arg Gly His Tyr Tyr Asp Ser Thr Tyr Asp Ala Phe Asp Ile Trp Gly
            100                 105                 110

Gln Gly Thr Met Val Thr Val Ser Ser
        115                 120


<210>  121
<211>  26
<212>  PRT
<213>  Homo sapiens

<400>  121

Thr Ile Gln Leu Thr Gln Ser Pro Ser Ser Leu Ser Ala Ser Val Gly
1               5                   10                  15

Asp Arg Val Thr Ile Thr Cys Arg Ala Ser
            20                  25


<210>  122
<211>  6
<212>  PRT
<213>  Homo sapiens

<400>  122

Gln Gly Ile Ser Asn Tyr
1               5

<210>  123
<211>  17
<212>  PRT
<213>  Homo sapiens

<400>  123

Leu Ala Trp Phe Gln Gln Lys Pro Gly Lys Ala Pro Lys Ser Leu Ile
1               5                   10                  15

Tyr

<210>  124
<211>  3
<212>  PRT
<213>  Homo sapiens

<400>  124

Ala Ala Ser
1

<210>  125
<211>  36
<212>  PRT
<213>  Homo sapiens

<400>  125

Ser Leu Gln Ser Gly Val Pro Ser Arg Phe Ser Gly Ser Gly Ser Gly
1               5                   10                  15

Thr Asp Phe Thr Leu Thr Ile Ser Ser Leu Gln Pro Glu Asp Phe Ala
            20                  25                  30

Thr Tyr Tyr Cys
        35

<210>  126
<211>  9
<212>  PRT
<213>  Homo sapiens

<400>  126

Gln Gln Ala Asn Ser Leu Pro Leu Thr
1               5

<210>  127
<211>  10
<212>  PRT
<213>  Homo sapiens

<400>  127

Phe Gly Gly Gly Thr Lys Val Asp Ile Lys
1               5                   10


<210>  128
<211>  107
<212>  PRT
<213>  Homo sapiens

<400>  128

Thr Ile Gln Leu Thr Gln Ser Pro Ser Ser Leu Ser Ala Ser Val Gly
1               5                   10                  15

Asp Arg Val Thr Ile Thr Cys Arg Ala Ser Gln Gly Ile Ser Asn Tyr
            20                  25                  30

Leu Ala Trp Phe Gln Gln Lys Pro Gly Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ala Asn Ser Leu Pro Leu
                85                  90                  95

Thr Phe Gly Gly Gly Thr Lys Val Asp Ile Lys
            100                 105


<210>  129
<211>  25
<212>  PRT
<213>  Homo sapiens

<400>  129

Gln Leu Gln Leu Gln Glu Ser Gly Pro Gly Leu Val Lys Pro Ser Gln
1               5                   10                  15

Thr Leu Ser Leu Thr Cys Ala Val Ser
            20                  25


<210>  130
<211>  10
<212>  PRT
<213>  Homo sapiens

<400>  130

Gly Gly Ser Ile Ser Ser Gly Asp Tyr Tyr
1               5                   10

<210>  131
<211>  17
<212>  PRT
<213>  Homo sapiens

<400>  131

Trp Asn Trp Ile Arg Gln Pro Ala Gly Glu Arg Leu Glu Trp Ile Gly
1               5                   10                  15

Arg

<210>  132
<211>  7
<212>  PRT
<213>  Homo sapiens

<400>  132

Thr Tyr Ser Ser Gly Ile Thr
1               5

<210>  133
<211>  38
<212>  PRT
<213>  Homo sapiens

<400>  133

Asn Tyr Asn Pro Ser Leu Leu Ser Arg Val Thr Ile Ser Val Asp Thr
1               5                   10                  15

Ser Lys Asn Gln Phe Ser Leu Lys Leu Asn Ser Val Thr Ala Ala Asp
            20                  25                  30

Thr Ala Val Tyr Tyr Cys
        35

<210>  134
<211>  11
<212>  PRT
<213>  Homo sapiens

<400>  134

Val Arg Lys Arg Glu Arg Gly Thr Leu Asp Ile
1               5                   10

<210>  135
<211>  11
<212>  PRT

<213>  Homo sapiens

<400>  135

Trp Gly Gln Gly Thr Met Val Thr Val Pro Ser
1               5                   10


<210>  136
<211>  119
<212>  PRT
<213>  Homo sapiens

<400>  136

Gln Leu Gln Leu Gln Glu Ser Gly Pro Gly Leu Val Lys Pro Ser Gln
1               5                   10                  15


Thr Leu Ser Leu Thr Cys Ala Val Ser Gly Gly Ser Ile Ser Ser Gly
            20                  25                  30


Asp Tyr Tyr Trp Asn Trp Ile Arg Gln Pro Ala Gly Glu Arg Leu Glu
        35                  40                  45


Trp Ile Gly Arg Thr Tyr Ser Ser Gly Ile Thr Asn Tyr Asn Pro Ser
    50                  55                  60


Leu Leu Ser Arg Val Thr Ile Ser Val Asp Thr Ser Lys Asn Gln Phe
65                  70                  75                  80


Ser Leu Lys Leu Asn Ser Val Thr Ala Ala Asp Thr Ala Val Tyr Tyr
                85                  90                  95


Cys Val Arg Lys Arg Glu Arg Gly Thr Leu Asp Ile Trp Gly Gln Gly
            100                 105                 110


Thr Met Val Thr Val Pro Ser
        115


<210>  137
<211>  26
<212>  PRT
<213>  Homo sapiens

<400>  137

Asp Ile Gln Leu Thr Gln Ser Pro Ser Ser Leu Ser Ala Ser Val Gly
1               5                   10                  15


Asp Arg Val Ser Ile Thr Cys Arg Ala Ser
            20                  25


<210>  138

<211>  6
<212>  PRT
<213>  Homo sapiens

<400>  138

Gln Gly Ile Ser Ser Tyr
1               5



<210>  139
<211>  17
<212>  PRT
<213>  Homo sapiens

<400>  139

Leu Ala Trp Tyr Gln Gln Lys Pro Gly Thr Ala Pro Lys Leu Leu Ile
1               5                   10                  15


Tyr




<210>  140
<211>  3
<212>  PRT
<213>  Homo sapiens

<400>  140

Ser Ile Ser
1



<210>  141
<211>  36
<212>  PRT
<213>  Homo sapiens

<400>  141

Thr Leu Gln Ser Gly Val Pro Ser Arg Phe Ser Gly Ser Gly Ser Gly
1               5                   10                  15


Thr Glu Phe Thr Pro Thr Ile Asn Asn Leu Gln Pro Glu Asp Phe Ala
            20                  25                  30


Thr Tyr Tyr Cys
        35



<210>  142
<211>  10
<212>  PRT
<213>  Homo sapiens

<400>  142

Gln Gln Leu Asn Ser Asp Pro Pro Tyr Thr

1               5                   10


<210>  143
<211>  10
<212>  PRT
<213>  Homo sapiens

<400>  143

Phe Gly Gln Gly Thr Lys Val Glu Ile Lys
1               5                   10


<210>  144
<211>  108
<212>  PRT
<213>  Homo sapiens

<400>  144

Asp Ile Gln Leu Thr Gln Ser Pro Ser Ser Leu Ser Ala Ser Val Gly
1               5                   10                  15


Asp Arg Val Ser Ile Thr Cys Arg Ala Ser Gln Gly Ile Ser Ser Tyr
            20                  25                  30


Leu Ala Trp Tyr Gln Gln Lys Pro Gly Thr Ala Pro Lys Leu Leu Ile
        35                  40                  45


Tyr Ser Ile Ser Thr Leu Gln Ser Gly Val Pro Ser Arg Phe Ser Gly
    50                  55                  60


Ser Gly Ser Gly Thr Glu Phe Thr Pro Thr Ile Asn Asn Leu Gln Pro
65                  70                  75                  80


Glu Asp Phe Ala Thr Tyr Tyr Cys Gln Gln Leu Asn Ser Asp Pro Pro
                85                  90                  95


Tyr Thr Phe Gly Gln Gly Thr Lys Val Glu Ile Lys
            100                 105


<210>  145
<211>  25
<212>  PRT
<213>  Homo sapiens

<400>  145

Glu Val Gln Leu Leu Glu Ser Gly Gly Gly Leu Val Lys Pro Gly Arg
1               5                   10                  15


Ser Leu Arg Leu Ser Cys Ala Ala Ser
            20                  25

<210>  146
<211>  8
<212>  PRT
<213>  Homo sapiens

<400>  146

Gly Phe Ile Phe Ser Asn Tyr Asp
1               5


<210>  147
<211>  17
<212>  PRT
<213>  Homo sapiens

<400>  147

Met His Trp Val Arg Gln Ala Pro Gly Lys Gly Leu Glu Trp Val Ser
1               5                   10                  15


Ser


<210>  148
<211>  8
<212>  PRT
<213>  Homo sapiens

<400>  148

Ile Ile Ser Ser Gly Asp His Thr
1               5


<210>  149
<211>  38
<212>  PRT
<213>  Homo sapiens

<400>  149

Tyr Tyr Ala Asp Ser Val Lys Gly Arg Phe Thr Ile Ser Arg Asp Asn
1               5                   10                  15


Ala Lys Asp Ser Leu His Leu Gln Val Asn Ser Leu Arg Val Glu Asp
            20                  25                  30


Thr Ala Val Tyr His Cys
        35


<210>  150
<211>  21
<212>  PRT
<213>  Homo sapiens

<400>  150

Ala Arg Ala Glu Gly Phe Cys Ser Gly Gly Ser Cys Tyr Ser Ser Gly
1               5                   10                  15

Trp Ser Phe Asp Leu
            20

<210>  151
<211>  11
<212>  PRT
<213>  Homo sapiens

<400>  151

Trp Gly Gln Gly Thr Met Val Thr Val Ser Ser
1               5                   10

<210>  152
<211>  128
<212>  PRT
<213>  Homo sapiens

<400>  152

Glu Val Gln Leu Leu Glu Ser Gly Gly Gly Leu Val Lys Pro Gly Arg
1               5                   10                  15

Ser Leu Arg Leu Ser Cys Ala Ala Ser Gly Phe Ile Phe Ser Asn Tyr
            20                  25                  30

Asp Met His Trp Val Arg Gln Ala Pro Gly Lys Gly Leu Glu Trp Val
        35                  40                  45

Ser Ser Ile Ile Ser Ser Gly Asp His Thr Tyr Tyr Ala Asp Ser Val
    50                  55                  60

Lys Gly Arg Phe Thr Ile Ser Arg Asp Asn Ala Lys Asp Ser Leu His
65                  70                  75                  80

Leu Gln Val Asn Ser Leu Arg Val Glu Asp Thr Ala Val Tyr His Cys
                85                  90                  95

Ala Arg Ala Glu Gly Phe Cys Ser Gly Gly Ser Cys Tyr Ser Ser Gly
            100                 105                 110

Trp Ser Phe Asp Leu Trp Gly Gln Gly Thr Met Val Thr Val Ser Ser
        115                 120                 125

<210>  153
<211>  26
<212>  PRT
<213>  Homo sapiens

<400>  153

Asp Ile Val Met Thr Gln Ser Pro Asp Ser Leu Ala Val Ser Leu Gly
1               5                   10                  15

Glu Arg Ala Thr Ile Asn Cys Lys Ser Ser
            20                  25

<210>  154
<211>  12
<212>  PRT
<213>  Homo sapiens

<400>  154

Gln Ser Val Leu Tyr Ser Ser Asn Asn Lys Asn Tyr
1               5                   10

<210>  155
<211>  17
<212>  PRT
<213>  Homo sapiens

<400>  155

Leu Ala Trp Tyr Gln Gln Lys Pro Gly Gln Pro Pro Lys Leu Leu Ile
1               5                   10                  15

Tyr

<210>  156
<211>  3
<212>  PRT
<213>  Homo sapiens

<400>  156

Trp Ala Ser
1

<210>  157
<211>  36
<212>  PRT
<213>  Homo sapiens

<400>  157

Thr Arg Glu Ser Gly Val Pro Asp Arg Phe Ser Gly Ser Gly Ser Gly
1               5                   10                  15

Thr Asp Phe Thr Leu Thr Ile Ser Ser Leu Gln Ala Glu Asp Val Ala
            20                  25                  30

Val Tyr Phe Cys
        35

<210>  158
<211>  9
<212>  PRT
<213>  Homo sapiens

<400>  158

Gln Gln Tyr Asn Arg Thr Pro Trp Thr
1               5

<210>  159
<211>  10
<212>  PRT
<213>  Homo sapiens

<400>  159

Phe Gly Gln Gly Thr Lys Val Asp Ile Lys
1               5                   10

<210>  160
<211>  113
<212>  PRT
<213>  Homo sapiens

<400>  160

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Val Leu Tyr Ser
            20                  25                  30

Ser Asn Asn Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Phe Cys Gln Gln
                85                  90                  95

Tyr Asn Arg Thr Pro Trp Thr Phe Gly Gln Gly Thr Lys Val Asp Ile
            100                 105                 110

Lys

<210>  161
<211>  25
<212>  PRT
<213>  Homo sapiens

<400>  161

Glu Val Gln Leu Val Glu Ser Gly Gly Asp Leu Val Gln Pro Gly Arg
1               5                   10                  15

Ser Leu Arg Leu Ser Cys Ala Ala Ser
            20                  25

<210>  162
<211>  8
<212>  PRT
<213>  Homo sapiens

<400>  162

Gly Phe Thr Phe Glu Asp Tyr Ala
1               5

<210>  163
<211>  17
<212>  PRT
<213>  Homo sapiens

<400>  163

Met His Trp Val Arg Gln Ala Pro Gly Lys Gly Leu Glu Trp Val Ser
1               5                   10                  15

Ser

<210>  164
<211>  8
<212>  PRT
<213>  Homo sapiens

<400>  164

Ile Asn Trp Ser Ser Gly Ser Ile
1               5

<210>  165
<211>  38
<212>  PRT
<213>  Homo sapiens

<400>  165

Val Tyr Ala Asp Ser Val Lys Gly Arg Phe Thr Ile Ser Arg Asp Asn
1               5                   10                  15

Ala Lys Asn Ser Leu Tyr Leu Gln Met Asn Ser Leu Arg Pro Asp Asp
            20                  25                  30

Thr Ala Leu Tyr Tyr Cys
        35

<210> 166
<211> 16
<212> PRT
<213> Homo sapiens

<400> 166

Ala Lys Asp Ser Gly Pro Tyr Gly Ser Gly His Tyr Gly Met Asp Val
1               5                   10                  15

<210> 167
<211> 11
<212> PRT
<213> Homo sapiens

<400> 167

Trp Gly Gln Gly Thr Thr Val Thr Val Ser Ser
1               5                   10

<210> 168
<211> 123
<212> PRT
<213> Homo sapiens

<400> 168

Glu Val Gln Leu Val Glu Ser Gly Gly Asp Leu Val Gln Pro Gly Arg
1               5                   10                  15

Ser Leu Arg Leu Ser Cys Ala Ala Ser Gly Phe Thr Phe Glu Asp Tyr
            20                  25                  30

Ala Met His Trp Val Arg Gln Ala Pro Gly Lys Gly Leu Glu Trp Val
        35                  40                  45

Ser Ser Ile Asn Trp Ser Ser Gly Ser Ile Val Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Pro Asp Asp Thr Ala Leu Tyr Tyr Cys
                85                  90                  95

Ala Lys Asp Ser Gly Pro Tyr Gly Ser Gly His Tyr Gly Met Asp Val
            100                 105                 110

Trp Gly Gln Gly Thr Thr Val Thr Val Ser Ser
        115                 120

<210>  169
<211>  26
<212>  PRT
<213>  Homo sapiens

<400>  169

Glu Ile Val Leu Thr Gln Ser Pro Asp Phe Gln Ser Val Thr Pro Arg
1               5                   10                  15

Glu Glu Val Thr Ile Thr Cys Arg Ala Ser
            20                  25

<210>  170
<211>  12
<212>  PRT
<213>  Homo sapiens

<400>  170

Gln Ser Ile Leu Tyr Ser Ser Asn Asn Lys Asn Tyr
1               5                   10

<210>  171
<211>  17
<212>  PRT
<213>  Homo sapiens

<400>  171

Leu Ala Trp Tyr Gln Gln Lys Pro Gly Gln Pro Pro Lys Leu Leu Ile
1               5                   10                  15

Tyr

<210>  172
<211>  3
<212>  PRT
<213>  Homo sapiens

<400>  172

Trp Ala Ser
1

<210>  173
<211>  36
<212>  PRT

<213>  Homo sapiens

<400>  173

Thr Arg Glu Ser Gly Val Pro Asp Arg Phe Ser Gly Ser Gly Ser Gly
1               5                   10                  15

Thr Asp Phe Thr Leu Thr Ile Ser Ser Leu Gln Ala Glu Asp Val Ala
            20                  25                  30

Val Tyr Phe Cys
        35

<210>  174
<211>  9
<212>  PRT
<213>  Homo sapiens

<400>  174

Gln Gln Tyr Asn Arg Thr Pro Trp Thr
1               5

<210>  175
<211>  10
<212>  PRT
<213>  Homo sapiens

<400>  175

Phe Gly Gln Gly Thr Lys Val Asp Ile Lys
1               5                   10

<210>  176
<211>  107
<212>  PRT
<213>  Homo sapiens

<400>  176

Glu Ile Val Leu Thr Gln Ser Pro Asp Phe Gln Ser Val Thr Pro Arg
1               5                   10                  15

Glu Glu Val Thr Ile Thr Cys Arg Ala Ser Gln Ser Ile Gly Ser Asp
            20                  25                  30

Leu His Trp Tyr Gln Gln Arg Pro Asp Gln Pro Pro Arg Leu Leu Val
        35                  40                  45

Lys Tyr Ala Ser Gln Ser Leu Ser Gly Val Pro Ser Arg Phe Arg Gly
    50                  55                  60

Ser Gly Ser Gly Thr Asp Phe Thr Leu Thr Ile Asp Ser Pro Gln Pro
65                  70                  75                  80

Asp Asp Ile Ala Thr Tyr Tyr Cys His Gln Thr Ser Ala Ser Pro Trp
                85                  90                  95

Thr Phe Gly Gln Gly Thr Lys Leu Glu Ile Lys
            100                 105

<210>  177
<211>  25
<212>  PRT
<213>  Homo sapiens

<400>  177

Glu Val Gln Leu Val Glu Ser Gly Gly Asp Leu Val Gln Pro Gly Arg
1               5                   10                  15

Ser Leu Arg Leu Ser Cys Ala Ala Ser
            20                  25

<210>  178
<211>  8
<212>  PRT
<213>  Homo sapiens

<400>  178

Gly Phe Thr Phe Glu Asp Tyr Ala
1               5

<210>  179
<211>  17
<212>  PRT
<213>  Homo sapiens

<400>  179

Met His Trp Val Arg Gln Ala Pro Gly Lys Gly Leu Glu Trp Val Ser
1               5                   10                  15

Ser

<210>  180
<211>  8
<212>  PRT
<213>  Homo sapiens

<400>  180

Ile Asn Trp Ser Ser Gly Ser Ile
1               5

<210>  181

<211>  38
<212>  PRT
<213>  Homo sapiens

<400>  181

Val Tyr Ala Asp Ser Val Lys Gly Arg Phe Thr Ile Ser Arg Asp Asn
1               5                   10                  15

Ala Lys Asn Ser Leu Tyr Leu Gln Met Asn Ser Leu Arg Pro Asp Asp
            20                  25                  30

Thr Ala Leu Tyr Tyr Cys
        35

<210>  182
<211>  16
<212>  PRT
<213>  Homo sapiens

<400>  182

Ala Lys Asp Ser Gly Pro Tyr Gly Ser Gly His Tyr Gly Met Asp Val
1               5                   10                  15

<210>  183
<211>  11
<212>  PRT
<213>  Homo sapiens

<400>  183

Trp Gly Gln Gly Thr Thr Val Thr Val Ser Ser
1               5                   10

<210>  184
<211>  123
<212>  PRT
<213>  Homo sapiens

<400>  184

Glu Val Gln Leu Val Glu Ser Gly Gly Asp Leu Val Gln Pro Gly Arg
1               5                   10                  15

Ser Leu Arg Leu Ser Cys Ala Ala Ser Gly Phe Thr Phe Glu Asp Tyr
            20                  25                  30

Ala Met His Trp Val Arg Gln Ala Pro Gly Lys Gly Leu Glu Trp Val
        35                  40                  45

Ser Ser Ile Asn Trp Ser Ser Gly Ser Ile Val Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Pro Asp Asp Thr Ala Leu Tyr Tyr Cys
                85                  90                  95

Ala Lys Asp Ser Gly Pro Tyr Gly Ser Gly His Tyr Gly Met Asp Val
            100                 105                 110

Trp Gly Gln Gly Thr Thr Val Thr Val Ser Ser
        115                 120

<210>  185
<211>  26
<212>  PRT
<213>  Homo sapiens

<400>  185

Glu Ile Val Leu Thr Gln Ser Pro Asp Phe Gln Ser Val Thr Pro Arg
1               5                   10                  15

Glu Glu Val Thr Ile Thr Cys Arg Ala Ser
            20                  25

<210>  186
<211>  6
<212>  PRT
<213>  Homo sapiens

<400>  186

Gln Ser Ile Gly Ser Asp
1               5

<210>  187
<211>  17
<212>  PRT
<213>  Homo sapiens

<400>  187

Leu His Trp Tyr Gln Gln Arg Pro Asp Gln Pro Pro Arg Leu Leu Val
1               5                   10                  15

Lys

<210>  188
<211>  3
<212>  PRT
<213>  Homo sapiens

<400>  188

Tyr Ala Ser
1

<210> 189
<211> 36
<212> PRT
<213> Homo sapiens

<400> 189

Gln Ser Leu Ser Gly Val Pro Ser Arg Phe Arg Gly Ser Gly Ser Gly
1               5                   10                  15

Thr Asp Phe Thr Leu Thr Ile Asp Ser Pro Gln Pro Asp Asp Ile Ala
            20                  25                  30

Thr Tyr Tyr Cys
        35

<210> 190
<211> 9
<212> PRT
<213> Homo sapiens

<400> 190

His Gln Thr Ser Ala Ser Pro Trp Thr
1               5

<210> 191
<211> 10
<212> PRT
<213> Homo sapiens

<400> 191

Phe Gly Gln Gly Thr Lys Leu Glu Ile Lys
1               5                   10

<210> 192
<211> 107
<212> PRT
<213> Homo sapiens

<400> 192

Glu Ile Val Leu Thr Gln Ser Pro Asp Phe Gln Ser Val Thr Pro Arg
1               5                   10                  15

Glu Glu Val Thr Ile Thr Cys Arg Ala Ser Gln Ser Ile Gly Ser Asp
            20                  25                  30

Leu His Trp Tyr Gln Gln Arg Pro Asp Gln Pro Pro Arg Leu Leu Val
        35                  40                  45

Lys Tyr Ala Ser Gln Ser Leu Ser Gly Val Pro Ser Arg Phe Arg Gly
    50                  55                  60

Ser Gly Ser Gly Thr Asp Phe Thr Leu Thr Ile Asp Ser Pro Gln Pro
65                  70                  75                  80

Asp Asp Ile Ala Thr Tyr Tyr Cys His Gln Thr Ser Ala Ser Pro Trp
                85                  90                  95

Thr Phe Gly Gln Gly Thr Lys Leu Glu Ile Lys
            100                 105

<210>  193
<211>  25
<212>  PRT
<213>  Homo sapiens

<400>  193

Gln Val Gln Leu Val Gln Ser Gly Ala Glu Val Lys Lys Pro Gly Glu
1               5                   10                  15

Ser Leu Arg Ile Ser Cys Lys Ala Ser
            20                  25

<210>  194
<211>  8
<212>  PRT
<213>  Homo sapiens

<400>  194

Gly Phe Ser Phe Ser Asn Tyr Trp
1               5

<210>  195
<211>  17
<212>  PRT
<213>  Homo sapiens

<400>  195

Ile Ser Trp Val Arg Gln Leu Pro Gly Lys Gly Leu Glu Trp Met Gly
1               5                   10                  15

Arg

<210>  196
<211>  8
<212>  PRT
<213>  Homo sapiens

<400>  196

Ile Asp Pro Ser Asp Ser Tyr Thr
1               5

<210>  197
<211>  38
<212>  PRT
<213>  Homo sapiens

<400>  197

Asn Tyr Ser Pro Ser Phe Glu Gly Arg Val Thr Met Ser Thr Asp Asn
1               5                   10                  15

Ser Ile Thr Thr Ala Tyr Leu Gln Trp Ser Ser Leu Lys Ala Ser Asp
            20                  25                  30

Thr Ala Met Tyr Tyr Cys
        35

<210>  198
<211>  18
<212>  PRT
<213>  Homo sapiens

<400>  198

Ala Arg Arg Glu Gly Gly Tyr Thr Phe Gly Phe His Tyr Tyr Gly Met
1               5                   10                  15

Asp Val

<210>  199
<211>  11
<212>  PRT
<213>  Homo sapiens

<400>  199

Trp Gly Gln Gly Thr Thr Val Thr Val Ser Ser
1               5                   10

<210>  200
<211>  125
<212>  PRT
<213>  Homo sapiens

<400>  200

Gln Val Gln Leu Val Gln Ser Gly Ala Glu Val Lys Lys Pro Gly Glu
1               5                   10                  15

Ser Leu Arg Ile Ser Cys Lys Ala Ser Gly Phe Ser Phe Ser Asn Tyr
            20                  25                  30

Trp Ile Ser Trp Val Arg Gln Leu Pro Gly Lys Gly Leu Glu Trp Met
        35                  40                  45

Gly Arg Ile Asp Pro Ser Asp Ser Tyr Thr Asn Tyr Ser Pro Ser Phe
    50                  55                  60

Glu Gly Arg Val Thr Met Ser Thr Asp Asn Ser Ile Thr Thr Ala Tyr
65                  70                  75                  80

Leu Gln Trp Ser Ser Leu Lys Ala Ser Asp Thr Ala Met Tyr Tyr Cys
                85                  90                  95

Ala Arg Arg Glu Gly Gly Tyr Thr Phe Gly Phe His Tyr Tyr Gly Met
            100                 105                 110

Asp Val Trp Gly Gln Gly Thr Thr Val Thr Val Ser Ser
        115                 120                 125

<210> 201
<211> 26
<212> PRT
<213> Homo sapiens

<400> 201

Glu Ile Val Met Thr Gln Ser Pro Ala Thr Leu Ser Val Ser Pro Gly
1               5                   10                  15

Glu Arg Ala Thr Leu Ser Cys Arg Ala Ser
            20                  25

<210> 202
<211> 6
<212> PRT
<213> Homo sapiens

<400> 202

Gln Ser Val Ser Ser Asn
1               5

<210> 203
<211> 17
<212> PRT
<213> Homo sapiens

<400> 203

Leu Ala Trp Tyr Gln Gln Lys Pro Gly Gln Ala Pro Arg Leu Leu Ile
1               5                   10                  15

Tyr

<210> 204
<211> 3
<212> PRT
<213> Homo sapiens

<400> 204

Gly Ala Ser
1

<210> 205
<211> 36
<212> PRT
<213> Homo sapiens

<400> 205

Thr Arg Ala Thr Gly Ile Pro Ala Arg Phe Ser Gly Ser Gly Ser Gly
1               5                   10                  15

Thr Glu Phe Thr Leu Thr Ile Ser Ser Leu Gln Ser Glu Asp Phe Ala
            20                  25                  30

Val Tyr Tyr Cys
        35

<210> 206
<211> 9
<212> PRT
<213> Homo sapiens

<400> 206

Gln Gln Tyr Asn Asn Trp Pro Leu Thr
1               5

<210> 207
<211> 10
<212> PRT
<213> Homo sapiens

<400> 207

Phe Gly Pro Gly Thr Lys Val Asp Ile Lys
1               5                   10

<210> 208
<211> 107
<212> PRT
<213> Homo sapiens

<400>  208

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Val Ser Ser Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Ser Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Asn Asn Trp Pro Leu
                85                  90                  95

Thr Phe Gly Pro Gly Thr Lys Val Asp Ile Lys
            100                 105


<210>  209
<211>  25
<212>  PRT
<213>  Homo sapiens

<400>  209

Glu Val Gln Leu Val Glu Ser Gly Ala Glu Val Lys Lys Pro Gly Glu
1               5                   10                  15

Ser Leu Lys Ile Ser Cys Lys Gly Ser
            20                  25


<210>  210
<211>  8
<212>  PRT
<213>  Homo sapiens

<400>  210

Gly Tyr Ser Phe Thr Ser Tyr Trp
1               5


<210>  211
<211>  17
<212>  PRT
<213>  Homo sapiens

<400>  211

Ile Gly Trp Val His Gln Met Pro Gly Lys Gly Leu Glu Trp Met Gly
1               5                   10                  15

Ile

<210>  212
<211>  8
<212>  PRT
<213>  Homo sapiens

<400>  212

Ile Tyr Pro Gly Asp Ser Asp Thr
1               5

<210>  213
<211>  38
<212>  PRT
<213>  Homo sapiens

<400>  213

Arg Tyr Ser Pro Ser Phe Gln Gly Gln Val Thr Ile Ser Ala Asp Lys
1               5                   10                  15

Ser Ile Ser Thr Ala Tyr Leu Gln Trp Ser Ser Leu Lys Ala Ser Asp
            20                  25                  30

Thr Ala Met Tyr Tyr Cys
        35

<210>  214
<211>  18
<212>  PRT
<213>  Homo sapiens

<400>  214

Ala Arg Arg Asp Gln Asn Cys Gly Gly Asp Cys Tyr Ala Asp Ala Phe
1               5                   10                  15

Asp Ile

<210>  215
<211>  11
<212>  PRT
<213>  Homo sapiens

<400>  215

Trp Gly Gln Gly Thr Met Val Thr Val Ser Ser
1               5                   10

<210>  216
<211>  125
<212>  PRT
<213>  Homo sapiens

<400>  216

Glu Val Gln Leu Val Glu Ser Gly Ala Glu Val Lys Lys Pro Gly Glu
1               5                   10                  15

Ser Leu Lys Ile Ser Cys Lys Gly Ser Gly Tyr Ser Phe Thr Ser Tyr
            20                  25                  30

Trp Ile Gly Trp Val His Gln Met Pro Gly Lys Gly Leu Glu Trp Met
        35                  40                  45

Gly Ile Ile Tyr Pro Gly Asp Ser Asp Thr Arg Tyr Ser Pro Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Arg Asp Gln Asn Cys Gly Gly Asp Cys Tyr Ala Asp Ala Phe
            100                 105                 110

Asp Ile Trp Gly Gln Gly Thr Met Val Thr Val Ser Ser
        115                 120                 125


<210>  217
<211>  26
<212>  PRT
<213>  Homo sapiens

<400>  217

Asp Ile Gln Met Thr Gln Ser Pro Phe Ser Leu Ser Ala Ser Val Gly
1               5                   10                  15

Asp Thr Val Thr Ile Thr Cys Arg Ala Ser
            20                  25


<210>  218
<211>  6
<212>  PRT
<213>  Homo sapiens

<400>  218

Gln Gly Val Ser Ser Trp

1               5


<210>  219
<211>  17
<212>  PRT
<213>  Homo sapiens

<400>  219

Leu Ala Trp Tyr Gln Gln Lys Pro Asp Lys Ala Pro Lys Ser Leu Ile
1               5                   10                  15


Tyr




<210>  220
<211>  3
<212>  PRT
<213>  Homo sapiens

<400>  220

Ala Ala Ser
1


<210>  221
<211>  36
<212>  PRT
<213>  Homo sapiens

<400>  221

Ser Leu Gln Ser Gly Val Pro Ser Arg Phe Ser Gly Ser Gly Ser Gly
1               5                   10                  15


Thr Asp Phe Thr Leu Thr Ile Thr Ser Leu Gln Pro Glu Asp Phe Ala
            20                  25                  30


Thr Tyr Tyr Cys
        35


<210>  222
<211>  9
<212>  PRT
<213>  Homo sapiens

<400>  222

Leu Gln Asp Phe Asp Tyr Pro Tyr Thr
1               5


<210>  223
<211>  10
<212>  PRT
<213>  Homo sapiens

<400>  223

Phe Gly Gln Gly Thr Lys Leu Asp Ile Lys
1               5                   10


<210>  224
<211>  107
<212>  PRT
<213>  Homo sapiens

<400>  224

Asp Ile Gln Met Thr Gln Ser Pro Phe Ser Leu Ser Ala Ser Val Gly
1               5                   10                  15

Asp Thr Val Thr Ile Thr Cys Arg Ala Ser Gln Gly Val Ser Ser Trp
            20                  25                  30

Leu Ala Trp Tyr Gln Gln Lys Pro Asp Lys Ala Pro Lys Ser Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Thr Ser Leu Gln Pro
65                  70                  75                  80

Glu Asp Phe Ala Thr Tyr Tyr Cys Leu Gln Asp Phe Asp Tyr Pro Tyr
                85                  90                  95

Thr Phe Gly Gln Gly Thr Lys Leu Asp Ile Lys
            100                 105


<210>  225
<211>  25
<212>  PRT
<213>  Homo sapiens

<400>  225

Glu Val Gln Leu Val Glu Ser Gly Gly Asp Leu Val Gln Pro Gly Arg
1               5                   10                  15

Ser Leu Arg Leu Ser Cys Ala Ala Ser
            20                  25


<210>  226
<211>  8
<212>  PRT
<213>  Homo sapiens

<400>  226

Gly Phe Thr Phe Glu Asp Tyr Ala
1               5

<210>  227
<211>  17
<212>  PRT
<213>  Homo sapiens

<400>  227

Met His Trp Val Arg Gln Ala Pro Gly Lys Gly Leu Glu Trp Val Ser
1               5                   10                  15

Ser

<210>  228
<211>  8
<212>  PRT
<213>  Homo sapiens

<400>  228

Ile Asn Trp Ser Ser Gly Ser Ile
1               5

<210>  229
<211>  38
<212>  PRT
<213>  Homo sapiens

<400>  229

Val Tyr Ala Asp Ser Val Lys Gly Arg Phe Thr Ile Ser Arg Asp Asn
1               5                   10                  15

Ala Lys Asn Ser Leu Tyr Leu Gln Met Asn Ser Leu Arg Pro Asp Asp
            20                  25                  30

Thr Ala Leu Tyr Tyr Cys
        35

<210>  230
<211>  16
<212>  PRT
<213>  Homo sapiens

<400>  230

Ala Lys Asp Ser Gly Pro Tyr Gly Ser Gly His Tyr Gly Met Asp Val
1               5                   10                  15

<210>  231
<211>  11

<212>  PRT
<213>  Homo sapiens

<400>  231

Trp Gly Gln Gly Thr Thr Val Thr Val Ser Ser
1               5                   10


<210>  232
<211>  123
<212>  PRT
<213>  Homo sapiens

<400>  232

Glu Val Gln Leu Val Glu Ser Gly Gly Asp Leu Val Gln Pro Gly Arg
1               5                   10                  15

Ser Leu Arg Leu Ser Cys Ala Ala Ser Gly Phe Thr Phe Glu Asp Tyr
            20                  25                  30

Ala Met His Trp Val Arg Gln Ala Pro Gly Lys Gly Leu Glu Trp Val
        35                  40                  45

Ser Ser Ile Asn Trp Ser Ser Gly Ser Ile Val Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Pro Asp Asp Thr Ala Leu Tyr Tyr Cys
                85                  90                  95

Ala Lys Asp Ser Gly Pro Tyr Gly Ser Gly His Tyr Gly Met Asp Val
            100                 105                 110

Trp Gly Gln Gly Thr Thr Val Thr Val Ser Ser
        115                 120


<210>  233
<211>  26
<212>  PRT
<213>  Homo sapiens

<400>  233

Glu Ile Val Leu Thr Gln Ser Pro Asp Phe Gln Ser Val Thr Pro Arg
1               5                   10                  15

Glu Glu Val Thr Ile Thr Cys Arg Ala Ser
            20                  25

<210>  234
<211>  6
<212>  PRT
<213>  Homo sapiens

<400>  234

Gln Ser Ile Gly Ser Asp
1               5



<210>  235
<211>  17
<212>  PRT
<213>  Homo sapiens

<400>  235

Leu His Trp Tyr Gln Gln Arg Pro Asp Gln Pro Pro Arg Leu Leu Val
1               5                   10                  15


Lys




<210>  236
<211>  3
<212>  PRT
<213>  Homo sapiens

<400>  236

Tyr Ala Ser
1



<210>  237
<211>  36
<212>  PRT
<213>  Homo sapiens

<400>  237

Gln Ser Leu Ser Gly Val Pro Ser Arg Phe Arg Gly Ser Gly Ser Gly
1               5                   10                  15


Thr Asp Phe Thr Leu Thr Ile Asp Ser Pro Gln Pro Asp Asp Ile Ala
            20                  25                  30


Thr Tyr Tyr Cys
        35


<210>  238
<211>  9
<212>  PRT
<213>  Homo sapiens

<400>  238

His Gln Thr Ser Ala Ser Pro Trp Thr
1               5

<210>  239
<211>  10
<212>  PRT
<213>  Homo sapiens

<400>  239

Phe Gly Gln Gly Thr Lys Leu Glu Ile Lys
1               5                   10

<210>  240
<211>  107
<212>  PRT
<213>  Homo sapiens

<400>  240

Glu Ile Val Leu Thr Gln Ser Pro Asp Phe Gln Ser Val Thr Pro Arg
1               5                   10                  15

Glu Glu Val Thr Ile Thr Cys Arg Ala Ser Gln Ser Ile Gly Ser Asp
            20                  25                  30

Leu His Trp Tyr Gln Gln Arg Pro Asp Gln Pro Pro Arg Leu Leu Val
        35                  40                  45

Lys Tyr Ala Ser Gln Ser Leu Ser Gly Val Pro Ser Arg Phe Arg Gly
    50                  55                  60

Ser Gly Ser Gly Thr Asp Phe Thr Leu Thr Ile Asp Ser Pro Gln Pro
65                  70                  75                  80

Asp Asp Ile Ala Thr Tyr Tyr Cys His Gln Thr Ser Ala Ser Pro Trp
                85                  90                  95

Thr Phe Gly Gln Gly Thr Lys Leu Glu Ile Lys
            100                 105

<210>  241
<211>  25
<212>  PRT
<213>  Homo sapiens

<400>  241

Gln Val Gln Leu Val Gln Ser Gly Ala Glu Val Lys Lys Pro Gly Ala
1               5                   10                  15

Ser Val Lys Val Ser Cys Lys Val Ser
            20                  25

<210>  242
<211>  8
<212>  PRT
<213>  Homo sapiens

<400>  242

Gly Tyr Thr Leu Thr Glu Leu Ser
1               5


<210>  243
<211>  17
<212>  PRT
<213>  Homo sapiens

<400>  243

Met His Trp Val Arg Gln Ala Pro Gly Lys Gly Leu Glu Trp Met Gly
1               5                   10                  15


Gly


<210>  244
<211>  8
<212>  PRT
<213>  Homo sapiens

<400>  244

Phe Asp Pro Glu Asp Gly Glu Thr
1               5


<210>  245
<211>  38
<212>  PRT
<213>  Homo sapiens

<400>  245

Ile Tyr Ala Gln Lys Phe Gln Gly Arg Val Thr Met Thr Glu Asp Thr
1               5                   10                  15


Ser Thr Asp Thr Ala Tyr Met Glu Leu Ser Ser Leu Arg Ser Glu Asp
            20                  25                  30


Thr Ala Val Tyr Tyr Cys
        35


<210>  246
<211>  18
<212>  PRT
<213>  Homo sapiens

<400>  246

Ala Thr Gly Arg Tyr Cys Ser Gly Gly Ser Cys Tyr Pro Asp Ala Phe
1               5                   10                  15

Asp Ile

<210>  247
<211>  11
<212>  PRT
<213>  Homo sapiens

<400>  247

Trp Gly Gln Gly Thr Met Val Thr Val Ser Ser
1               5                   10

<210>  248
<211>  125
<212>  PRT
<213>  Homo sapiens

<400>  248

Gln Val Gln Leu Val Gln Ser Gly Ala Glu Val Lys Lys Pro Gly Ala
1               5                   10                  15

Ser Val Lys Val Ser Cys Lys Val Ser Gly Tyr Thr Leu Thr Glu Leu
            20                  25                  30

Ser Met His Trp Val Arg Gln Ala Pro Gly Lys Gly Leu Glu Trp Met
        35                  40                  45

Gly Gly Phe Asp Pro Glu Asp Gly Glu Thr Ile Tyr Ala Gln Lys Phe
    50                  55                  60

Gln Gly Arg Val Thr Met Thr Glu Asp Thr Ser Thr Asp Thr Ala Tyr
65                  70                  75                  80

Met Glu Leu Ser Ser Leu Arg Ser Glu Asp Thr Ala Val Tyr Tyr Cys
                85                  90                  95

Ala Thr Gly Arg Tyr Cys Ser Gly Gly Ser Cys Tyr Pro Asp Ala Phe
            100                 105                 110

Asp Ile Trp Gly Gln Gly Thr Met Val Thr Val Ser Ser
        115                 120                 125

<210>  249
<211>  26
<212>  PRT

<213>  Homo sapiens

<400>  249

Glu Ile Val Met Thr Gln Ser Pro His Ser Leu Ala Val Ser Leu Gly
1               5                   10                  15

Glu Arg Ala Thr Ile Tyr Cys Lys Ser Ser
            20                  25

<210>  250
<211>  12
<212>  PRT
<213>  Homo sapiens

<400>  250

Gln Thr Val Leu Tyr Ser Ser Asn Asn Arg Asn Tyr
1               5                   10

<210>  251
<211>  17
<212>  PRT
<213>  Homo sapiens

<400>  251

Leu Ala Trp Tyr Gln Gln Lys Pro Arg Gln Pro Pro Lys Leu Leu Leu
1               5                   10                  15

Ser

<210>  252
<211>  3
<212>  PRT
<213>  Homo sapiens

<400>  252

Trp Ala Ser
1

<210>  253
<211>  36
<212>  PRT
<213>  Homo sapiens

<400>  253

Thr Arg Glu Ser Gly Val Pro Glu Arg Phe Ser Gly Ser Gly Ser Gly
1               5                   10                  15

Thr Glu Phe Thr Leu Thr Ile Ser Ala Leu Gln Ala Glu Asp Val Ala
            20                  25                  30

Val Tyr Tyr Cys
        35

<210>  254
<211>  9
<212>  PRT
<213>  Homo sapiens

<400>  254

Gln Gln Tyr Tyr Ser Ser Pro Gln Thr
1               5

<210>  255
<211>  10
<212>  PRT
<213>  Homo sapiens

<400>  255

Phe Gly Gln Gly Thr Lys Leu Glu Ile Lys
1               5                   10

<210>  256
<211>  113
<212>  PRT
<213>  Homo sapiens

<400>  256

Glu Ile Val Met Thr Gln Ser Pro His Ser Leu Ala Val Ser Leu Gly
1               5                   10                  15

Glu Arg Ala Thr Ile Tyr Cys Lys Ser Ser Gln Thr Val Leu Tyr Ser
            20                  25                  30

Ser Asn Asn Arg Asn Tyr Leu Ala Trp Tyr Gln Gln Lys Pro Arg Gln
        35                  40                  45

Pro Pro Lys Leu Leu Leu Ser Trp Ala Ser Thr Arg Glu Ser Gly Val
    50                  55                  60

Pro Glu Arg Phe Ser Gly Ser Gly Ser Gly Thr Glu Phe Thr Leu Thr
65                  70                  75                  80

Ile Ser Ala Leu Gln Ala Glu Asp Val Ala Val Tyr Tyr Cys Gln Gln
                85                  90                  95

Tyr Tyr Ser Ser Pro Gln Thr Phe Gly Gln Gly Thr Lys Leu Glu Ile
            100                 105                 110

Lys

<210>  257
<211>  25
<212>  PRT
<213>  Camelus dromedarius

<400>  257

<210>  258
<211>  8
<212>  PRT
<213>  Camelus dromedarius

<400>  258

Gly Phe Thr Phe Ser Asn Tyr Ala
1               5

<210>  259
<211>  17
<212>  PRT
<213>  Camelus dromedarius

<400>  259

Met Thr Trp Val Arg Gln Ala Pro Gly Lys Gly Leu Glu Trp Val Ser
1               5                   10                  15

Gly

<210>  260
<211>  8
<212>  PRT
<213>  Camelus dromedarius

<400>  260

Ile Ser Gly Ser Gly Ser Gly Ala
1               5

<210>  261
<211>  37
<212>  PRT
<213>  Camelus dromedarius

<400>  261

Tyr Tyr Ala Asp Ser Val Lys Gly Arg Phe Thr Ile Ser Arg Asp Asn

1                   5                   10                  15

Ser Gln Asn Thr Leu Tyr Leu Gln Met Asn Ser Leu Arg Ala Glu Asp
                20                  25                  30

Thr Ala Val Tyr Tyr
        35

<210>  262
<211>  16
<212>  PRT
<213>  Camelus dromedarius

<400>  262

Cys Ala Lys Asp Arg Ser Gly Ser Ser Pro Pro Arg Ser Phe Asn Tyr
1                   5                   10                  15

<210>  263
<211>  11
<212>  PRT
<213>  Camelus dromedarius

<400>  263

Trp Gly Gln Gly Thr Thr Val Thr Val Ser Ser
1                   5                   10

<210>  264
<211>  122
<212>  PRT
<213>  Camelus dromedarius

<400>  264

Glu Val Gln Leu Val Glu Ser Gly Gly Gly Leu Val Gln Pro Gly Gly
1                   5                   10                  15

Ser Leu Arg Leu Ser Cys Ala Ala Ser Gly Phe Thr Phe Ser Asn Tyr
                20                  25                  30

Ala Met Thr Trp Val Arg Gln Ala Pro Gly Lys Gly Leu Glu Trp Val
        35                  40                  45

Ser Gly Ile Ser Gly Ser Gly Ser Gly Ala Tyr Tyr Ala Asp Ser Val
    50                  55                  60

Lys Gly Arg Phe Thr Ile Ser Arg Asp Asn Ser Gln Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Arg Ser Gly Ser Ser Pro Pro Arg Ser Phe Asn Tyr Trp
            100                 105                 110

Gly Gln Gly Thr Thr Val Thr Val Ser Ser
        115                 120

<210>  265
<211>  25
<212>  PRT
<213>  Camelus dromedarius

<400>  265

Glu Val Gln Leu Val Glu Ser Gly Ala Glu Val Lys Lys Pro Gly Glu
1               5                   10                  15

Ser Leu Arg Ile Ser Cys Lys Ala Ser
            20                  25

<210>  266
<211>  7
<212>  PRT
<213>  Camelus dromedarius

<400>  266

Gly Tyr Thr Phe Ser Ser Tyr
1               5

<210>  267
<211>  18
<212>  PRT
<213>  Camelus dromedarius

<400>  267

Trp Ile Ala Trp Val Arg Gln Met Pro Gly Lys Gly Leu Glu Trp Met
1               5                   10                  15

Gly Ile

<210>  268
<211>  8
<212>  PRT
<213>  Camelus dromedarius

<400>  268

Ile Tyr Pro Gly Asp Phe Asp Thr
1               5

<210>  269
<211>  37

<212>  PRT
<213>  Camelus dromedarius

<400>  269

Lys Tyr Ser Pro Ser Phe Arg Gly Gln Val Thr Ile Ser Ala Asp Arg
1               5                   10                  15

Ser Ile Ser Thr Ala Tyr Leu Gln Trp Ser Ser Leu Lys Ala Ser Asp
            20                  25                  30

Thr Ala Ile Tyr Tyr
        35

<210>  270
<211>  16
<212>  PRT
<213>  Camelus dromedarius

<400>  270

Cys Ala Arg Ser Pro Ser Gly Thr Thr Tyr Trp Tyr His Phe Asp Asp
1               5                   10                  15

<210>  271
<211>  11
<212>  PRT
<213>  Camelus dromedarius

<400>  271

Trp Gly Arg Gly Thr Thr Val Thr Val Ser Ser
1               5                   10

<210>  272
<211>  122
<212>  PRT
<213>  Camelus dromedarius

<400>  272

Glu Val Gln Leu Val Glu Ser Gly Ala Glu Val Lys Lys Pro Gly Glu
1               5                   10                  15

Ser Leu Arg Ile Ser Cys Lys Ala Ser Gly Tyr Thr Phe Ser Ser Tyr
            20                  25                  30

Trp Ile Ala Trp Val Arg Gln Met Pro Gly Lys Gly Leu Glu Trp Met
        35                  40                  45

Gly Ile Ile Tyr Pro Gly Asp Phe Asp Thr Lys Tyr Ser Pro Ser Phe
    50                  55                  60

Arg Gly Gln Val Thr Ile Ser Ala Asp Arg Ser Ile Ser Thr Ala Tyr

65                  70                  75                  80

Leu Gln Trp Ser Ser Leu Lys Ala Ser Asp Thr Ala Ile Tyr Tyr Cys
                85                  90                  95

Ala Arg Ser Pro Ser Gly Thr Thr Tyr Trp Tyr His Phe Asp Asp Trp
            100                 105                 110

Gly Arg Gly Thr Thr Val Thr Val Ser Ser
        115                 120

<210>  273
<211>  25
<212>  PRT
<213>  Camelus dromedarius

<400>  273

Gln Val Gln Leu Val Glu Ser Gly Gly Gly Ser Val Gln Ala Gly Arg
1               5                   10                  15

Ser Leu Arg Leu Ser Cys Val Ile Ser
            20                  25

<210>  274
<211>  8
<212>  PRT
<213>  Camelus dromedarius

<400>  274

Gly Gly Thr Leu Ser Thr Tyr Ala
1               5

<210>  275
<211>  16
<212>  PRT
<213>  Camelus dromedarius

<400>  275

Met Gly Trp Phe Arg Gln Ala Pro Gly Lys Glu Cys Glu Leu Val Ser
1               5                   10                  15

<210>  276
<211>  8
<212>  PRT
<213>  Camelus dromedarius

<400>  276

Ile Leu Asn Arg Asp Gly Ser Thr
1               5

<210>  277
<211>  37
<212>  PRT
<213>  Camelus dromedarius

<400>  277

Lys Tyr Ala Asp Ser Val Lys Gly Arg Phe Thr Ile Ser Arg Asp Asn
1               5                   10                  15

Ala Lys Asn Met Val Tyr Leu Gln Met Asn Asn Leu Asn Pro Glu Asp
            20                  25                  30

Thr Ala Met Tyr Tyr
        35

<210>  278
<211>  17
<212>  PRT
<213>  Camelus dromedarius

<400>  278

Cys Ala Pro Arg Thr Leu Pro Pro Pro Thr Cys Pro Gly Arg Asn Thr
1               5                   10                  15

Tyr

<210>  279
<211>  11
<212>  PRT
<213>  Camelus dromedarius

<400>  279

Trp Gly His Gly Thr Leu Val Thr Val Ser Ser
1               5                   10

<210>  280
<211>  122
<212>  PRT
<213>  Camelus dromedarius

<400>  280

Gln Val Gln Leu Val Glu Ser Gly Gly Gly Ser Val Gln Ala Gly Arg
1               5                   10                  15

Ser Leu Arg Leu Ser Cys Val Ile Ser Gly Gly Thr Leu Ser Thr Tyr
            20                  25                  30

Ala Met Gly Trp Phe Arg Gln Ala Pro Gly Lys Glu Cys Glu Leu Val
        35                  40                  45

Ser Ile Leu Asn Arg Asp Gly Ser Thr Lys Tyr Ala Asp Ser Val Lys
    50                  55                  60

Gly Arg Phe Thr Ile Ser Arg Asp Asn Ala Lys Asn Met Val Tyr Leu
65                  70                  75                  80

Gln Met Asn Asn Leu Asn Pro Glu Asp Thr Ala Met Tyr Tyr Cys Ala
                85                  90                  95

Pro Arg Thr Leu Pro Pro Pro Thr Cys Pro Gly Arg Asn Thr Tyr Trp
            100                 105                 110

Gly His Gly Thr Leu Val Thr Val Ser Ser
        115                 120

<210>  281
<211>  25
<212>  PRT
<213>  Camelus dromedarius

<400>  281

Glu Val Gln Leu Val Glu Ser Ala Gly Gly Leu Val Gln Pro Gly Gly
1               5                   10                  15

Ser Leu Arg Leu Ser Cys Ala Ala Ser
            20                  25

<210>  282
<211>  8
<212>  PRT
<213>  Camelus dromedarius

<400>  282

Gly Phe Thr Phe Ser Thr Tyr Tyr
1               5

<210>  283
<211>  17
<212>  PRT
<213>  Camelus dromedarius

<400>  283

Met Ser Trp Val Arg Gln Ala Pro Gly Lys Gly Leu Glu Trp Val Ser
1               5                   10                  15

Gly

<210>  284

<211>  7
<212>  PRT
<213>  Camelus dromedarius

<400>  284

Ile Tyr Ser Asp Gly Val Thr
1               5


<210>  285
<211>  37
<212>  PRT
<213>  Camelus dromedarius

<400>  285

Val Tyr Gly Asp Ser Val Lys Gly Arg Phe Thr Ile Ser Arg Asp Asn
1               5                   10                  15


Ala Lys Asn Thr Leu Tyr Leu Gln Met Asn Ser Leu Lys Pro Glu Asp
            20                  25                  30


Thr Ala Val Tyr Tyr
        35


<210>  286
<211>  17
<212>  PRT
<213>  Camelus dromedarius

<400>  286

Cys Ala Gly Gly Pro Ile Gly Gly Ser Trp Asp Gly Ala Asp Phe Ser
1               5                   10                  15


Tyr


<210>  287
<211>  11
<212>  PRT
<213>  Camelus dromedarius

<400>  287

Trp Ala Gln Gly Thr Met Val Thr Val Ser Ser
1               5                   10


<210>  288
<211>  122
<212>  PRT
<213>  Camelus dromedarius

<400>  288

Glu Val Gln Leu Val Glu Ser Ala Gly Gly Leu Val Gln Pro Gly Gly

1               5                   10                  15

Ser Leu Arg Leu Ser Cys Ala Ala Ser Gly Phe Thr Phe Ser Thr Tyr
            20                  25                  30

Tyr Met Ser Trp Val Arg Gln Ala Pro Gly Lys Gly Leu Glu Trp Val
        35                  40                  45

Ser Gly Ile Tyr Ser Asp Gly Val Thr Val Tyr Gly Asp Ser Val Lys
    50                  55                  60

Gly Arg Phe Thr Ile Ser Arg Asp Asn Ala Lys Asn Thr Leu Tyr Leu
65                  70                  75                  80

Gln Met Asn Ser Leu Lys Pro Glu Asp Thr Ala Val Tyr Tyr Cys Ala
                85                  90                  95

Gly Gly Pro Ile Gly Gly Ser Trp Asp Gly Ala Asp Phe Ser Tyr Trp
            100                 105                 110

Ala Gln Gly Thr Met Val Thr Val Ser Ser
        115                 120

<210>  289
<211>  25
<212>  PRT
<213>  Camelus dromedarius

<400>  289

Gln Val Gln Leu Val Glu Ser Gly Gly Gly Ser Val Gln Ala Gly Gly
1               5                   10                  15

Ser Leu Arg Leu Ser Cys Ala Ala Ser
            20                  25

<210>  290
<211>  8
<212>  PRT
<213>  Camelus dromedarius

<400>  290

Gly Tyr Thr Tyr Cys Ser Tyr Asp
1               5

<210>  291
<211>  17
<212>  PRT
<213>  Camelus dromedarius

<400>  291

Met Ser Trp Tyr Arg Gln Ala Pro Gly Lys Glu Arg Glu Phe Val Ser
1               5                   10                  15

Ala

<210>  292
<211>  7
<212>  PRT
<213>  Camelus dromedarius

<400>  292

Ile Asp Ser Asp Gly Ser Thr
1               5

<210>  293
<211>  37
<212>  PRT
<213>  Camelus dromedarius

<400>  293

Ser Tyr Ala Asp Ser Val Lys Gly Arg Phe Thr Ile Ser Gln Gly Asn
1               5                   10                  15

Ala Lys Asn Thr Val Tyr Leu Gln Met Asn Ser Leu Lys Pro Glu Asp
            20                  25                  30

Thr Ala Met Tyr Tyr
        35

<210>  294
<211>  21
<212>  PRT
<213>  Camelus dromedarius

<400>  294

Cys Lys Thr Gly Phe Ser Asp Pro Leu Arg Pro Gly Ser Asn Pro Cys
1               5                   10                  15

Asn Ala Leu Asp Ala
            20

<210>  295
<211>  11
<212>  PRT
<213>  Camelus dromedarius

<400>  295

Trp Gly Gln Gly Thr Leu Val Thr Val Ser Ser
1               5                   10

<210>  296
<211>  126
<212>  PRT
<213>  Camelus dromedarius

<400>  296

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Cys Ser Tyr
            20                  25                  30

Asp Met Ser Trp Tyr Arg Gln Ala Pro Gly Lys Glu Arg Glu Phe Val
        35                  40                  45

Ser Ala Ile Asp Ser Asp Gly Ser Thr Ser Tyr Ala Asp Ser Val Lys
    50                  55                  60

Gly Arg Phe Thr Ile Ser Gln Gly Asn Ala Lys Asn Thr Val Tyr Leu
65                  70                  75                  80

Gln Met Asn Ser Leu Lys Pro Glu Asp Thr Ala Met Tyr Tyr Cys Lys
                85                  90                  95

Thr Gly Phe Ser Asp Pro Leu Arg Pro Gly Ser Asn Pro Cys Asn Ala
            100                 105                 110

Leu Asp Ala Trp Gly Gln Gly Thr Leu Val Thr Val Ser Ser
        115                 120                 125


<210>  297
<211>  25
<212>  PRT
<213>  Camelus dromedarius

<400>  297

Glu Val Gln Leu Val Glu Ser Gly Gly Gly Ser Val Gln Ala Gly Gly
1               5                   10                  15

Ser Leu Arg Leu Ser Cys Val Val Ser
            20                  25


<210>  298
<211>  9
<212>  PRT
<213>  Camelus dromedarius

<400>  298

Gly Ser Ser Asp Cys Thr Tyr Asn Ser
1               5

<210>  299
<211>  17
<212>  PRT
<213>  Camelus dromedarius

<400>  299

Met Ser Trp Tyr Arg Gln Ala Pro Gly Lys Glu Arg Glu Phe Val Ser
1               5                   10                  15

Gly

<210>  300
<211>  7
<212>  PRT
<213>  Camelus dromedarius

<400>  300

Ile Arg Asp Asp Gly Arg Thr
1               5

<210>  301
<211>  37
<212>  PRT
<213>  Camelus dromedarius

<400>  301

Ser Tyr Arg Asp Ser Val Lys Gly Arg Phe Thr Ile Ser Gln Asp Gly
1               5                   10                  15

Ala Lys Asn Thr Met Tyr Leu Gln Met Asp Ser Leu Lys Pro Glu Asp
            20                  25                  30

Thr Ala Val Tyr Tyr
        35

<210>  302
<211>  15
<212>  PRT
<213>  Camelus dromedarius

<400>  302

Cys Thr Arg Ala Tyr Glu Tyr Gly Leu Pro Ser Pro Phe Gly Tyr
1               5                   10                  15

<210>  303
<211>  11
<212>  PRT

<213>  Camelus dromedarius

<400>  303

Leu Gly Gln Gly Thr Met Val Thr Val Ser Ser
1               5                   10


<210>  304
<211>  121
<212>  PRT
<213>  Camelus dromedarius

<400>  304

Glu Val Gln Leu Val Glu Ser Gly Gly Gly Ser Val Gln Ala Gly Gly
1               5                   10                  15


Ser Leu Arg Leu Ser Cys Val Val Ser Gly Ser Ser Asp Cys Thr Tyr
            20                  25                  30


Asn Ser Met Ser Trp Tyr Arg Gln Ala Pro Gly Lys Glu Arg Glu Phe
        35                  40                  45


Val Ser Gly Ile Arg Asp Asp Gly Arg Thr Ser Tyr Arg Asp Ser Val
    50                  55                  60


Lys Gly Arg Phe Thr Ile Ser Gln Asp Gly Ala Lys Asn Thr Met Tyr
65                  70                  75                  80


Leu Gln Met Asp Ser Leu Lys Pro Glu Asp Thr Ala Val Tyr Tyr Cys
                85                  90                  95


Thr Arg Ala Tyr Glu Tyr Gly Leu Pro Ser Pro Phe Gly Tyr Leu Gly
            100                 105                 110


Gln Gly Thr Met Val Thr Val Ser Ser
        115                 120


<210>  305
<211>  25
<212>  PRT
<213>  Camelus dromedarius

<400>  305

Glu Val Gln Leu Val Glu Ser Gly Ala Glu Val Lys Lys Pro Gly Glu
1               5                   10                  15


Ser Leu Arg Ile Ser Cys Lys Ala Ser
            20                  25


<210>  306

<211>  7
<212>  PRT
<213>  Camelus dromedarius

<400>  306

Gly Tyr Thr Phe Ser Ser Tyr
1               5

<210>  307
<211>  18
<212>  PRT
<213>  Camelus dromedarius

<400>  307

Trp Ile Ala Trp Val Arg Gln Met Pro Gly Lys Gly Leu Glu Trp Met
1               5                   10                  15

Gly Ile

<210>  308
<211>  8
<212>  PRT
<213>  Camelus dromedarius

<400>  308

Ile Tyr Pro Gly Asp Phe Asp Thr
1               5

<210>  309
<211>  37
<212>  PRT
<213>  Camelus dromedarius

<400>  309

Lys Tyr Ser Pro Ser Phe Arg Gly Gln Val Thr Ile Ser Ala Asp Arg
1               5                   10                  15

Ser Ile Ser Thr Ala Tyr Leu Gln Trp Ser Ser Leu Lys Ala Ser Asp
            20                  25                  30

Thr Ala Ile Tyr Tyr
        35

<210>  310
<211>  16
<212>  PRT
<213>  Camelus dromedarius

<400>  310

Cys Ala Arg Ser Pro Ser Gly Thr Thr Tyr Trp Tyr His Phe Asp Asp

1               5                   10                  15


<210>  311
<211>  11
<212>  PRT
<213>  Camelus dromedarius

<400>  311

Trp Gly Arg Gly Thr Thr Val Thr Val Ser Ser
1               5                   10


<210>  312
<211>  122
<212>  PRT
<213>  Camelus dromedarius

<400>  312

Glu Val Gln Leu Val Glu Ser Gly Ala Glu Val Lys Lys Pro Gly Glu
1               5                   10                  15


Ser Leu Arg Ile Ser Cys Lys Ala Ser Gly Tyr Thr Phe Ser Ser Tyr
            20                  25                  30


Trp Ile Ala Trp Val Arg Gln Met Pro Gly Lys Gly Leu Glu Trp Met
        35                  40                  45


Gly Ile Ile Tyr Pro Gly Asp Phe Asp Thr Lys Tyr Ser Pro Ser Phe
    50                  55                  60


Arg Gly Gln Val Thr Ile Ser Ala Asp Arg Ser Ile Ser Thr Ala Tyr
65                  70                  75                  80


Leu Gln Trp Ser Ser Leu Lys Ala Ser Asp Thr Ala Ile Tyr Tyr Cys
                85                  90                  95


Ala Arg Ser Pro Ser Gly Thr Thr Tyr Trp Tyr His Phe Asp Asp Trp
            100                 105                 110


Gly Arg Gly Thr Thr Val Thr Val Ser Ser
        115                 120


<210>  313
<211>  25
<212>  PRT
<213>  Camelus dromedarius

<400>  313

Gln Val Gln Leu Val Glu Ser Gly Gly Gly Leu Val Gln Pro Gly Gly
1               5                   10                  15

Ser Leu Thr Leu Ser Cys Ala Ala Ser
            20                  25

<210>  314
<211>  8
<212>  PRT
<213>  Camelus dromedarius

<400>  314

Gly Phe Thr Phe Ser Asn Tyr Val
1               5

<210>  315
<211>  17
<212>  PRT
<213>  Camelus dromedarius

<400>  315

Met Ser Trp Val Arg Gln Ala Pro Gly Lys Gly Leu Glu Trp Val Ser
1               5                   10                  15

Thr

<210>  316
<211>  8
<212>  PRT
<213>  Camelus dromedarius

<400>  316

Ile Glu Ser Gly Gly Gly Thr Thr
1               5

<210>  317
<211>  37
<212>  PRT
<213>  Camelus dromedarius

<400>  317

Tyr Tyr Ala Asp Ser Val Lys Gly Arg Phe Thr Ile Thr Arg Asp Asn
1               5                   10                  15

Ala Lys Asn Thr Leu Tyr Leu Gln Leu Asn Ser Leu Lys Ser Glu Asp
            20                  25                  30

Thr Ala Met Tyr Tyr
        35

<210>  318
<211>  13

<212>  PRT
<213>  Camelus dromedarius

<400>  318

Cys Gly Asn Phe Ile Ala Tyr Tyr Tyr Tyr Ile Asp Tyr
1               5                   10


<210>  319
<211>  11
<212>  PRT
<213>  Camelus dromedarius

<400>  319

Trp Gly Arg Gly Thr Leu Val Thr Val Ser Ser
1               5                   10


<210>  320
<211>  119
<212>  PRT
<213>  Camelus dromedarius

<400>  320

Gln Val Gln Leu Val Glu Ser Gly Gly Gly Leu Val Gln Pro Gly Gly
1               5                   10                  15


Ser Leu Thr Leu Ser Cys Ala Ala Ser Gly Phe Thr Phe Ser Asn Tyr
            20                  25                  30


Val Met Ser Trp Val Arg Gln Ala Pro Gly Lys Gly Leu Glu Trp Val
        35                  40                  45


Ser Thr Ile Glu Ser Gly Gly Gly Thr Thr Tyr Tyr Ala Asp Ser Val
    50                  55                  60


Lys Gly Arg Phe Thr Ile Thr Arg Asp Asn Ala Lys Asn Thr Leu Tyr
65                  70                  75                  80


Leu Gln Leu Asn Ser Leu Lys Ser Glu Asp Thr Ala Met Tyr Tyr Cys
                85                  90                  95


Gly Asn Phe Ile Ala Tyr Tyr Tyr Tyr Ile Asp Tyr Trp Gly Arg Gly
            100                 105                 110


Thr Leu Val Thr Val Ser Ser
        115


<210>  321
<211>  25
<212>  PRT
<213>  Camelus dromedarius

<400>  321

Gln Val Gln Leu Val Glu Ser Gly Gly Gly Ser Val Gln Ala Gly Gly
1               5                   10                  15

Ser Leu Arg Leu Ser Cys Ala Ala Ser
            20                  25

<210>  322
<211>  8
<212>  PRT
<213>  Camelus dromedarius

<400>  322

Gly Tyr Thr Tyr Ser Ser Tyr Cys
1               5

<210>  323
<211>  17
<212>  PRT
<213>  Camelus dromedarius

<400>  323

Met Gly Trp Phe Arg Gln Ala Pro Gly Lys Glu Arg Glu Gly Val Ala
1               5                   10                  15

Ala

<210>  324
<211>  8
<212>  PRT
<213>  Camelus dromedarius

<400>  324

Ile Asn Ser Gly Gly Gly Ser Thr
1               5

<210>  325
<211>  37
<212>  PRT
<213>  Camelus dromedarius

<400>  325

Tyr Tyr Ala Asp Ser Val Lys Gly Arg Phe Thr Ile Ser Gln Asp Asn
1               5                   10                  15

Ala Lys Asn Thr Val Tyr Leu Gln Met Asn Ser Leu Lys Pro Glu Asp
            20                  25                  30

Thr Ala Ile Tyr Tyr
        35

<210>  326
<211>  18
<212>  PRT
<213>  Camelus dromedarius

<400>  326

Cys Ala Ala Asp Arg Cys Ala Pro Gly Gly Ser Trp Tyr Lys Val Phe
1               5                   10                  15

Asp Tyr

<210>  327
<211>  11
<212>  PRT
<213>  Camelus dromedarius

<400>  327

Trp Gly Gln Gly Thr Leu Val Thr Val Ser Ser
1               5                   10

<210>  328
<211>  124
<212>  PRT
<213>  Camelus dromedarius

<400>  328

Gln Val Gln Leu Val Glu Ser Gly Gly Gly Ser Val Gln Ala Gly Gly
1               5                   10                  15

Ser Leu Arg Leu Ser Cys Ala Ala Ser Gly Tyr Thr Tyr Ser Ser Tyr
            20                  25                  30

Cys Met Gly Trp Phe Arg Gln Ala Pro Gly Lys Glu Arg Glu Gly Val
        35                  40                  45

Ala Ala Ile Asn Ser Gly Gly Gly Ser Thr Tyr Tyr Ala Asp Ser Val
    50                  55                  60

Lys Gly Arg Phe Thr Ile Ser Gln Asp Asn Ala Lys Asn Thr Val Tyr
65                  70                  75                  80

Leu Gln Met Asn Ser Leu Lys Pro Glu Asp Thr Ala Ile Tyr Tyr Cys
                85                  90                  95

Ala Ala Asp Arg Cys Ala Pro Gly Gly Ser Trp Tyr Lys Val Phe Asp
            100                 105                 110

Tyr Trp Gly Gln Gly Thr Leu Val Thr Val Ser Ser
        115                 120

<210>  329
<211>  25
<212>  PRT
<213>  Camelus dromedarius

<400>  329

Glu Val Gln Leu Val Glu Ser Gly Gly Gly Leu Val Lys Pro Gly Gly
1               5                   10                  15

Ser Leu Arg Leu Ser Cys Ala Ala Ser
            20                  25

<210>  330
<211>  8
<212>  PRT
<213>  Camelus dromedarius

<400>  330

Gly Phe Thr Phe Ser Ser Asn Tyr
1               5

<210>  331
<211>  17
<212>  PRT
<213>  Camelus dromedarius

<400>  331

Met Ser Trp Val Arg Gln Ala Pro Gly Lys Gly Leu Glu Trp Val Ser
1               5                   10                  15

Gly

<210>  332
<211>  8
<212>  PRT
<213>  Camelus dromedarius

<400>  332

Ile Asn Arg Asp Gly Ser Asn Thr
1               5

<210>  333
<211>  37
<212>  PRT
<213>  Camelus dromedarius

<400>  333

Tyr Tyr Ala Asp Ser Val Lys Gly Arg Phe Thr Ile Ser Arg Asp Asn
1               5                   10                  15

Ala Lys Asn Thr Leu Tyr Leu Gln Met Asn Ser Leu Lys Ser Glu Asp
            20                  25                  30

Thr Ala Leu Tyr Tyr
        35

<210>  334
<211>  18
<212>  PRT
<213>  Camelus dromedarius

<400>  334

Cys Ala Thr Ala Tyr Gly Gly Ser Trp Tyr Gly Val Pro Ala Asp Phe
1               5                   10                  15

Gly Tyr

<210>  335
<211>  11
<212>  PRT
<213>  Camelus dromedarius

<400>  335

Trp Asp Gln Gly Thr Leu Val Thr Val Ser Ser
1               5                   10

<210>  336
<211>  124
<212>  PRT
<213>  Camelus dromedarius

<400>  336

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Asn
            20                  25                  30

Tyr Met Ser Trp Val Arg Gln Ala Pro Gly Lys Gly Leu Glu Trp Val
        35                  40                  45

Ser Gly Ile Asn Arg Asp Gly Ser Asn Thr Tyr Tyr Ala Asp Ser Val
    50                  55                  60

Lys Gly Arg Phe Thr Ile Ser Arg Asp Asn Ala Lys Asn Thr Leu Tyr
65                  70                  75                  80

Leu Gln Met Asn Ser Leu Lys Ser Glu Asp Thr Ala Leu Tyr Tyr Cys
                85                  90                  95

Ala Thr Ala Tyr Gly Gly Ser Trp Tyr Gly Val Pro Ala Asp Phe Gly
            100                 105                 110

Tyr Trp Asp Gln Gly Thr Leu Val Thr Val Ser Ser
        115                 120

<210>  337
<211>  25
<212>  PRT
<213>  Camelus dromedarius

<400>  337

Glu Val Gln Leu Val Glu Ser Gly Gly Gly Leu Val Gln Pro Gly Gly
1               5                   10                  15

Ser Leu Arg Leu Ser Cys Val Ala Ser
            20                  25

<210>  338
<211>  8
<212>  PRT
<213>  Camelus dromedarius

<400>  338

Gly Val Ala Phe Ser Ile Asn Tyr
1               5

<210>  339
<211>  17
<212>  PRT
<213>  Camelus dromedarius

<400>  339

Met Ser Trp Val Arg Gln Ala Pro Gly Lys Glu Leu Asp Trp Val Ser
1               5                   10                  15

Asp

<210>  340
<211>  8
<212>  PRT
<213>  Camelus dromedarius

<400>  340

Ile Arg Met Asp Gly Thr Ser Thr
1               5

<210>  341
<211>  37
<212>  PRT
<213>  Camelus dromedarius

<400>  341

Tyr Tyr Ala Asp Ser Val Lys Gly Arg Phe Thr Ile Ser Arg Asp Asn
1               5                   10                  15

Ala Lys Asn Thr Leu Tyr Leu Gln Met Asn Ser Leu Arg Ser Asp Asp
            20                  25                  30

Thr Ala Leu Tyr Tyr
        35

<210>  342
<211>  6
<212>  PRT
<213>  Camelus dromedarius

<400>  342

Cys Leu Gly Phe Gly Tyr
1               5

<210>  343
<211>  11
<212>  PRT
<213>  Camelus dromedarius

<400>  343

Trp Gly Gln Gly Thr Thr Val Thr Val Ser Ser
1               5                   10

<210>  344
<211>  112
<212>  PRT
<213>  Camelus dromedarius

<400>  344

Glu Val Gln Leu Val Glu Ser Gly Gly Gly Leu Val Gln Pro Gly Gly
1               5                   10                  15

Ser Leu Arg Leu Ser Cys Val Ala Ser Gly Val Ala Phe Ser Ile Asn
            20                  25                  30

Tyr Met Ser Trp Val Arg Gln Ala Pro Gly Lys Glu Leu Asp Trp Val
        35                  40                  45

Ser Asp Ile Arg Met Asp Gly Thr Ser Thr Tyr Tyr Ala Asp Ser Val
    50                  55                  60

Lys Gly Arg Phe Thr Ile Ser Arg Asp Asn Ala Lys Asn Thr Leu Tyr
65                  70                  75                  80

Leu Gln Met Asn Ser Leu Arg Ser Asp Asp Thr Ala Leu Tyr Tyr Cys
                85                  90                  95

Leu Gly Phe Gly Tyr Trp Gly Gln Gly Thr Thr Val Thr Val Ser Ser
            100                 105                 110

<210>  345
<211>  25
<212>  PRT
<213>  Camelus dromedarius

<400>  345

Gln Val Gln Leu Val Glu Ser Gly Gly Gly Leu Val Gln Pro Gly Gly
1               5                   10                  15

Ser Leu Arg Leu Ser Cys Ala Ala Ser
            20                  25

<210>  346
<211>  8
<212>  PRT
<213>  Camelus dromedarius

<400>  346

Gly Phe Thr Phe Ser Asn Tyr Trp
1               5

<210>  347
<211>  17
<212>  PRT
<213>  Camelus dromedarius

<400>  347

Met Tyr Trp Val Arg Gln Ala Pro Arg Lys Gly Leu Glu Trp Ile Thr
1               5                   10                  15

Thr

<210>  348
<211>  8
<212>  PRT
<213>  Camelus dromedarius

<400>  348

Ile Asn Ser Ala Gly Gly Thr Thr
1               5

<210>  349
<211>  37
<212>  PRT
<213>  Camelus dromedarius

<400>  349

Tyr Tyr Ala Asp Ser Val Lys Gly Arg Phe Thr Ile Ser Arg Asp Asn
1               5                   10                  15

Ala Lys Asn Thr Val Tyr Leu Gln Met Asn Ser Leu Lys Pro Glu Asp
            20                  25                  30

Thr Ala Val Tyr Tyr
        35

<210>  350
<211>  16
<212>  PRT
<213>  Camelus dromedarius

<400>  350

Cys Thr Arg Asp Leu Phe Glu Ser Val Val Thr Val Thr Phe Asn Tyr
1               5                   10                  15

<210>  351
<211>  11
<212>  PRT
<213>  Camelus dromedarius

<400>  351

Trp Gly Gln Gly Thr Leu Val Thr Val Ser Ser
1               5                   10

<210>  352
<211>  122
<212>  PRT
<213>  Camelus dromedarius

<400>  352

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Trp Met Tyr Trp Val Arg Gln Ala Pro Arg Lys Gly Leu Glu Trp Ile
        35                  40                  45

Thr Thr Ile Asn Ser Ala Gly Gly Thr Thr Tyr Tyr Ala Asp Ser Val
    50                  55                  60

Lys Gly Arg Phe Thr Ile Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Thr Arg Asp Leu Phe Glu Ser Val Val Thr Val Thr Phe Asn Tyr Trp
            100                 105                 110

Gly Gln Gly Thr Leu Val Thr Val Ser Ser
        115                 120

<210>  353
<211>  25
<212>  PRT
<213>  Camelus dromedarius

<400>  353

<210>  354
<211>  8
<212>  PRT
<213>  Camelus dromedarius

<400>  354

Gly Phe Ser Phe Asn Gly His Tyr
1               5

<210>  355
<211>  16
<212>  PRT
<213>  Camelus dromedarius

<400>  355

Met Ser Val Arg Gln Ala Pro Gly Lys Gly Leu Glu Trp Val Ser Asn
1               5                   10                  15

<210>  356
<211>  8

<212>  PRT
<213>  Camelus dromedarius

<400>  356

Ile Asn Pro Asp Gly Gly Gly Lys
1               5


<210>  357
<211>  37
<212>  PRT
<213>  Camelus dromedarius

<400>  357

Tyr Tyr Ser Asn Ser Val Arg Asp Arg Phe Thr Ile Ser Arg Asp Asn
1               5                   10                  15


Ala Lys Asn Met Leu Tyr Leu Glu Met Asn Ser Leu Lys Ser Glu Asp
            20                  25                  30


Thr Ala Leu Tyr Tyr
        35


<210>  358
<211>  11
<212>  PRT
<213>  Camelus dromedarius

<400>  358

Cys Ala Lys Glu Glu Ser Tyr Ser Ile Glu Asp
1               5                   10


<210>  359
<211>  11
<212>  PRT
<213>  Camelus dromedarius

<400>  359

Trp Gly Lys Gly Thr Met Val Thr Val Ser Ser
1               5                   10


<210>  360
<211>  116
<212>  PRT
<213>  Camelus dromedarius

<400>  360

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Ser Phe Asn Gly His
            20                  25                  30

Tyr Met Ser Val Arg Gln Ala Pro Gly Lys Gly Leu Glu Trp Val Ser
        35                  40                  45

Asn Ile Asn Pro Asp Gly Gly Gly Lys Tyr Tyr Ser Asn Ser Val Arg
    50                  55                  60

Asp Arg Phe Thr Ile Ser Arg Asp Asn Ala Lys Asn Met Leu Tyr Leu
65                  70                  75                  80

Glu Met Asn Ser Leu Lys Ser Glu Asp Thr Ala Leu Tyr Tyr Cys Ala
                85                  90                  95

Lys Glu Glu Ser Tyr Ser Ile Glu Asp Trp Gly Lys Gly Thr Met Val
            100                 105                 110

Thr Val Ser Ser
        115

<210>  361
<211>  25
<212>  PRT
<213>  Camelus dromedarius

<400>  361

Gln Val Gln Leu Val Glu Ser Gly Gly Asp Leu Val Gln Pro Gly Gly
1               5                   10                  15

Ser Leu Arg Leu Ser Cys Ala Ala Ser
            20                  25

<210>  362
<211>  8
<212>  PRT
<213>  Camelus dromedarius

<400>  362

Gly Phe Thr Phe Ser Asn Arg Trp
1               5

<210>  363
<211>  17
<212>  PRT
<213>  Camelus dromedarius

<400>  363

Met Tyr Trp Val Arg Gln Ala Pro Gly Lys Gly Leu Glu Trp Val Ser
1               5                   10                  15

Val

<210>  364
<211>  8
<212>  PRT
<213>  Camelus dromedarius

<400>  364

Ile Asn Gly Gly Gly Gly Thr Thr
1               5

<210>  365
<211>  37
<212>  PRT
<213>  Camelus dromedarius

<400>  365

Tyr Cys Ala Asp Ser Val Lys Gly Arg Phe Thr Ile Ser Lys Asp Asn
1               5                   10                  15

Ala Lys Asn Thr Leu Tyr Leu Gln Met Asn Ser Leu Lys Pro Glu Asp
            20                  25                  30

Thr Ala Val Tyr Tyr
        35

<210>  366
<211>  12
<212>  PRT
<213>  Camelus dromedarius

<400>  366

Cys Ala Arg Asp Ala Gly Tyr Trp Ser Ala Asp Tyr
1               5                   10

<210>  367
<211>  11
<212>  PRT
<213>  Camelus dromedarius

<400>  367

Trp Gly Gln Gly Thr Leu Val Thr Val Ser Ser
1               5                   10

<210>  368
<211>  118
<212>  PRT
<213>  Camelus dromedarius

<400>  368

Gln Val Gln Leu Val Glu Ser Gly Gly Asp Leu Val Gln Pro Gly Gly
1               5                   10                  15

Ser Leu Arg Leu Ser Cys Ala Ala Ser Gly Phe Thr Phe Ser Asn Arg
            20                  25                  30

Trp Met Tyr Trp Val Arg Gln Ala Pro Gly Lys Gly Leu Glu Trp Val
        35                  40                  45

Ser Val Ile Asn Gly Gly Gly Gly Thr Thr Tyr Cys Ala Asp Ser Val
    50                  55                  60

Lys Gly Arg Phe Thr Ile Ser Lys Asp Asn Ala Lys Asn Thr Leu Tyr
65                  70                  75                  80

Leu Gln Met Asn Ser Leu Lys Pro Glu Asp Thr Ala Val Tyr Tyr Cys
                85                  90                  95

Ala Arg Asp Ala Gly Tyr Trp Ser Ala Asp Tyr Trp Gly Gln Gly Thr
            100                 105                 110

Leu Val Thr Val Ser Ser
        115

<210>  369
<211>  25
<212>  PRT
<213>  Camelus dromedarius

<400>  369

Glu Val Gln Leu Val Glu Ser Gly Pro Gly Leu Val Lys Pro Ser Gln
1               5                   10                  15

Thr Leu Thr Leu Thr Cys Thr Val Ser
            20                  25

<210>  370
<211>  10
<212>  PRT
<213>  Camelus dromedarius

<400>  370

Gly Gly Ser Ile Thr Thr Ser Tyr Tyr Ala
1               5                   10

<210>  371
<211>  17
<212>  PRT
<213>  Camelus dromedarius

<400>  371

Trp Ser Trp Ile Arg Gln Pro Pro Gly Lys Gly Leu Glu Trp Met Gly
1               5                   10                  15

Ala

<210>  372
<211>  7
<212>  PRT
<213>  Camelus dromedarius

<400>  372

Ile Arg Tyr Ser Gly Ser Thr
1               5

<210>  373
<211>  37
<212>  PRT
<213>  Camelus dromedarius

<400>  373

Tyr Tyr Ser Pro Ser Leu Lys Ser Arg Thr Ser Ile Ser Arg Asp Thr
1               5                   10                  15

Ser Lys Asn Gln Phe Ser Leu Gln Leu Ser Ser Val Thr Pro Glu Asp
            20                  25                  30

Thr Ala Val Tyr Tyr
        35

<210>  374
<211>  11
<212>  PRT
<213>  Camelus dromedarius

<400>  374

Cys Ala Ser Asp Val Gln Tyr Ser Met Asp Asp
1               5                   10

<210>  375
<211>  11
<212>  PRT
<213>  Camelus dromedarius

<400>  375

Trp Gly Lys Gly Thr Met Val Thr Val Ser Ser
1               5                   10

<210>  376

<211>  118
<212>  PRT
<213>  Camelus dromedarius

<400>  376

Glu Val Gln Leu Val Glu Ser Gly Pro Gly Leu Val Lys Pro Ser Gln
1               5                   10                  15

Thr Leu Thr Leu Thr Cys Thr Val Ser Gly Gly Ser Ile Thr Thr Ser
            20                  25                  30

Tyr Tyr Ala Trp Ser Trp Ile Arg Gln Pro Pro Gly Lys Gly Leu Glu
        35                  40                  45

Trp Met Gly Ala Ile Arg Tyr Ser Gly Ser Thr Tyr Tyr Ser Pro Ser
    50                  55                  60

Leu Lys Ser Arg Thr Ser Ile Ser Arg Asp Thr Ser Lys Asn Gln Phe
65                  70                  75                  80

Ser Leu Gln Leu Ser Ser Val Thr Pro Glu Asp Thr Ala Val Tyr Tyr
                85                  90                  95

Cys Ala Ser Asp Val Gln Tyr Ser Met Asp Asp Trp Gly Lys Gly Thr
            100                 105                 110

Met Val Thr Val Ser Ser
        115


<210>  377
<211>  25
<212>  PRT
<213>  Camelus dromedarius

<400>  377

Gln Met Gln Leu Val Gln Trp Gly Ala Gly Leu Leu Lys Pro Ser Glu
1               5                   10                  15

Thr Leu Ser Leu Thr Cys Ala Val Tyr
            20                  25


<210>  378
<211>  8
<212>  PRT
<213>  Camelus dromedarius

<400>  378

Gly Gly Ser Phe Ser Gly Tyr Tyr
1               5

<210>  379
<211>  17
<212>  PRT
<213>  Camelus dromedarius

<400>  379

Trp Ser Trp Ile Arg Gln Pro Pro Gly Lys Gly Leu Glu Trp Ile Gly
1               5                   10                  15

Glu

<210>  380
<211>  7
<212>  PRT
<213>  Camelus dromedarius

<400>  380

Ile Asn His Ser Gly Ser Thr
1               5

<210>  381
<211>  37
<212>  PRT
<213>  Camelus dromedarius

<400>  381

Asn Tyr Asn Pro Ser Leu Lys Ser Arg Val Thr Ile Ser Val Asp Thr
1               5                   10                  15

Ser Lys Asn Gln Phe Ser Leu Lys Leu Ser Ser Val Thr Ala Ala Asp
            20                  25                  30

Thr Ala Val Tyr Tyr
        35

<210>  382
<211>  23
<212>  PRT
<213>  Camelus dromedarius

<400>  382

Cys Ala Arg Glu Asp Arg Asp Ile Val Val Val Pro Ala Ala Ile Arg
1               5                   10                  15

Trp Gly Asn Trp Phe Asp Pro
            20

<210>  383
<211>  11

<212>  PRT
<213>  Camelus dromedarius

<400>  383

Trp Gly Gln Gly Thr Leu Val Thr Val Ser Ser
1               5                   10


<210>  384
<211>  128
<212>  PRT
<213>  Camelus dromedarius

<400>  384

Gln Met Gln Leu Val Gln Trp Gly Ala Gly Leu Leu Lys Pro Ser Glu
1               5                   10                  15

Thr Leu Ser Leu Thr Cys Ala Val Tyr Gly Gly Ser Phe Ser Gly Tyr
            20                  25                  30

Tyr Trp Ser Trp Ile Arg Gln Pro Pro Gly Lys Gly Leu Glu Trp Ile
        35                  40                  45

Gly Glu Ile Asn His Ser Gly Ser Thr Asn Tyr Asn Pro Ser Leu Lys
    50                  55                  60

Ser Arg Val Thr Ile Ser Val Asp Thr Ser Lys Asn Gln Phe Ser Leu
65                  70                  75                  80

Lys Leu Ser Ser Val Thr Ala Ala Asp Thr Ala Val Tyr Tyr Cys Ala
                85                  90                  95

Arg Glu Asp Arg Asp Ile Val Val Val Pro Ala Ala Ile Arg Trp Gly
            100                 105                 110

Asn Trp Phe Asp Pro Trp Gly Gln Gly Thr Leu Val Thr Val Ser Ser
        115                 120                 125


<210>  385
<211>  25
<212>  PRT
<213>  Camelus dromedarius

<400>  385

Glu Val Gln Leu Val Glu Ser Gly Ala Glu Val Lys Lys Pro Gly Glu
1               5                   10                  15

Ser Leu Arg Ile Ser Cys Lys Ala Ser
            20                  25

<210>  386
<211>  7
<212>  PRT
<213>  Camelus dromedarius

<400>  386

Gly Tyr Thr Phe Ser Ser Tyr
1               5

<210>  387
<211>  18
<212>  PRT
<213>  Camelus dromedarius

<400>  387

Trp Ile Ala Trp Val Arg Gln Met Pro Gly Lys Gly Leu Glu Trp Met
1               5                   10                  15

Gly Ile

<210>  388
<211>  8
<212>  PRT
<213>  Camelus dromedarius

<400>  388

Ile Tyr Pro Gly Asp Phe Asp Thr
1               5

<210>  389
<211>  37
<212>  PRT
<213>  Camelus dromedarius

<400>  389

Lys Tyr Ser Pro Ser Phe Arg Gly Gln Val Thr Ile Ser Ala Asp Arg
1               5                   10                  15

Ser Ile Ser Thr Ala Tyr Leu Gln Trp Ser Ser Leu Lys Ala Ser Asp
            20                  25                  30

Thr Ala Ile Tyr Tyr
        35

<210>  390
<211>  16
<212>  PRT
<213>  Camelus dromedarius

<400>  390

Cys Ala Arg Ser Pro Ser Gly Thr Thr Tyr Trp Tyr His Phe Gly Asp
1               5                   10                  15

<210>  391
<211>  11
<212>  PRT
<213>  Camelus dromedarius

<400>  391

Trp Gly Arg Gly Thr Thr Val Thr Val Ser Ser
1               5                   10

<210>  392
<211>  122
<212>  PRT
<213>  Camelus dromedarius

<400>  392

Glu Val Gln Leu Val Glu Ser Gly Ala Glu Val Lys Lys Pro Gly Glu
1               5                   10                  15

Ser Leu Arg Ile Ser Cys Lys Ala Ser Gly Tyr Thr Phe Ser Ser Tyr
            20                  25                  30

Trp Ile Ala Trp Val Arg Gln Met Pro Gly Lys Gly Leu Glu Trp Met
        35                  40                  45

Gly Ile Ile Tyr Pro Gly Asp Phe Asp Thr Lys Tyr Ser Pro Ser Phe
    50                  55                  60

Arg Gly Gln Val Thr Ile Ser Ala Asp Arg Ser Ile Ser Thr Ala Tyr
65                  70                  75                  80

Leu Gln Trp Ser Ser Leu Lys Ala Ser Asp Thr Ala Ile Tyr Tyr Cys
                85                  90                  95

Ala Arg Ser Pro Ser Gly Thr Thr Tyr Trp Tyr His Phe Gly Asp Trp
            100                 105                 110

Gly Arg Gly Thr Thr Val Thr Val Ser Ser
        115                 120

<210>  393
<211>  1080
<212>  DNA
<213>  Staphylococcus aureus

<400>  393
gcacatacat taatagagaa aaagaaaaaa gatggcaaag atattcaact aactattgat        60

gctaaagttc aaaagagtat ttataacaac atgaaaaatg attatggctc aggtactgct       120

atccaccctc aaacaggtga attattagca cttgtaagca caccttcata tgacgtctat 180

ccatttatgt atggcatgag taacgaagaa tataataaat taaccgaaga taaaaaagaa 240

cctctgctca acaagttcca gattacaact tcaccaggtt caactcaaaa aatattaaca 300

gcaatgattg ggttaaataa caaaacatta gacgataaaa caagttataa aatcgatggt 360

aaaggttggc aaaaagataa atcttggggt ggttacaacg ttacaagata tgaagtggta 420

aatggtaata tcgacttaaa acaagcaata gaatcatcag ataacatttt ctttgctaga 480

gtagcactcg aattaggcag taagaaattt gaaaaaggca tgaaaaaact aggtgttggt 540

gaagatatac caagtgatta tccattttat aatgctcaaa tttcaaacaa aaatttagat 600

aatgaaatat tattagctga ttcaggttac ggacaaggtg aaatactgat taacccagta 660

cagatccttt caatctatag cgcattagaa aataatggca atattaacgc acctcactta 720

ttaaaagaca cgaaaaacaa agtttggaag aaaaatatta tttccaaaga aaatatcaat 780

ctattaactg atggtatgca acaagtcgta aataaaacac ataaagaaga tatttataga 840

tcttatgcaa acttaattgg caaatccggt actgcagaac tcaaaatgaa acaaggagaa 900

actggcagac aaattgggtg gtttatatca tatgataaag ataatccaaa catgatgatg 960

gctattaatg ttaaagatgt acaagataaa ggaatggcta gctacaatgc caaaatctca 1020

ggtaaagtgt atgatgagct atatgagaac ggtaataaaa aatacgatat agatgaataa 1080

<210> 394
<211> 359
<212> PRT
<213> Staphylococcus aureus

<400> 394

Ala His Thr Leu Ile Glu Lys Lys Lys Lys Asp Gly Lys Asp Ile Gln
1               5                   10                  15

Leu Thr Ile Asp Ala Lys Val Gln Lys Ser Ile Tyr Asn Asn Met Lys
            20                  25                  30

Asn Asp Tyr Gly Ser Gly Thr Ala Ile His Pro Gln Thr Gly Glu Leu
        35                  40                  45

Leu Ala Leu Val Ser Thr Pro Ser Tyr Asp Val Tyr Pro Phe Met Tyr
    50                  55                  60

Gly Met Ser Asn Glu Glu Tyr Asn Lys Leu Thr Glu Asp Lys Lys Glu
65                  70                  75                  80

Pro Leu Leu Asn Lys Phe Gln Ile Thr Thr Ser Pro Gly Ser Thr Gln
                85                  90                  95

Lys Ile Leu Thr Ala Met Ile Gly Leu Asn Asn Lys Thr Leu Asp Asp
            100                 105                 110

Lys Thr Ser Tyr Lys Ile Asp Gly Lys Gly Trp Gln Lys Asp Lys Ser
        115                 120                 125

Trp Gly Gly Tyr Asn Val Thr Arg Tyr Glu Val Val Asn Gly Asn Ile
    130                 135                 140

Asp Leu Lys Gln Ala Ile Glu Ser Ser Asp Asn Ile Phe Phe Ala Arg
145                 150                 155                 160

Val Ala Leu Glu Leu Gly Ser Lys Lys Phe Glu Lys Gly Met Lys Lys
                165                 170                 175

Leu Gly Val Gly Glu Asp Ile Pro Ser Asp Tyr Pro Phe Tyr Asn Ala
            180                 185                 190

Gln Ile Ser Asn Lys Asn Leu Asp Asn Glu Ile Leu Leu Ala Asp Ser
        195                 200                 205

Gly Tyr Gly Gln Gly Glu Ile Leu Ile Asn Pro Val Gln Ile Leu Ser
    210                 215                 220

Ile Tyr Ser Ala Leu Glu Asn Asn Gly Asn Ile Asn Ala Pro His Leu
225                 230                 235                 240

Leu Lys Asp Thr Lys Asn Lys Val Trp Lys Lys Asn Ile Ile Ser Lys
                245                 250                 255

Glu Asn Ile Asn Leu Leu Thr Asp Gly Met Gln Gln Val Val Asn Lys
            260                 265                 270

Thr His Lys Glu Asp Ile Tyr Arg Ser Tyr Ala Asn Leu Ile Gly Lys
        275                 280                 285

Ser Gly Thr Ala Glu Leu Lys Met Lys Gln Gly Glu Thr Gly Arg Gln
    290                 295                 300

Ile Gly Trp Phe Ile Ser Tyr Asp Lys Asp Asn Pro Asn Met Met Met
305                 310                 315                 320

Ala Ile Asn Val Lys Asp Val Gln Asp Lys Gly Met Ala Ser Tyr Asn
                325                 330                 335

Ala Lys Ile Ser Gly Lys Val Tyr Asp Glu Leu Tyr Glu Asn Gly Asn

            340                 345                 350
Lys Lys Tyr Asp Ile Asp Glu
        355

## Description

### FIELD OF THE INVENTION

The invention pertains to protein binding compounds such as antibodies, nanobodies, or antigen-binding fragments thereof, targeting penicillin-binding protein 2a (PBP2a) to increase the susceptibility of resistant pathogens to β-lactam antibiotics. The invention provides protein binding compounds (PBC) specifically binding to the transpeptidase domain of PBP2a, nucleic acids encoding therefor, recombinant cells expressing them, and pharmaceutical compositions comprising such compounds of the invention. The products of the invention are preferably for use in medicine such as for treating or preventing infections of antibiotic resistant-pathogens, such as methicillin-resistant *Staphylococcus aureus* (MRSA). Furthermore, the products of the invention find use in diagnostic applications for the detection of pathogenic organisms such as MRSA. Besides, there is provided a method for the identification of therapeutics based on the binding of such candidate compounds to the transpeptidase domain of PBP2a.

### DESCRIPTION

*Staphylococcus aureus* (*S. aureus*) is a bacterium of order Bacillales and family Staphylococcaceae that is frequently found in the human respiratory tract and on the skin. Although *S*. *aureus* is not always pathogenic, it is a common cause of skin infections (e.g., boils), respiratory disease (e.g., sinusitis), and food poisoning. It has demonstrated an exceptional capacity to acquire resistance to antibacterial agents. Methicillin resistant *S. aureus* (MRSA) started to spread in the hospitals in the late 1980s and later community acquired strains (CA-MRSA) followed. It is still one of the five most common causes of hospital-acquired infections and is often the cause of postsurgical wound infections. Today MRSA pose a problem for hospital hygiene in most countries of the world.

MRSA refers to a subgroup of *Staph. aureus* that is resistant to a range of β-lactam antibiotics such as methicillin. MRSA first appeared in 1961 soon after the introduction of the antibiotic methicillin. Both Methicillin-Sensitive *Staphylococcus aureus* and MRSA have virulence/pathogenicity factors that allow for adhesion to cell surfaces and immune evasion/killing, however, the major difference is that MRSA's resistance arises from the presence of the PBP2a protein on the surface of the bacteria. PBP2a protein is encoded by the mecA gene.

In order to tackle MRSA infections there have been attempts at developing DNA vaccines against MRSA using plasmids with nucleic acid sequences that encode PBP2a or fragments thereof. For example (Ohwada *et al.*, 1999) describe the intramuscular injection of a DNA plasmid that comprises the PBP2a protein-encoding mecA gene cloned from the N315 MRSA isolate. Roth D M, et al. (Roth *et al*., 2006), and Senna J P, et al. (Senna *et al.*, 2003) report intramuscular injection was used to deliver a DNA plasmid that comprised only a 249 base pair fragment of the mecA gene cloned from the HSP-03 clinical MRSA isolate.

WO 2019/086633 discloses a treatment with inhibitors of isoprenoid lipid synthesis, such as statins or Zaragozic acid, to reduce or reverse resistance to beta-lactam antibiotics induced by low affinity penicillin-binding proteins (PBPs).

There remains a need for medication useful to prevent or treat MRSA infections.

Therefore, there is a need, from one or more of the above perspectives, for novel approaches to render pathogenic bacteria more susceptible to the treatment with antibiotics, and in particular to circumvent β-lactam antibiotic resistance. The present invention seeks to provide, in particular, novel therapeutic approaches and methods involving existing or novel compounds; for example, compounds and PBC that sensitise PBP2a towards antibiotics such as methicillin. Furthermore, the invention seeks to provide novel strategies to diagnose infections and/or resistances, as wells as screening approaches for the identification of compounds that are useful in the treatment of certain disorders. Accordingly, it is an object of the present invention to provide alternative, improved, simpler, cheaper and/or integrated means or methods that address one or more of these or other problems. Such an object underlying the present invention is solved by the subject matter as disclosed or defined anywhere herein, for example by the subject matter of the attached claims.

### BRIEF DESCRIPTION OF THE INVENTION

Generally, and by way of brief description, the main aspects of the present invention can be described as follows:
In **a first aspect**, the invention pertains a protein binding compound (PBC) specifically binding to the transpeptidase domain of penicillin binding protein 2 a (PBP2a), wherein the PBC when bound to the transpeptidase domain of PBP2a increases sensitivity of PBP2a towards a β-lactam antibiotic compared to when the PBC is not bound to the transpeptidase domain of PBP2a.
In **a second aspect**, the invention pertains to a protein binding compound (PBC), which is an isolated PBC comprising an amino acid sequence at least 80% identical to a sequence according to any one of the herein disclosed SEQ ID NO: 1 to 392
In **a third aspect**, the invention pertains to an isolated PBC, which competes with an PBC as recited in any one of claims 10 to 25 for binding to the transpeptidase domain of PBP2a, preferably of *Staphylococcus aureus,* and is capable of increasing sensitivity of said PBP2a to a β-lactam antibiotic, preferably capable of increasing of the binding of the β-lactam antibiotic to the PBP2a
In **a fourth aspect**, the invention pertains to an isolated nucleic acid encoding for a protein binding compound which is a protein or polypeptide according to any one of the preceding aspects.
In **a fifth aspect**, the invention pertains to a recombinant host cell, comprising a protein binding compound or an isolated nucleic acid according to any one of the preceding aspects of the invention.
In **a sixth aspect**, the invention pertains to a pharmaceutical composition comprising a protein binding compound recited in any of the preceding aspects of the invention, an isolated nucleic acid, or a recombinant host cell of the previous aspects, together with a pharmaceutically acceptable carrier and/or excipient.
In **a sevenths aspect**, the invention pertains to a product for use in medicine, wherein the product is selected from a protein binding compound, an isolated nucleic acid recited, a recombinant host cell, and a pharmaceutical composition of any of the previous aspects of the invention.
In **an eighth aspect**, the invention pertains to an *in-vitro* method for identifying and/or characterising a compound suitable for the treatment of a disease, disorder or condition that is associated with, or caused by, a bacterial cell expressing PBP2a, or a variant thereof, and/or that is characterised by antibiotic resistance against a treatment with a β-lactam antibiotic, the method comprising the steps of:
   - bringing into contact a candidate compound and a protein of PBP2a (or a variant thereof), or a protein-fragment of PBP2a comprising, or consisting essentially of, a transpeptidase domain of PBP2a (or of the variant thereof); and
   - detecting and/or quantifying a binding of the candidate compound to the transpeptidase domain of the protein of PBP2a (or the variant thereof), or the protein-fragment of PBP2a (or of the variant thereof),
   wherein a binding of the candidate compound to the transpeptidase domain of PBP2a (or the variant thereof) compared to a control indicates that the compound is suitable for the treatment of a disease, disorder or condition that is associated with, or caused by, a bacterial cell expressing PBP2a, or a variant thereof, and/or that is characterised by antibiotic resistance against a treatment with a β-lactam antibiotic.
In a **ninth aspect**, the invention pertains to an *in-vitro* method for identifying and/or characterising a compound suitable for the treatment of a disease, disorder or condition that is associated with, or caused by, a bacterial cell expressing PBP2a, or a variant thereof, and/or that is characterised by antibiotic resistance against a treatment with a β-lactam antibiotic, the method comprising the steps of:
   - bringing into contact a candidate compound and a bacterial cell expressing a protein of PBP2a (or a variant thereof), or a protein-fragment of PBP2a comprising, or consisting essentially of, a transpeptidase domain of PBP2a (or of the variant thereof) an; and
   - determining the growth or viability of the bacterial cell of (a) in response to a treatment with a β-lactam antibiotic,
   wherein a reduced growth or viability of the bacterial cell compared to a control indicates that the compound is suitable for the treatment of a disease, disorder or condition that is associated with, or caused by, a bacterial cell expressing PBP2a, or a variant thereof, and/or that is characterised by antibiotic resistance against a treatment with a β-lactam antibiotic.
In **a tenth aspect**, the present invention pertains to an *in-vitro* diagnostic method for determining whether a subject has, or is at risk of, developing a disease, disorder or condition that is associated with the undesired presence of PBP2a-positive cells (or cells positive for a variant of PBP2a) and/or that is characterized by a resistance to a treatment with a β-lactam antibiotic, the method comprising the step of:
   - detecting PBP2a (or a variant thereof), in particular the presence (or an amount) of or expression and/or activity of PBP2a (or a variant thereof), in a biological sample of the subject, for example a sample suspected to comprising bacterial cells of the disease, with a protein binding compound of the aspects of the invention,
   wherein the detection of PBP2a (or the variant thereof) in the sample indicates such disease, disorder or condition, or a risk of developing such disease, disorder or condition, in the subject; and

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect**, the invention pertains to a protein binding compound (PBC) specifically binding to the transpeptidase domain of penicillin binding protein 2 a (PBP2a), or to a transpeptidase domain of a variant of PBP2a, wherein the PBC when bound to the transpeptidase domain of PBP2a, or to the transpeptidase domain of the variant of PBP2a, increases sensitivity of PBP2a towards a β-lactam antibiotic compared to when the PBC is not bound to the transpeptidase domain of PBP2a, or to the transpeptidase domain of the variant of PBP2a.

In context of the present invention it was surprisingly found that compounds binding to certain areas in the PBP2a protein, when bound, may re-establish sensitivity of the PBP2a protein to antibiotics such as the methicillin. In particular the invention describes compounds binding to the transpeptidase domain of the PBP2a as being useful for therapeutic applications involving PBP2a mediated resistance to antibiotics.

A "protein binding compound" ("PBC") as used herein means a protein or other compound that specifically binds to a target protein antigen, such as to one or more epitope(s) displayed by or present on a target antigen. The antigen of the PBC of the invention is PBP2a or an orthologue (or paralogue) or other variant thereof; and the PBC can, optionally bind to one or more transpeptidase domains of said PBP2a or variant (such as the epitope(s) can be displayed by or present on one or more transpeptidase domains of said PBP2a or variant). Typically, an antigen binding protein is an antibody (or a fragment thereof), however other forms of PBC are also envisioned by the invention. For example, the PBC may be another (non-antibody) receptor protein derived from small and robust non-immunoglobulin "scaffolds", such as those equipped with binding functions for example by using methods of combinatorial protein design (Gebauer & Skerra, 2009). Particular examples of such non-antibody PBCs include: Affibody molecules based on the Z domain of Protein A (Nygren, 2008); Affilins based on gamma-B crystalline and/or ubiquitin (Ebersbach *et al*., 2007); Affimers based on cystatin (Johnson *et al*., 2012); Affitins based on Sac7d from *Sulfolobus acidcaldarius* (Krehenbrink *et al*., 2008); Alphabodies based on a triple helix coiled coil (Desmet *et al*., 2014); Anticalins based on lipocalins (Skerra, 2008); Avimers based on A domains of various membrane receptors (Silverman *et al*., 2005); DARPins based on an ankyrin repeat motif (Stumpp *et al*., 2008); Fynomers based on an SH3 domain of Fyn (Grabulovski *et al*., 2007); Kunitz domain peptides based on Kunitz domains of various protease inhibitors (Nixon & Wood, 2006) and Centyrins and Monobodies based on a 10th type III domain of fibronectin (Diem *et al*., 2014, Koide & Koide, 2007). Preferred PBC of the invention are antibodies, or antigen binding derivatives or fragments thereof, nanobodies, or antigen binding derivatives or fragments thereof, affilins, or antigen binding derivatives or fragments thereof.

The term "epitope" includes any determinant capable of being bound by a protein binding compound of the invention, such as an antibody, nanobody or non-antibody binding proteins such as affilins. An epitope is a region of an antigen that is bound by an antigen binding protein that targets that antigen, and when the antigen is a protein, includes specific amino acids that bind the antigen binding protein (such as via an antigen binding domain of said protein). Epitope determinants can include chemically active surface groupings of molecules such as amino acids, sugar side chains, phosphoryl or sulfonyl groups, and can have specific three-dimensional structural characteristics, and/or specific charge characteristics. Generally, antigen binding proteins specific for a particular target antigen will preferentially recognise an epitope on the target antigen in a complex mixture of proteins and/or macromolecules.

A protein binding compound is "specific" when it binds to one target antigen (such as PBP2a; e.g., of *S. aureus*, orthologues and other variants thereof) more preferentially (e.g., more strongly or more extensively) than it binds to a second antigen. The term "specifically binds" (or "binds specifically" and the like) used herein in the context of an PBC means that said PBC will preferentially bind to the desired antigen (e.g., PBP2a, in particular a transpeptidase domain of PBP2a) than to bind to other proteins (or molecules).

The term "Penicillin Binding Proteins" (PBPs) are a family of essential bacterial enzymes involved in the synthesis of peptidoglycan, the major structural polymer found in the bacterial cell wall. Beta-lactam antibiotics bind with high affinity to PBPs and inhibit their transpeptidase function, resulting in disruption of peptidoglycan cell wall synthesis and rapid cell lysis of actively dividing bacteria. As there are no close mammalian homologues to PBPs, and beta-lactams are well-regarded for their safety and efficacy, PBPs represent an ideal target for antibacterials. The term "PBP2a" relates to a protein encoded by the gene *mecA* and is also known under the designation "beta-lactam-inducible penicillin-binding protein". The protein is found in the UniProt database under the designation Q6I7E7 and shown in the enclosed SEQ ID NO: 394.

The term "orthologue" as used herein means a variant that descends from the same ancestral gene but which is present in another organism due to a speciation event. Orthologues of PBP2a are typically expected to retain the same function as (or have a similar function to) *S. aureus* PBP2a. Those orthologues of *S. aureus* PBP2a include those of other *Staphylococcus* species or *Streptococcus,* such as *Streptococcus pneumoniae.*

The term "transpeptidase domain of PBP2a", refers to a region within the amino acid sequence of PBP2a, or the variant thereof, which folds into a transpeptidase domain. Preferably a transpeptidase domain of PBP2a is found between amino acid residues 250 - 668 of *S. aureus* PBP2a, preferably of SEQ ID NO: 394, +/- 20 amino acid residues, or is located in a homologous region of a homologous PBP2a protein.

The term "variant" as used herein in the context of a protein means any natural or non-natural version of such protein which comprises one or more amino acid mutations compared to the reference protein, but which shares significant amino acid sequence identity with the reference protein, e.g., at least 70% or 75% amino acid sequence identity, preferably at least 80% amino acid sequence identity, more preferably at least 90% amino acid sequence identity and most preferably at least 95%, 96%, 97%, 98% or 99% amino acid sequence identity. Preferably, the variant of the protein possesses and/or maintains at least one function/activity that is the same, essentially the same or similar as the reference protein. Variants of PBP2a may include orthologues to and natural variants of *S. aureus* PBP2. Variants of PBP2a may also correspond to *S. aureus* PBP2a with one or more amino acid residues inserted into, or deleted from the amino acid sequence, such as those variants of PBP2a naturally found within a population or those made by genetic manipulation, such as to specifically engineer amino acid changes into one or more domains (such as transpeptidase domains) of the variant. A variant of PBP2a can, in certain embodiments, comprise a fragment of PBP2a, for example a polypeptide that consists of one or more transpeptidase domains (or regions or (sub)domains thereof) of *S. aureus* PBP2a without one or other allosteric domains of PBP2a. Preferred such variants of PBP2a that are fragments include those that comprise a transpeptidase domain of PBP2a, including its active site, but without any of the allosteric domain, and/or Lobes 1 to 3. Variants of PBP2a also include versions of *S. aureus* PBP2a (or orthologues thereof) that have been modified to display only specific domains (such as the transpeptidase domain), or not to display one or more other domains, and/or to display certain domains (e.g., allosteric domain) of *S. aureus* PBP2a in combination with domains from paralogues and/or orthologues of *S. aureus* PBP2a, or from other PBPs. A "functional variant" of PBP2a (such as a functional fragment of a PBP2a protein) is a variant of the protein of PBP2a that provides, possesses and/or maintains one or more of the herein described functions/activities of the non-variant protein of PBP2a. For example, such functional variant may be involved in bacterial cell-wall biosynthesis, preferably in the final synthesis of peptidoglycan, more preferably in the catalysis of a transpeptidase reaction. In other embodiments, such a functional variant may possess other activities than those possessed by the non-variant PBP2a protein, as long as, preferably, it provides, possesses and/or maintains at least one function/activity that is the same, essentially the same or similar as PBP2a protein. In more preferred embodiments, a functional variant of PBP2a mediates a bacterial resistance to antibiotics, preferably penicillin-like antibiotics such as methicillin.

The term "identity" refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) are preferably addressed by a particular mathematical model or computer program (i.e., an "algorithm"). Methods that can be used to calculate the identity of the aligned nucleic acids or polypeptides include those described in Computational Molecular Biology (Lesk & Tramontano, 1989); Biocomputing Informatics and Genome Projects (Smith, 1994); Computer Analysis of Sequence Data (Griffin & Griffin, 1994, Heijne, 1987); Sequence Analysis Primer (Gribskov & Devereux, 1993, Carrillo & Lipman, 1988).

In some preferred embodiments of the present invention PBP2a is *S. aureus* PBP2a, and/or is a protein comprising an amino acid sequence selected from the group consisting of: SEQ ID NO: 394, or a protein having no more than two, four, six, eight, or ten, for example no more than one, two or three, such as no more than one, amino acid substitutions, insertions or deletions compared to this sequence.

In the context of variants of PBP2a, the invention includes those embodiments where a variant of PBP2a is a protein comprising an amino acid sequence having at least 80%, 85%, 90%, 92% 95% or 97% sequence identity (in particular, at least 92% or 95% sequence identity) to the sequence of SEQ ID NO: 394.

A PBC of the invention may, in particular embodiments, be able to increase the sensitivity of a PBP2a protein or a variant thereof to an antibiotic, preferably wherein the antibiotic reduces the function and/or stability of the PBP2a protein, or of the variant of the PBP2a protein. In some embodiments, without being bound to theory, the PBC of the invention, when bound to PBP2a, or to the variant of PBP2a, increases a binding of the antibiotic to the PBP2a, or to the variant of PBP2a respectively.

In some preferred embodiments of the present invention, the term antibiotic shall refer to β-lactam antibiotics, which are preferably selected from a penicillin or penicillin derivative, such as methicillin, amoxicillin, amoxicillin/clavulanate, ampicillin, ampicilin/sulbactam, bacampicillin, carbenicillin, cloxacillin, dicloxacillin, methicillin, mezlocillin, nafcillin, oxacillin, penicillin G, penicillin V, piperacillin, piperacillin/tazobactam, ticarcillin, ticarcillin/clavulanate. Most preferably, in particular embodiments of the invention, the antibiotic is methicillin.

A methicillin is preferably a compound according to the following formula: 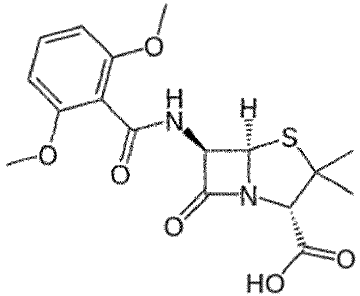 or any derivative thereof. Such derivatives are well known in the art and the present invention shall not be restricted to any specific methicillin or penicillin compound.

In some preferred embodiments the PBC of the invention binds specifically to an amino acid residue in the transpeptidase domain of PBP2a, or the variant of PBP2a. More preferably the PBC of the invention may at least bind to an epitope of or comprised in the transpeptidase domain of PBP2a, or of the variant of PBP2a. Most preferably, in particular embodiments of the invention the binding between the PBC and the transpeptidase domain of PBP2a in accordance with the invention involves a covalent or non-covalent interaction between the PBC and one of the following amino acids of *Staphylococcus aureus* PBP2A: K334, N338, N339, K341, N377, T384, E385, D386, K387, K388, E389, E391, N415, N416, K417, G440, Y441, Y444, Y446, K477, K484, K485, Y499, Q502, N505, N510, I512, L513, D516, G522, E523, L525, W558,3 K559, K560, N561, K584, E585, D586, K589, E602, L603, K604, G611, R612, K634, K639, Y644, K651, D654, E655, E658, N659, and/or E668, or an PBP2a amino acid located in 5 or less amino acid positions in N- or C-terminal direction.

In **a second aspect**, the invention pertains to a protein binding compound (PBC), which is an isolated PBC comprising an amino acid sequence at least 80% identical to a sequence according to any one of SEQ ID NO: 1 to 392. The various embodiments and explanations provided herein above in context of the first aspect shall equally apply for the PBC of the second aspect. Hence, in particular embodiments, the PBC of the second aspect is a PBC of the first aspect.

In particular embodiments, a PBC of the invention can preferentially be an antigen binding protein (ABP) and more preferably an ABP such as an affilin, or alternatively an ABP which comprises at least one complementarity determining region (CDR), such as one from an antibody or nanobody, and in particular embodiments the ABP can comprise a CDR having an amino acid sequence with at least 80%, 85%, 90% or 95% sequence identity to (preferably, at least 90% sequence identity to), or having no more than three or two, preferably no more than one amino acid substitution(s), deletion(s) or insertion(s) compared to, a CDR sequence (CDR 1 to CDR3) set forth in Table 11 and 12.

The term "affilin" as used herein, refers to antibody mimetics that are developed by using either gamma-B crystalline or ubiquitin as a scaffold and modifying amino-acids on the surface of these proteins by random mutagenesis. Selection of affilins with the desired target specificity, i.e. against Btk, is effected, for example, by phage display or ribosome display techniques. Depending on the scaffold, affilins have a molecular weight of approximately 10 or 20kDa. As used herein, the term affilin also refers to di- or multimerised forms of affilins (Weidle *et al*., 2013). An affilin as an ABP of the invention is preferably specifically binding the transpeptidase domain of PBP2a, or the variant of PBP2a, more preferably to any one or more than of the amino acid residues described herein before as preferred epitopes of PBC to PBP2a binding.

The term "complementarity determining region" (or "CDR" or "hypervariable region"), as used herein, refers broadly to one or more of the hypervariable or complementarity determining regions (CDRs) found in the variable regions of light or heavy chains of an antibody. See, for example: "IMGT", (Lefranc, 2003, Honegger & Pluckthun, 2001, Abhinandan & Martin, 2008, Borden & Kabat, 1987). These expressions include the hypervariable regions as defined by Kabat and Chothia (Kabat *et al*., 1983, Chothia & Lesk, 1987). The CDRs in each chain are held in close proximity by framework regions and, with the CDRs from the other chain, contribute to the formation of the antigen-binding site. Within the CDRs there are select amino acids that have been described as the selectivity determining regions (SDRs) which represent the critical contact residues used by the CDR in the antibody-antigen interaction (Kashmiri *et al.*, 2005).

As described above, in particular embodiments of the invention, an ABP can comprise at least one complementarity determining region (CDR). In certain of such embodiments, an ABP of the invention comprises at least one complementarity determining region 3 (CDR3), such as one having an amino acid sequence with at least 80%, 85%, 90% or 95% (preferably at least 90%) sequence identity to, or having no more than three or two, preferably no more than one amino acid substitution(s), deletion(s) or insertion(s) compared to, a sequence selected from those heavy and light chain CDR3 sequences shown in Table 11 and 12 (e.g., a sequence selected from the list consisting of SEQ ID Nos: 6, 14, 22, 30, 38, 46, 54, 62, 70, 78, 86, 94, 102, 110, 118, 126, 134, 142, 150, 158, 166, 174, 182, 190, 198, 206, 214, 222, 230, 238, 246, 254, 262, 270, 278, 286, 294, 302, 310, 318, 326, 334, 342, 350, 358, 366, 374, 382, and 390).

An ABP of the invention may, alternatively or as well as a CDR3 sequence, comprise at least one CDR1, and/or at least one CDR2 (such as one from an antibody, in particular from a human antibody). Preferably, and ABP of the invention comprises at least one such CDR3, as well as at least one such CDR1 and at least one such CDR2, more preferably where each of such CDRs having an amino acid sequence with at least 80%, 85%, 90% or 95% (preferably at least 90%) sequence identity to, or having no more than three or two, preferably no more than one amino acid substitution(s), deletion(s) or insertion(s) compared to, a sequence selected from the corresponding (heavy and light chain) CDR1, CDR2 and CDR3 sequences shown in Table 11 and Table 12.

In particular embodiments, an ABP of the invention can be an antibody or an antigen binding fragment thereof.

As used herein, the term "antibody" may be understood in the broadest sense as any immunoglobulin (Ig) that enables binding to its epitope. An antibody as such is a species of an ABP. Full Length "antibodies" or "immunoglobulins" are generally heterotetrameric glycoproteins of about 150 kDa, composed of two identical light and two identical heavy chains. Each light chain is linked to a heavy chain by one covalent disulphide bond, while the number of disulphide linkages varies between the heavy chains of different immunoglobulin isotypes. Each heavy and light chain also has regularly spaced intrachain disulphide bridges. Each heavy chain has an amino terminal variable domain (VH) followed by three carboxy terminal constant domains (CH). Each light chain has a variable N-terminal domain (VL) and a single C-terminal constant domain (CL). The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to cells or factors, including various cells of the immune system (e.g., effector cells) and the first component (C1q) of the classical complement system. Other forms of antibodies include heavy-chain antibodies, being those which consist only of two heavy chains and lack the two light chains usually found in antibodies. Heavy-chain antibodies include the hcIgG (IgG-like) antibodies of camelids such as dromedaries, camels, llamas and alpacas, and the IgNAR antibodies of cartilaginous fishes (for example sharks). And yet other forms of antibodies include single-domain antibodies (sdAb, called Nanobody by Ablynx, the developer) being an antibody fragment consisting of a single monomeric variable antibody domain. Single-domain antibodies are typically produced from heavy-chain antibodies, but may also be derived from conventional antibodies.

Antibodies (or those from which fragments thereof can be isolated) can include, for instance, chimeric, humanized, (fully) human, or hybrid antibodies with dual or multiple antigen or epitope specificities, antibody fragments and antibody sub-fragments, e.g., Fab, Fab' or F(ab')2 fragments, single chain antibodies (scFv) and the like (described below), including hybrid fragments of any immunoglobulin or any natural, synthetic or genetically engineered protein that acts like an antibody by binding to a specific antigen to form a complex. VSIR is an immunoglobulin-like protein, and as such it is not (nor its variants) considered - for the purposes of the present invention - an antibody that binds to IGSF11.

Accordingly, in certain embodiments an ABP of the invention can comprise an antibody heavy chain, or an antigen binding fragment thereof, and/or an antibody light chain, or an antigen binding fragment thereof.

In further embodiments, an ABP of the invention can comprise an antibody heavy chain variable region, or an antigen binding fragment thereof, and/or an antibody light chain variable region, or an antigen binding fragment thereof, and in yet further embodiments, an ABP of the invention can comprise an antibody heavy chain variable region CDR1, CDR2, and CDR3, and/or an antibody light chain variable region CDR1, CDR2, and CDR3.

In some particular embodiments, the ABP of the invention comprises only antibody heavy chain sequence, such as and preferably, in the format of a nanobody. As used herein, the term "nanobody" refers to a single-domain antibody or an antibody fragment comprising a single monomeric variable antibody domain. A nanobody is able to bind selectively to a specific antigen. Preferably, the nanobody is a V_{H}H antibody. As used herein, the term "V_{H}H antibody" refers to a single monomeric variable region of a heavy chain (VH). Advantageously, nanobodies exhibit a good solubility in water, a high heat resistance, a high resistance to gastric acid and stability towards proteases compared to antibodies. Specific embodiments of preferred nanobodies of the invention are disclosed herein below. The isolated PBC of the invention which is an ABP therefore may be preferred, which (i) comprises an antibody heavy chain variable region, or an antigen binding fragment thereof, and an antibody light chain variable region, or an antigen binding fragment thereof, or is (ii) a nanobody comprising an antibody heavy chain variable region, or an antigen binding fragment thereof, and not comprising an antibody light chain variable region, or an antigen binding fragment thereof.

In particular embodiments of the invention, when the ABP comprises an antibody heavy chain sequence and/or an antibody light chain sequence, or an antigen binding fragment thereof; the antibody heavy chain sequence, or the fragment thereof, can comprise a CDR3 having at least 80%, 85%, 90%; or 95% (preferably at least 90%) sequence identity to, or having no more than three or two, preferably no more than one amino acid substitution(s), deletion(s) or insertion(s) compared to, a CDR3 sequence selected from those heavy chain CDR3 sequences shown in Table 11 and 12 (e.g., a sequence selected from the list consisting of SEQ ID Nos: 6, 22, 38, 54, 70, 86, 102, 118, 134, 150, 166, 182, 198, 214, 230, 246, 262, 270, 278, 286, 294, 302, 310, 318, 326, 334, 342, 350, 358, 366, 374, 382, and 390, and/or wherein antibody light chain sequence, or the fragment thereof, can comprise a CDR3 having at least 80%, 85%, 90%; or 95% (preferably at least 90%) sequence identity to, or having no more than three or two, preferably no more than one amino acid substitution(s), deletion(s) or insertion(s) compared to, a CDR3 sequence selected from those light chain CDR3 sequences shown in Table 11 (e.g., a sequence selected from the list consisting of SEQ ID Nos: 14, 30, 46, 62, 78, 94, 110, 126, 142, 158, 174, 190, 206, 222, 238, and 254).

In further embodiments of the invention, when the ABP comprises an antibody heavy chain, or an antigen binding fragment thereof, the antibody heavy chain sequence, or the fragment thereof, can further comprise a CDR1 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 2, 18, 34, 50, 66, 82, 98, 114, 130, 146, 162, 178, 194, 210, 226, 242, 258, 266, 274, 282, 290, 298, 306, 314, 322, 330, 338, 346, 354, 362, 370, 378, and 386; (e.g., a heavy chain CDR1 sequence disclosed in Table 11 and 12); and/or a CDR2 having at 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 4, 20, 36, 52, 68, 84, 100, 116, 132, 148, 164, 180, 196, 212, 228, 244, 260, 268, 276, 284, 292, 300, 308, 316, 324, 332, 340, 348, 356, 364, 372, 380, and 388 (e.g., a CDR2 sequence disclosed in Table 11 and 12).

In yet further embodiments of the present invention, an ABP of the invention comprises an antibody light chain, or an antigen binding fragment thereof, wherein the antibody light chain sequence, or the fragment thereof, further comprises a CDR1 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 10, 26, 42, 58, 74, 90, 106, 122, 138, 154, 170, 186, 202, 218, 234, and 250 (e.g., a light chain CDR1 sequence disclosed in Table 11 and 12); and/or a CDR2 having at least 80%, 85%, 90%; or 95% (preferably at least 90%) sequence identity to, or having no more than three or two, preferably no more than one amino acid substitution(s), deletion(s) or insertion(s) compared to, a sequence selected from SEQ ID NOs. 12, 28, 44, 60, 76, 92, 108, 124, 140, 156, 172, 188, 204, 220, 236, and 252 (e.g., a light chain CDR2 sequence disclosed in Table 11 and 12).

In other embodiments of the present invention, an ABP of the invention can comprise an antibody variable chain sequence having at least 80%, 85%, 90%; or 95% (preferably at least 90%) sequence identity to, or having no more than ten, nine, eight, seven, six, five, four, three, two or one, preferably no more than three, two or one amino acid substitution(s), deletion(s) or insertion(s) compared to, a sequence selected from SEQ ID NOs. 8, 16, 24, 32, 40, 48, 56, 64, 72, 80, 88, 96, 104, 112, 120, 128, 136, 144, 152, 160, 168, 176, 184, 192, 200, 208, 216, 224, 232, 240, 248, 256, 264, 272, 280, 288, 296, 304, 312, 320, 328, 336, 344, 352, 360, 368, 376, 384 and 392 (e.g., a VH, V_{H}H or VL sequence disclosed in Table 11 and 12).

In particular embodiments of the invention, an ABP of the invention comprises an antigen binding fragment of an antibody, wherein the antigen binding fragment comprises CDR1, CDR2 and CDR3. In certain of such embodiments, the CDR1 is selected from those disclosed in Table 11 and 12, the CDR2 is selected from those disclosed in Table 11 and 12 and the CDR3 is selected from those disclosed in Table 11 and 12; in each case independently, optionally with no more than three or two, preferably no more than one, amino acid substitution(s), insertion(s) or deletion(s) compared to these sequences.

In further particular embodiments of the present invention, the PBC is an ABP which is an antibody, or an antigen binding fragment thereof, composed of at least one antibody heavy chain sequences, and at least one antibody light chain sequences, wherein at least one of said antibody heavy chain sequences and antibody light chain sequences comprise CDR1 to CDR3 sequences in the following combination:

**Table 1: The combination of CDR1 to CDR3 sequences of antibody heavy chain and light chain**

| | **Heavy Chain CDR1 to CDR3 (SEQ ID NO)** | | | **Light Chain CDR1 to CDR3 (SEQ ID NO)** | | |
|---|---|---|---|---|---|---|
| **AB001** | 2 | 4 | 6 | 10 | 12 | 14 |
| **AB002** | 18 | 20 | 22 | 26 | 28 | 30 |
| **AB003** | 34 | 36 | 38 | 42 | 44 | 46 |
| **AB004** | 50 | 52 | 54 | 58 | 60 | 62 |
| **AB005** | 66 | 68 | 70 | 74 | 76 | 78 |
| **AB006** | 82 | 84 | 86 | 90 | 92 | 94 |
| **AB007** | 98 | 100 | 102 | 106 | 108 | 110 |
| **AB008** | 114 | 116 | 118 | 122 | 124 | 126 |
| **AB009** | 130 | 132 | 134 | 138 | 140 | 142 |
| **AB010** | 146 | 148 | 150 | 154 | 156 | 158 |
| **AB011** | 162 | 164 | 166 | 170 | 172 | 174 |
| **AB012** | 178 | 180 | 182 | 186 | 188 | 190 |
| **AB013** | 194 | 196 | 198 | 202 | 204 | 206 |
| **AB014** | 210 | 212 | 214 | 218 | 220 | 222 |
| **AB015** | 226 | 228 | 230 | 234 | 236 | 238 |
| **AB016** | 242 | 244 | 246 | 250 | 252 | 254 |

in each case independently, optionally with not more than three or two, preferably one, amino acid substitution(s), insertion(s) or deletion(s) compared to these sequences.

In further particular embodiments of the present invention, the PBC is an ABP which is an antibody, or an antigen binding fragment thereof, composed of at least one antibody heavy chain sequence, and at least one antibody light chain sequence, wherein said antibody heavy chain sequence and the antibody light chain sequence comprises each a variable region sequences of the following combination:

**Table 2: The combination of variable region sequences of antibody heavy chain and light chain**

| | **Heavy Chain Variable Region (SEQ ID NO)** | **Light Chain Variable Region (SEQ ID NO)** |
|---|---|---|
| **AB001** | 8 | 16 |
| **AB002** | 24 | 32 |
| **AB003** | 40 | 48 |
| **AB004** | 56 | 64 |
| **AB005** | 72 | 80 |
| **AB006** | 88 | 96 |
| **AB007** | 104 | 112 |
| **AB008** | 120 | 128 |
| **AB009** | 136 | 144 |
| **AB010** | 152 | 160 |
| **AB011** | 168 | 176 |
| **AB012** | 184 | 192 |
| **AB013** | 200 | 208 |
| **AB014** | 216 | 224 |
| **AB015** | 232 | 240 |
| **AB016** | 248 | 256 |

in each case independently, optionally with not more than three or two, preferably one, amino acid substitution(s), insertion(s) or deletion(s) compared to these sequences.

In further particular embodiments of the present invention, the PBC is an ABP wherein in (ii) the isolated PBC comprises a nanobody heavy chain sequence, or an antigen binding fragment thereof; wherein the nanobody heavy chain sequence, or the fragment thereof, comprises a CDR3 having at least 80% sequence identity to an amino acid sequence selected from SEQ ID Nos. 262, 270, 278, 286, 294, 302, 310, 318, 326, 334, 342, 350, 358, 366, 374, 382, and 390 in each case independently, optionally with not more than three or two, preferably one, amino acid substitution(s), insertion(s) or deletion(s) compared to these sequences.

Yet in further particular embodiments of the invention, the isolated PBC further comprises a CDR1 having at least 80% sequence identity to an amino acid sequence selected from SEQ ID Nos. 258, 266, 274, 282, 290, 298, 306, 314, 322, 330, 338, 346, 354, 362, 370, 378 and 386; and/or a CDR2 having at 80% sequence identity to an amino acid sequence selected from SEQ ID Nos. 260, 268, 276, 284, 292, 300, 308, 316, 324, 332, 340, 348, 356, 364, 372, 380 and 388, in each case independently, optionally with not more than three or two, preferably one, amino acid substitution(s), insertion(s) or deletion(s) compared to these sequences.

In further particular embodiments of the present invention, the PBC is an ABP which is a nanobody, or an antigen binding fragment thereof, composed of an antibody heavy chain sequence comprising CDR1 to CDR3 sequences in the following combination:

**Table 3: The combination of CDR1 to CDR3 sequences of antibody heavy chain**

| | **Heavy Chain CDR1 to CDR3 (SEQ ID NO)** | | |
|---|---|---|---|
| **NB001** | 258 | 260 | 262 |
| **NB002** | 266 | 268 | 270 |
| **NB003** | 274 | 276 | 278 |
| **NB004** | 282 | 284 | 286 |
| **NB005** | 290 | 292 | 294 |
| **NB006** | 298 | 300 | 302 |
| **NB007** | 306 | 308 | 310 |
| **NB008** | 314 | 316 | 318 |
| **NB009** | 322 | 324 | 326 |
| **NB010** | 330 | 332 | 334 |
| **NB011** | 338 | 340 | 342 |
| **NB012** | 346 | 348 | 350 |
| **NB013** | 354 | 356 | 358 |
| **NB014** | 362 | 364 | 366 |
| **NB015** | 370 | 372 | 374 |
| **NB016** | 378 | 380 | 382 |
| **NB017** | 386 | 388 | 390 |

in each case independently, optionally with not more than three or two, preferably one, amino acid substitution(s), insertion(s) or deletion(s) compared to these sequences.

In some preferred embodiments wherein the PBC is a nanobody or an antigen binding fragment thereof, such nanobody, or antigen binding fragment thereof, is composed of an antibody heavy chain sequence comprising any one of the sequences selected from SEQ ID Nos 264, 272, 280, 288, 296, 304, 312, 320, 328, 336, 344, 352, 360, 368, 376, 384 and 392, in each case independently, optionally with not more than ten, nine, eight, five, three or two, preferably one, amino acid substitution(s), insertion(s) or deletion(s) compared to these sequences.

In **a third aspect**, which is a related aspect, the invention further pertains to an isolated PBC, which competes with an PBC as recited in the above disclosure regarding the second aspect of the invention, for binding to the transpeptidase domain of PBP2a, preferably of *Staphylococcus aureus,* and is capable of increasing sensitivity of said PBP2a to a β-lactam antibiotic, preferably capable of increasing of the binding of the β-lactam antibiotic to the PBP2a.

The term "compete" when used in the context of PBCs (e.g., ABPs such as antibodies or nanobodies) that compete for binding for the same antigen (or epitope displayed by such antigen) means competition between PBCs as may be determined by an assay in which the PBC (e.g., antibody or binding fragment thereof) being tested prevents or inhibits (e.g., reduces) binding of a reference PBC (e.g., a reference antibody) to a common antigen (e.g., an epitope in the transpeptidase domain of PBP2a).

In preferred embodiments of all PBCs of the invention, the PBC is isolated and/or substantially pure.

The term "isolated" as used herein in the context of a protein, such as an PBC (an example of which could be an antibody), refers to a protein that is purified from proteins or polypeptides or other contaminants that would interfere with its therapeutic, diagnostic, prophylactic, research or other use. An isolated PBC according to the invention may be a recombinant, synthetic or modified (non-natural) PBC. The term "isolated" as used herein in the context of a nucleic acid or cells refers to a nucleic acid or cells that is/are purified from DNA, RNA, proteins or polypeptides or other contaminants (such as other cells) that would interfere with its therapeutic, diagnostic, prophylactic, research or other use, or it refers to a recombinant, synthetic or modified (non-natural) nucleic acid. Preferably an isolated ABP or nucleic acid or cells is/are substantially pure. In this context, a "recombinant" protein or nucleic acid is one made using recombinant techniques. Methods and techniques for the production of recombinant nucleic acids and proteins are well known in the art.

In one embodiment, a PBC of the invention is an ABP and preferably a polyclonal antibody (mixture), or the antigen binding fragment is a fragment of a polyclonal antibody (mixture).

In an alternative, and preferred, embodiment of all PBCs of the invention, the PBC is an antibody or an antigen binding fragment thereof, and the antibody is a monoclonal antibody, or wherein the antigen binding fragment is a fragment of a monoclonal antibody.

The term "monoclonal antibody" or "mAb" as used herein refers to an antibody obtained from a population of substantially identical antibodies based on their amino acid sequence. Monoclonal antibodies are typically highly specific. Furthermore, in contrast to conventional (polyclonal) antibody preparations which typically include different antibodies directed against different determinants (e.g., epitopes) of an antigen, each mAb is typically directed against a single determinant on the antigen. In addition to their specificity, mAbs are advantageous in that they can be synthesized by cell culture (hybridomas, recombinant cells or the like) uncontaminated by other immunoglobulins. The mAbs herein include for example chimeric, humanized or human antibodies or antibody fragments.

In a further preferred embodiment, a PBC of the invention is an antibody or an antigen binding fragment thereof, wherein the antibody is a human antibody, a humanised antibody or a chimeric-human antibody, or wherein the antigen binding fragment is a fragment of a human antibody, a humanised antibody or a chimeric-human antibody.

Human antibodies can also be derived by in-vitro methods. Suitable examples include but are not limited to phage display (CAT, Morphosys, Dyax, Biosite/Medarex, Xoma, Yumab, Symphogen, Alexion, Affimed) and the like. In phage display, a polynucleotide encoding a single Fab or Fv antibody fragment is expressed on the surface of a phage particle (see e.g., (Marks *et al*., 1991, Clackson *et al*., 1991) and U.S. Patent No. 5,885,793). Phages are "screened" to identify those antibody fragments having affinity for target. Thus, certain such processes mimic immune selection through the display of antibody fragment repertoires on the surface of filamentous bacteriophage, and subsequent selection of phage by their binding to target. In certain such procedures, high affinity functional neutralizing antibody fragments are isolated. A complete repertoire of human antibody genes may thus be created by cloning naturally rearranged human V genes from peripheral blood lymphocytes (see, e.g., Mullinax *et al*., 1990) or by generating fully synthetic or semi-synthetic phage display libraries with human antibody sequences (see Knappik *et al*., 2000, de Kruif *et al*., 1995).

The antibodies described herein may alternatively be prepared through the utilization of the XenoMouse® technology. Such mice are capable of producing human immunoglobulin molecules and antibodies and are deficient in the production of murine immunoglobulin molecules and antibodies. In particular, a preferred embodiment of transgenic production of mice and antibodies is disclosed in U.S. Patent Application Serial No. 08/759,620, filed December 3, 1996 and International Patent Application Nos. WO 98/24893, published June 11, 1998 and WO 00/76310, published December 21, 2000. See also Mendez *et al*., 1997. Through the use of such technology, fully human monoclonal antibodies to a variety of antigens have been produced. Essentially, XenoMouse® lines of mice are immunized with an antigen of interest. e.g., IGSF11 (VSIG3), lymphatic cells (such as B-cells) are recovered from the hyper-immunized mice, and the recovered lymphocytes are fused with a myeloid-type cell line to prepare immortal hybridoma cell lines. These hybridoma cell lines are screened and selected to identify hybridoma cell lines that produce antibodies specific to the antigen of interest. Other "humanised" mice are also commercially available: e.g., Medarex - HuMab mouse, Kymab - Kymouse, Regeneron - Velocimmune mouse, Kirin - TC mouse, Trianni - Trianni mouse, OmniAb - OmniMouse, Harbour Antibodies - H2L2 mouse, Merus - MeMo mouse. Also are available are "humanised" other species: rats: OmniAb - OmniRat, OMT - UniRat. Chicken: OmniAb - OmniChicken.

The term "humanised antibody" according to the present invention refers to immunoglobulin chains or fragments thereof (such as Fab, Fab', F(ab')2, Fv, or other antigen-binding sub-sequences of antibodies), which contain minimal sequence (but typically, still at least a portion) derived from non-human immunoglobulin. For the most part, humanised antibodies are human immunoglobulins (the recipient antibody) in which CDR residues of the recipient antibody are replaced by CDR residues from a non-human species immunoglobulin (the donor antibody) such as a mouse, rat or rabbit having the desired specificity, affinity and capacity. As such, at least a portion of the framework sequence of said antibody or fragment thereof may be a human consensus framework sequence. In some instances, Fv framework residues of the human immunoglobulin need to be replaced by the corresponding non-human residues to increase specificity or affinity. Furthermore, humanised antibodies can comprise residues which are found neither in the recipient antibody nor in the imported CDR or framework sequences. These modifications are made to further refine and maximise antibody performance. In general, the humanised antibody will comprise substantially all of at least one, and typically at least two, variable domains, in which all or substantially all of the CDR regions correspond to those of a non-human immunoglobulin and all or substantially all of the framework regions are those of a human immunoglobulin consensus sequence. The humanised antibody optimally also will comprise at least a portion of an immunoglobulin constant region, typically that of a human immunoglobulin, which (e.g., human) immunoglobulin constant region may be modified (e.g., by mutations or glycoengineering) to optimise one or more properties of such region and/or to improve the function of the (e.g., therapeutic) antibody, such as to increase or reduce Fc effector functions or to increase serum half-life. Exemplary such Fc modification (e.g., Fc engineering or Fc enhancement) are described elsewhere herein.

The term "chimeric antibody" according to the present invention refers to an antibody whose light and/or heavy chain genes have been constructed, typically by genetic engineering, from immunoglobulin variable and constant regions which are identical to, or homologous to, corresponding sequences of different species, such as mouse and human. Alternatively, variable region genes derive from a particular antibody class or subclass while the remainder of the chain derives from another antibody class or subclass of the same or a different species. It covers also fragments of such antibodies. For example, a typical therapeutic chimeric antibody is a hybrid protein composed of the variable or antigen-binding domain from a mouse antibody and the constant or effector domain from a human antibody, although other mammalian species may be used.

In particular of such embodiments, a PBC of the invention comprises an antigen binding domain of an antibody wherein the antigen binding domain is of a human antibody. Preferably, PBC comprises (i) an antigen binding domain of an antibody or an antigen binding fragment thereof, which is a human antigen binding domain; (ii) the antibody is a monoclonal antibody, or wherein the antigen binding fragment is a fragment of a monoclonal antibody; and (iii) the antibody is a human antibody or a humanised antibody, or wherein the antigen binding fragment is a fragment of a human antibody, a humanised antibody or a chimeric-human antibody.

Light chains of human antibodies generally are classified as kappa and lambda light chains, and each of these contains one variable region and one constant domain. Heavy chains are typically classified as mu, delta, gamma, alpha, or epsilon chains, and these define the antibody's isotype as IgM, IgD, IgG, IgA, and IgE, respectively. Human IgG has several subtypes, including, but not limited to, IgG1, IgG2, IgG3, and IgG4. Human IgM subtypes include IgM, and IgM2. Human IgA subtypes include IgA1 and IgA2. In humans, the IgA and IgD isotypes contain four heavy chains and four light chains; the IgG and IgE isotypes contain two heavy chains and two light chains; and the IgM isotype contains ten or twelve heavy chains and ten or twelve light chains. Antibodies according to the invention may be IgG, IgE, IgD, IgA, or IgM immunoglobulins.

In some embodiments, the PBC of the invention is an IgG antibody or fragment thereof. In some embodiments, the PBC of the invention is an IgE antibody or fragment thereof. In some embodiments, the PBC of the invention is an IgD antibody or fragment thereof. In some embodiments, the PBC of the invention is an IgA antibody or fragment thereof. In some embodiments, the PBC of the invention is an IgM antibody or fragment thereof. Preferably the PBC of the invention is, comprises or is derived from an IgG immunoglobulin or fragment thereof; such as a human, human-derived IgG immunoglobulin, or a rabbit- or rat-derived IgG, and/or an IgG2 immunoglobulin, or fragment thereof. When the PBC of the invention is, comprises or is derived from a rat-derived IgG, then preferably, the PBC is, comprises or is derived from, a rat IgG2a or IgG2b immunoglobulin. When the PBC of the invention is, comprises or is derived from a human-derived IgG, then more preferably, the PBC of the invention is, comprises or is derived from a human IgG1, IgG2 or IgG4, most preferably, the PBC of the invention is, comprises or is derived from a human IgG1 or IgG2

Accordingly, in particular embodiments of the invention, an PBC is an antibody wherein the antibody is an IgG, IgE, IgD, IgA, or IgM immunoglobulin; preferably an IgG immunoglobulin.

A PBC of the invention, where comprising at least a portion of an immunoglobulin constant region (typically that of a human immunoglobulin) may have such (e.g., human) immunoglobulin constant region modified - for example by glycoengineering or mutations - to optimise one or more properties of such region and/or to improve the function of the (e.g., therapeutic) antibody, such as to increase or reduce Fc effector functions or to increase serum half-life.

Certain specific embodiments of the invention pertain to so called "nanobodies" or "VHH" antibody-constructs. A VHH is the variable domain of a heavy-chain-only antibody from a camelid (HcAb) or a molecule derived from such a VHH and having substantially the same properties as the original VHH in particular in respect of antigen recognition capacity (including when having no antigen recognition capacity). All the species of the Camelidea family have heavy-chain-only antibodies. In a preferred embodiment, the VHH of the invention is obtained from an alpaca (Lama *pacos*)*.*

Antibody fragments in accordance with the invention include "Fab fragments", which are composed of one constant and one variable domain of each of the heavy and the light chains, held together by the adjacent constant region of the light chain and the first constant domain (CH1) of the heavy chain. These may be formed by protease digestion, e.g., with papain, from conventional antibodies, but similar Fab fragments may also be produced by genetic engineering. Fab fragments include Fab', Fab and "Fab-SH" (which are Fab fragments containing at least one free sulfhydryl group).

Fab' fragments differ from Fab fragments in that they contain additional residues at the carboxy-terminus of the first constant domain of the heavy chain including one or more cysteines from the antibody hinge region. Fab' fragments include "Fab'-SH" (which are Fab' fragments containing at least one free sulfhydryl group).

Further, antibody fragments include F(ab')2 fragments, which contain two light chains and two heavy chains containing a portion of the constant region between the CH1 and CH2 domains ("hinge region"), such that an interchain disulphide bond is formed between the two heavy chains. A F(ab')2 fragment thus is composed of two Fab' fragments that are held together by a disulphide bond between the two heavy chains. F(ab')2 fragments may be prepared from conventional antibodies by proteolytic cleavage with an enzyme that cleaves below the hinge region, e.g., with pepsin, or by genetic engineering.

An "Fv region" comprises the variable regions from both the heavy and light chains, but lacks the constant regions. "Single-chain antibodies" or "scFv" are Fv molecules in which the heavy and light chain variable regions have been connected by a flexible linker to form a single polypeptide chain, which forms an antigen binding region.

An "Fc region" comprises two heavy chain fragments comprising the CH2 and CH3 domains of an antibody. The two heavy chain fragments are held together by two or more disulphide bonds and by hydrophobic interactions of the CH3 domains.

Accordingly, in some embodiments, the ABP of the invention is an antibody fragment selected from the list consisting of: Fab', Fab, Fab'-SH, Fab-SH, Fv, scFv and F(ab')2.

Derivatives of nanobodies might include homodimers of the nanobodies of the invention, which may be obtained for example by introducing one or more cysteines for the formation of disulphide bonds. The generation of VHH homodimers is well known to the skilled artisan.

In those embodiments of ABPs that are fragments of immunoglobulins, such as an antibody fragment, preferred are those fragments capable of binding to (e.g., an epitope displayed by) the extracellular domain(s) of IGSF11, or a paralogue, orthologue or other variant thereof, such as any epitope or other binding characteristic as described herein: and more preferably said fragment is a modulator (such as an inhibitor or antagonist) of the expression, function, activity and/or stability of PBP2a or a paralogue, orthologue or other variant of PBP2a.

In a preferred embodiment, an ABP of the invention is an antibody wherein at least a portion of the framework sequence of said antibody or fragment thereof is a human consensus framework sequence, for example, comprises a human germline-encoded framework sequence.

In **a fourth aspect**, the invention relates to a nucleic acid encoding for a PBC (or ABP, or antigen binding domain (ABD)) of the invention (such as one described above) or of components thereof. For example, the component encoded by a nucleic acid of the invention may be all or part of one chain of an antibody or nanobody of the invention; or the component may be a scFV of said PBC. The component encoded by such a nucleic acid may be all or part of one or other of the chains of an antibody of the invention; for example, the component encoded by such a nucleic acid may be an ABD of the invention. The nucleic acids of the invention may also encode a fragment, derivative, mutant, or variant of an ABP of the invention, and/or represent components that are polynucleotides suitable and/or sufficient for use as hybridisation probes, polymerase chain reaction (PCR) primers or sequencing primers for identifying, analyzing, mutating or amplifying a polynucleotide encoding a polypeptide, antisense or inhibitory nucleic acids (such as RNAi/siRNA/shRNA or gRNA molecules) for inhibiting expression of a polynucleotide, and complementary sequences of the foregoing.

In one embodiment, the nucleic acid of the invention may be isolated or substantially pure. In another embodiment, the nucleic acid of the invention may be recombinant, synthetic and/or modified, or in any other way non-natural. For example, a nucleic acid of the invention may contain at least one nucleic acid substitution (or deletion) modification (such as 2, 3, 4, 5, 6, 7, 8, 9, 10 or more than 10 such modifications, in particular between 1 and about 5 such modifications, preferably 2 or 3 such modifications) relative to a product of nature, such as a human nucleic acid.

Nucleic acids encoding antibody polypeptides (e.g., heavy or light chain, variable domain only, or Full Length) may be isolated from B-cells of mice, rats, llamas, alpacas, chicken, or rabbits that have been immunized with a PBP2a antigen or fragment thereof, such as one or more transpeptidase domains (or a polynucleotide encoding and capable of expressing an PBP2a antigen or fragment thereof). The nucleic acid may be isolated by conventional procedures such as PCR.

In one related aspect, the invention relates to a nucleic acid construct (NAC) comprising at least one nucleic acid of the invention (such as described above). Such an NAC can comprise one or more additional features permitting the expression of the encoded ABP or component of said ABP (e.g., the ABD) in a cell (such as in a host cell). Examples of NACs of the invention include, but are not limited to, plasmid vectors, viral vectors, mRNA, non-episomal mammalian vectors and expression vectors, for example, recombinant expression vectors. The nucleic acid constructs of the invention can comprise a nucleic acid of the invention in a form suitable for expression of the nucleic acid in a cell, such as a host cell, (see below). The nucleic acid constructs of the invention will be, typically, recombinant nucleic acids, and/or may be isolated and/or substantially pure. Recombinant nucleic acids will, typically, be non-natural; particularly if they comprise portions that are derived from different species and/or synthetic, in-vitro or mutagenic methods.

In some embodiments, an NAC of the invention comprises one or more constructs either of which includes a nucleic acid encoding either a heavy or a light antibody chain. In some embodiments, the NAC of the invention comprises two constructs, one of which includes a nucleic acid encoding the heavy antibody chain, the other of which includes a nucleic acid encoding the light antibody chain, such that expression from both constructs can generate a complete antibody molecule. In some embodiments, the NAC of the invention comprises a construct which includes nucleic acids encoding both heavy and light antibody chains, such that a complete antibody molecule can be expressed from one construct. In other embodiments, an NAC of the invention can comprise a single construct that encodes a single chain which is sufficient to form an ABP of the invention; for example, if the encoded ABP is a scFv or a single-domain antibody (such as a camelid antibody or nanobody).

In some embodiments, the NAC of the invention includes sequences encoding all or part of a constant region, enabling an entire, or a part of, a heavy and/or light chain to be expressed.

In **a fifth aspect**, the invention relates to a cell (such as a host cell and/or a recombinant host cell) comprising one or more nucleic acid or NAC of the invention. Preferably, such cell is capable of expressing the ABP (or component thereof) encoded by said NAC(s). For example, if an ABP of the invention comprises two separate polypeptide chains (e.g., a heavy and light chain of an IgG), then the cell of the invention may comprise a first NAC that encodes (and can express) the heavy chain of such ABP as well as a second NAC that encodes (and can express) the light chain of such ABP; alternatively, the cell may comprise a single NAC that encodes both chains of such ABP. In these ways, such a cell of the invention would be capable of expressing a functional (e.g., binding and/or inhibitory) ABP of the invention. A (host) cell of invention may be one of the mammalian, prokaryotic or eukaryotic host cells as described elsewhere herein, in particularly where the cell is a Chinese hamster ovary (CHO) cell.

In certain embodiments of such aspect, the (host) cell is a human cell; in particular it may be a human cell that has been sampled from a specific individual (e.g., an autologous human cell). In such embodiments, such human cell can be propagated and/or manipulated in-vitro so as to introduce a NAC of the present invention. The utility of a manipulated human cell from a specific individual can be to produce an ABP of the invention, including to reintroduce a population of such manipulated human cells into a human subject, such as for use in therapy. In certain of such uses, the manipulated human cell may be introduced into the same human individual from which it was first sampled; for example, as an autologous human cell.

To be used in therapy, the PBCs, nucleic acids or NACs (or the cells, such as host cells) of the invention may be formulated into a pharmaceutical composition appropriate to facilitate administration to animals or humans. The term "pharmaceutical composition" means a mixture of substances including a therapeutically active substance (such as an PBC of the invention) for pharmaceutical use.

Accordingly, **in a sixth aspect**, the invention relates to a pharmaceutical composition comprising a compound of the invention, preferably that is modulator of the PBP2a mediated resistance, or of a variant of PBP2a and a pharmaceutically acceptable carrier, stabiliser and/or excipient. Accordingly, in a related aspect, herein provided is a pharmaceutical composition comprising a PBC of the invention, and/or at least one nucleic acid of the invention, and/or a (host) cell of the invention, and a pharmaceutically acceptable excipient or carrier.

By way of example, the pharmaceutical composition of the invention may comprise between 0.1% and 100% (w/w) active ingredient (for example, a nanobody of the invention), such as about 0.5%, 1%, 2%, 3%, 4%, 5%, 6%, 8% 10%, 15%, 20%, 25%, 30%, 40%, 50%, 60%, 70%, 80%, 90%, 95%, 96%, 97%, 98% or 99%, preferably between about 1% and about 20%, between about 10% and 50% or between about 40% and 90%.

As used herein the language "pharmaceutically acceptable" excipient, stabiliser or carrier is intended to include any and all solvents, solubilisers, fillers, stabilisers, binders, absorbents, bases, buffering agents, lubricants, controlled release vehicles, diluents, emulsifying agents, humectants, dispersion media, coatings, antibacterial or antifungal agents, isotonic and absorption delaying agents, and the like, compatible with pharmaceutical administration. The use of such media and agents for pharmaceutically active substances is well-known in the art. Except insofar as any conventional media or agent is incompatible with the active compound, use thereof in the compositions is contemplated. Supplementary agents can also be incorporated into the compositions.

The pharmaceutical composition of (or for use with) the invention is, typically, formulated to be compatible with its intended route of administration. Examples of routes of administration include oral, parenteral, e.g., intrathecal, intra-arterial, intravenous, intradermal, subcutaneous, oral, transdermal (topical) and transmucosal administration.

In some embodiments, the pharmaceutical composition comprising a PBC of the invention is in unit dose form of between 10 and 1000mg PBC. In some embodiments, the pharmaceutical composition comprising a PBC is in unit dose form of between 10 and 200mg PBC. In some embodiments, the pharmaceutical composition comprising an PBC is in unit dose form of between 200 and 400mg. In some embodiments, the pharmaceutical composition comprising a PBC is in unit dose form of between 400 and 600mg PBC. In some embodiments, the pharmaceutical composition is in unit dose form of between 600 and 800mg PBC. In some embodiments, the pharmaceutical composition comprising a PBC is in unit dose form of between 800 and 100 mg.

Exemplary unit dosage forms for pharmaceutical compositions comprising a PBC of the invention are tablets, capsules (e.g., as powder, granules, microtablets or micropellets), suspensions or as single-use pre-loaded syringes. In certain embodiments, kits are provided for producing a single-dose administration unit. The kit can contain both a first container having a dried active ingredient and a second container having an aqueous formulation. Alternatively, the kit can contain single and multi-chambered pre-loaded syringes.

A pharmaceutical composition of the invention in particular embodiments may comprise additional active ingredients. Such additional active ingredients are preferably selected from antibiotic compounds which act via, or bind to, PBP2a, or the variant of PBP2a. Hence, preferably such additional active ingredient is selected from β-lactam antibiotics. Preferably such β-lactam antibiotic is selected from the group consisting of a penicillin or penicillin derivative, such as methicillin, amoxicillin, amoxicillin/clavulanate, ampicillin, ampicillin/sulbactam, bacampicillin, carbenicillin, cloxacillin, dicloxacillin, methicillin, mezlocillin, nafcillin, oxacillin, penicillin G, penicillin V, piperacillin, piperacillin/tazobactam, ticarcillin, or ticarcillin/clavulanate.

The invention then pertains in **a seventh aspect** to a product for use in medicine, wherein the product comprises a PBC of the invention, and/or a nucleic acid or host cell of the invention.

Thus provided is, in this seventh aspect, a medical use which is a method for the treatment of a disease, disorder or condition in a mammalian subject by administering a product to the subject wherein the product of the invention (the PBC, nucleic acid, cell and/or pharmaceutical composition). In a related aspect, the invention relates to a use of such a product of the invention in a method for the manufacture of a medicament for use in a disease or condition described herein.

The term "treatment" in the present invention is meant to include therapy, e.g., therapeutic treatment, as well as prophylactic or suppressive measures for a disease (or disorder or condition). Thus, for example, successful administration of a PBC prior to onset of the disease results in treatment of the disease. "Treatment" also encompasses administration of a PBC after the appearance of the disease in order to ameliorate or eradicate the disease (or symptoms thereof). Administration of a PBC after onset and after clinical symptoms, with possible abatement of clinical symptoms and perhaps amelioration of the disease, also comprises treatment of the disease. Those "in need of treatment" include subjects (such as a human subject) already having the disease, disorder or condition, as well as those prone to or suspected of having the disease, disorder or condition, including those in which the disease, disorder or condition is to be prevented.

In particular embodiments a disease to be treated or prevented in accordance with the invention is a disease or disorder characterised by an infection with a bacterial pathogen expressing a PBP2a protein, or a variant protein thereof. Thereby, such disease or condition in preferred embodiments is characterised by a resistance of said bacterial pathogen to certain antibiotic compounds (e.g., Penicillin and related β-lactam antibiotics such as methicillin). Hence, in certain particular embodiments of the invention a treatment or prevention comprises the additional administration of such an antibiotic to the subject in need of the treatment. The present invention provides products for use in medicine which increase sensitivity of pathogenic organisms to a certain antibiotic agent, as described herein.

In some embodiments, diseases or conditions for which the various compositions and methods of the invention is useful are infectious diseases. The term "infectious disease" is art recognised, and as used herein includes those diseases, disorders or conditions associated with (e.g., resulting from or caused by) by any pathogen or agent that infects mammalian cells, preferable human cells. Examples of such pathogens include bacteria, yeast, fungi, protozoans, mycoplasma, viruses, prions, and parasites. Examples of infectious disease include (a) viral diseases such as, for example, diseases resulting from infection by an adenovirus, a herpesvirus (e.g., HSV-I, HSV-II, CMV, or VZV), a poxvirus (e.g., an orthopoxvirus such as variola or vaccinia, or molluscum contagiosum), a picornavirus (e.g., rhinovirus or enterovirus), an orthomyxovirus (e.g., influenza virus), a paramyxovirus (e.g., parainfluenza virus, mumps virus, measles virus, and respiratory syncytial virus (RSV)), a cononavirus (e.g., SARS), a papovavirus (e.g., papillomaviruses, such as those that cause genital warts, common warts, or plantar warts), a hepadnavirus (e.g., hepatitis B virus), a flavivirus (e.g., hepatitis C virus or Dengue virus), or a retrovirus (e.g., a lentivirus such as HIV); (b) bacterial diseases such as, for example, diseases resulting from infection by bacteria of, for example, the genus Escherichia, Enterobacter, Salmonella, Staphylococcus, Shigella, Listeria, Aerobacter, Helicobacter, Klebsiella, Proteus, Pseudomonas, Streptococcus, Chlamydia, Mycoplasma, Pneumococcus, Neisseria, Clostridium, Bacillus, Corynebacterium, Mycobacterium, Campylobacter, Vibrio, Serratia, Providencia, Chromobacterium, Brucella, Yersinia, Haemophilus, or Bordetella; (c) other infectious diseases, such chlamydia, fungal diseases including but not limited to candidiasis, aspergillosis, histoplasmosis, cryptococcal meningitis, parasitic diseases including but not limited to malaria, Pneumocystis camii pneumonia, leishmaniasis, cryptosporidiosis, toxoplasmosis, and trypanosome infection and prions that cause human disease such as Creutzfeldt-Jakob Disease (CJD), variant Creutzfeldt-Jakob Disease (vCJD), Gerstmann-Straeussler-Scheinker syndrome, Fatal Familial Insomnia and kuru.

In particular embodiments, the compounds, compositions and products of the invention and methods disclosed herein are useful in detecting, preventing, treating or controlling bacterial infections caused by antibiotic resistant bacteria, wherein the resistance is due to a PBP2a-driven mechanism, or driven by a variant of PBP2a. Some non-limiting examples of such bacteria known to have developed resistance due to PBP2a include *S. aureus, S. epidermidis, S. hominis, S. lugdunensis, S. xylosus,* and *S. felis* (Petinaki *et al*., 2001, Nascimento *et al*., 2015). In a specific embodiment, the antibiotic resistant bacterium is methicillin-resistant *Staphylococcus aureus* (MRSA). Non-limiting examples of infections that may be prevented or treated using the compositions and/or methods of the disclosure include: skin and soft tissue infections, febrile neutropenia, urinary tract infection, intraabdominal infections, respiratory tract infections, pneumonia (nosocomial), bacteremia meningitis, and surgical infections.

A subject may be a human, a livestock animal, a companion animal, a lab animal, or a zoological animal. In one embodiment, the subject may be a rodent, e.g., a mouse, a rat, a guinea pig, etc. In another embodiment, the subject may be a livestock animal. Non-limiting examples of suitable livestock animals may include pigs, cows, horses, goats, sheep, llamas and alpacas. In yet another embodiment, the subject may be a companion animal. Non-limiting examples of companion animals may include pets such as dogs, cats, rabbits, and birds. In yet another embodiment, the subject may be a zoological animal. As used herein, a "zoological animal" refers to an animal that may be found in a zoo. Such animals may include non-human primates, large cats, wolves, and bears. In certain embodiments, the animal is a laboratory animal. Non-limiting examples of a laboratory animal may include rodents, canines, felines, and non-human primates. In certain embodiments, the animal is a rodent. Non-limiting examples of rodents may include mice, rats, guinea pigs, etc.

In **an eighth aspect**, the invention provides a method for identifying and/or characterising a compound suitable for the treatment of a disease, disorder or condition that is associated with, or caused by, a bacterial cell expressing PBP2a, or a variant thereof, and/or that is characterised by antibiotic resistance against a treatment with a β-lactam antibiotic, the method comprising the steps of:
- bringing into contact a candidate compound and a protein of PBP2a (or a variant thereof), or a protein-fragment of PBP2a comprising, or consisting essentially of, a transpeptidase domain of PBP2a (or of the variant thereof); and
- detecting and/or quantifying a binding of the candidate compound to the transpeptidase domain of the protein of PBP2a (or the variant thereof), or the protein-fragment of PBP2a (or of the variant thereof),
wherein a binding of the candidate compound to the transpeptidase domain of PBP2a (or the variant thereof) compared to a control indicates that the compound is suitable for the treatment of a disease, disorder or condition that is associated with, or caused by, a bacterial cell expressing PBP2a, or a variant thereof, and/or that is characterised by antibiotic resistance against a treatment with a β-lactam antibiotic.

The candidate compound used in the screening methods may be one selected from a polypeptide, peptide, glycoprotein, a peptidomimetic, an affilin, a nanobody, an antibody or antibody-like molecule (such as an intra-body); a nucleic acid such as a DNA or RNA, for example an antisense DNA or RNA, a ribozyme, an RNA or DNA aptamer, siRNA, shRNA and the like, including variants or derivatives thereof such as a peptide nucleic acid (PNA); a genetic construct for targeted gene editing, such as a CRISPR/Cas9 construct and/or guide RNA/DNA (gRNA/gDNA) and/or tracrRNA; a hetero bi-functional compound such as a PROTAC or HyT molecule; a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, or small cell-permeable molecules.

In particular embodiments, the candidate compound is a PBC, such as one described elsewhere herein.

An in **a ninth aspect**, the invention provides a method for identifying and/or characterising a compound suitable for the treatment of a disease, disorder or condition that is associated with, or caused by, a bacterial cell expressing PBP2a, or a variant thereof, and/or that is characterised by antibiotic resistance against a treatment with a β-lactam antibiotic, the method comprising the steps of:
- bringing into contact a candidate compound and a bacterial cell expressing a protein of PBP2a (or a variant thereof), or a protein-fragment of PBP2a comprising, or consisting essentially of, a transpeptidase domain of PBP2a (or of the variant thereof) an; and
- determining the growth or viability of the bacterial cell of (a) in response to a treatment with a β-lactam antibiotic,
wherein a reduced growth or viability of the bacterial cell compared to a control indicates that the compound is suitable for the treatment of a disease, disorder or condition that is associated with, or caused by, a bacterial cell expressing PBP2a, or a variant thereof, and/or that is characterised by antibiotic resistance against a treatment with a beta-lactam antibiotic.

In certain embodiments it is preferred that the binding between the candidate compound and the transpeptidase domain of PBP2a involves a covalent or non-covalent interaction between the candidate compound and one of the following amino acids of *Staphylococcus aureus* PBP2a: K334, N338, N339, K341, N377, T384, E385, D386, K387, K388, E389, E391, N415, N416, K417, G440, Y441, Y444, Y446, K477, K484, K485, Y499, Q502, N505, N510, I512, L513, D516, G522, E523, L525, W558,3 K559, K560, N561, K584, E585, D586, K589, E602, L603, K604, G611, R612, K634, K639, Y644, K651, D654, E655, E658, N659, and/or E668, or an PBP2a amino acid located in 5 or less amino acid positions in N- or C-terminal direction.

Preferred candidate compounds are as described elsewhere herein.

Usually the detection of a methicillin resistance is based on determining the presence of the *mecA* gene in a sample of the subject to be diagnosed. The present invention however provides PBC specific and selective for PBP2a, or a variant thereof, which can be used for diagnostic purposes to detect, diagnose, or monitor diseases, disorders and/or conditions associated with the undesired presence of bacteria expressing PBP2a or a variant of PBP2a and/or associated with antibiotic resistance. The disease, disorder and/or conditions so detected, diagnosed, or monitored, can be one of those described elsewhere herein. In preferred embodiments of the detection and diagnosis methods of the invention, the diseases, disorders or conditions is an infectious disorder, such as an infection with MRSA.

Hence, the invention pertains in **a tenth aspect** to a diagnostic method for determining whether a subject has, or is at risk of, developing a disease, disorder or condition that is associated with the undesired presence of PBP2a-positive bacterial cells (or bacterial cells positive for a variant of PBP2a) and/or that is characterized by a resistance to a treatment with a β-lactam antibiotic, the method comprising the step of:
- detecting PBP2a (or a variant thereof), in particular the presence (or an amount) of or expression and/or activity of PBP2a (or a variant thereof), in a biological sample of the subject, for example a sample suspected to comprising bacterial cells of the disease, with a PBC of the invention,
wherein the detection of PBP2a (or the variant thereof) in the sample indicates such disease, disorder or condition, or a risk of developing such disease, disorder or condition, in the subject. In certain embodiments, the biological sample of the subject is suspected to comprise any bacterial material, such as living bacterial cells. In certain embodiments, the biological sample is one obtained from a mammalian subject like a human patient. The term "biological sample" is used in its broadest sense and can refer to a bodily sample obtained from the subject (e.g., a human patient). For example, the biological sample can include a clinical sample, i.e., a sample derived from a subject. Such samples can include, but are not limited to: peripheral bodily fluids, which may or may not contain cells, e.g., blood, urine, plasma, mucous, bile pancreatic juice, supernatant fluid, and serum; tissue or fine needle biopsy samples; tumour biopsy samples or sections (or cells thereof), and archival samples with known diagnosis, treatment and/or outcome history. Biological samples may also include sections of tissues, such as frozen sections taken for histological purposes. The term "biological sample" can also encompass any material derived by processing the sample. Derived materials can include, but are not limited to, cells (or their progeny) isolated from the biological sample, nucleic acids and/or proteins extracted from the sample. Processing of the biological sample may involve one or more of, filtration, distillation, extraction, amplification, concentration, fixation, inactivation of interfering components, addition of reagents, and the like.

In **another aspect**, the invention relates to a method of diagnosing and treating a disease, disorder or condition characterised by the undesired presence of PBP2a-positive bacterial cells (or bacterial cells positive for a variant of PBP2a) and/or by antibiotic resistance, (such as an infectious disorder, e.g., infection by MRSA) in a subject, such as a human patient, comprising:
- conducting a detection, determination and/or diagnostic method of the invention (such as one described above), thereby diagnosing if the subject is suffering from such a disease, disorder or condition; and
- administering an effective amount of a PBC of the invention (or another modulating compound of the invention), and/or a pharmaceutical composition of the invention, to the so diagnosed subject, in particular practicing a treatment method of the invention on the subject.

Accordingly, **in another aspect** the invention relates to a method of producing a recombinant cell line capable of expressing a PBC (preferably an affilin, antibody or nanobody, or their antigen binding fragments) specific for PBP2a, or for a PBP2a variant, the method comprising the steps of:
- providing a suitable host cell;
- providing at least one genetic construct comprising coding sequence(s) encoding the PBC of the present invention;
- introducing into said suitable host cell said genetic construct(s); and
- optionally, expressing said genetic construct(s) by said suitable host cell under conditions that allow for the expression of the PBC.

In yet **another aspect**, herein provided is a method of producing a PBC as described above, for example comprising culturing one or more cells of the invention under conditions allowing the expression of said ABP.

Accordingly, in another aspect, the invention relates to a method of producing a PBC specific for PBP2a, or for a PBP2a variant, the method comprising the steps of:
- providing a hybridoma or (host) cell capable of expressing a PBC according to the invention, for example a recombinant cell line comprising at least one genetic construct comprising coding sequence(s) encoding said compound or PBC; and
- culturing said hybridoma or host cell under conditions that allow for the expression of the PBC.

For producing the recombinant PBCs of the invention, the DNA molecules encoding the proteins (e.g., for antibodies, light and/or heavy chains or fragments thereof) are inserted into an expression vector (or NAC) such that the sequences are operatively linked to transcriptional and translational control sequences. Alternatively, DNA molecules encoding the PBC can be chemically synthesized. Synthetic DNA molecules can be ligated to other appropriate nucleotide sequences, including, e.g., constant region coding sequences, and expression control sequences, to produce conventional gene expression constructs encoding the desired PBC. For manufacturing the PBCs of the invention, the skilled artisan may choose from a great variety of expression systems well known in the art, e.g., those reviewed by Kipriyanow and Le Gall, 2004 (Kipriyanow & Gall, 2004). Expression vectors include, but are not limited to, plasmids, retroviruses, cosmids, EBV-derived episomes, and the like. The term "expression vector" or "NAC" comprises any vector suitable for the expression of a foreign DNA. Examples of such expression vectors are viral vectors, such as adenovirus, vaccinia virus, baculovirus and adeno-associated virus vectors. In this connection, the expression "virus vector" is understood to mean both a DNA and a viral particle. Examples of phage or cosmid vectors include pWE15, M13, 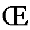 ^{a}EMBL3, 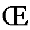 ^{a}EMBL4, 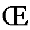 ^{a}FIXII, 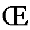 ^{a}DASHII, 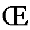 ^{a}ZAPII, 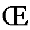 ^{a}gT10, 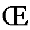 ^{a}gt11, Charon4A and Charon21A. Examples of plasmid vectors include pBR, pUC, pBluescriptII, pGEM, pTZ and pET groups. Various shuttle vectors may be used, e.g., vectors which may autonomously replicate in a plurality of host microorganisms such as *E. coli* and *Pseudomonas sp.* In addition, artificial chromosome vectors are considered as expression vectors. The expression vector and expression control sequences are selected to be compatible with the cell, such as a host cell. Examples of mammalian expression vectors include, but are not limited to, pcDNA3, pcDNA3.1(+/,àí), pGL3, pZeoSV2(+/,àí), pSecTag2, pDisplay, pEF/myc/cyto, pCMV/myc/cyto, pCR3.1, pSinRepS, D H26S, D HBB, pNMT1, pNMT41, pNMT81, which are available from Invitrogen™, pCI which is available from Promega, pMbac, pPbac, pBK-RSV and pBK-CMV which are available from Agilent Technologies, pTRES which is available from Clontech, and their derivatives.

For manufacturing antibodies, the antibody light chain gene and the antibody heavy chain gene can be inserted into separate vectors. In certain embodiments, both DNA sequences are inserted into the same expression vector. Convenient vectors are those that encode a functionally complete human CH or CL immunoglobulin sequence, with appropriate restriction sites engineered so that any VH or VL sequence can be easily inserted and expressed, as described above, wherein the CH1 and/or upper hinge region comprises at least one amino acid modification of the invention. The constant chain is usually kappa or lambda for the antibody light chain. The recombinant expression vector may also encode a signal peptide that facilitates secretion of the antibody chain from a (host) cell. The DNA encoding the antibody chain may be cloned into the vector such that the signal peptide is linked in-frame to the amino terminus of the mature antibody chain DNA. The signal peptide may be an immunoglobulin signal peptide or a heterologous peptide from a non-immunoglobulin protein. Alternatively, the DNA sequence encoding the antibody chain may already contain a signal peptide sequence.

In addition to the DNA sequences encoding the PBC (antibody) chains, the recombinant expression vectors carry regulatory sequences including promoters, enhancers, termination and polyadenylation signals and other expression control elements that control the expression of the antibody chains in a (host) cell. Examples for promoter sequences (exemplified for expression in mammalian cells) are promoters and/or enhancers derived from CMV (such as the CMV Simian Virus 40 (SV40) promoter/enhancer), adenovirus, (e.g., the adenovirus major late promoter (AdMLP)), polyoma and strong mammalian promoters such as native immunoglobulin and actin promoters. Examples for polyadenylation signals are BGH polyA, SV40 late or early polyA; alternatively, 3'UTRs of immunoglobulin genes etc. can be used.

The recombinant expression vectors may also carry sequences that regulate replication of the vector in (host) cells (e.g., origins of replication) and selectable marker genes. Nucleic acid molecules encoding the heavy chain or an antigen-binding portion thereof and/or the light chain or an antigen-binding portion thereof of an antibody of the present invention, and vectors comprising these DNA molecules can be introduced into (host) cells, e.g., bacterial cells or higher eukaryotic cells, e.g., mammalian cells, according to transfection methods well known in the art, including liposome-mediated transfection, polycation-mediated transfection, protoplast fusion, microinjections, calcium phosphate precipitation, electroporation or transfer by viral vectors.

For antibodies or fragments thereof, it is within ordinary skill in the art to express the heavy chain and the light chain from a single expression vector or from two separate expression vectors. Preferably, the DNA molecules encoding the heavy chain and the light chain are present on two vectors which are co-transfected into the (host) cell, preferably a mammalian cell

Mammalian cell lines available as hosts for expression are well known in the art and include, inter alia, Chinese hamster ovary (CHO, CHO-DG44, BI-HEX-CHO) cells, NSO, SP2/0 cells, HeLa cells, HEK293 cells, baby hamster kidney (BHK) cells, monkey kidney cells (COS), human carcinoma cells (e.g., Hep G2), A549 cells, 3T3 cells or the derivatives/progenies of any such cell line. Other mammalian cells, including but not limited to human, mice, rat, monkey and rodent cells lines, or other eukaryotic cells, including but not limited to yeast, insect and plant cells, or prokaryotic cells such as bacteria may be used. The antibody molecules of the invention are produced by culturing the host cells for a period of time sufficient to allow for expression of the antibody molecule in the host cells.

According to some embodiments of the method of producing a PBC, following expression, the intact antibody (or the antigen-binding fragment of the antibody) can be harvested and isolated using purification techniques well known in the art, e.g., Protein A, Protein G, affinity tags such as glutathione-S-transferase (GST) and histidine tags.

ABPs are preferably recovered from the culture medium as a secreted polypeptide or can be recovered from host cell lysates if for example expressed without a secretory signal. It is necessary to purify the ABP molecules using standard protein purification methods used for recombinant proteins and host cell proteins in a way that substantially homogenous preparations of the ABP are obtained. By way of example, state-of-the art purification methods useful for obtaining the ABP molecule of the invention include, as a first step, removal of cells and/or particulate cell debris from the culture medium or lysate. The PBC is then purified from contaminant soluble proteins, polypeptides and nucleic acids, for example, by fractionation on immunoaffinity or ion-exchange columns, ethanol precipitation, reverse phase HPLC, Sephadex chromatography, chromatography on silica or on a cation exchange resin. Preferably, ABPs are purified by standard protein A chromatography, e.g., using protein A spin columns (GE Healthcare). Protein purity may be verified by reducing SDS-PAGE. ABP concentrations may be determined by measuring absorbance at 280nm and utilizing the protein specific extinction coefficient. As a final step in the process for obtaining an ABP molecule preparation, the purified ABP molecule may be dried, e.g., lyophilized, for therapeutic applications.

Accordingly, certain embodiments of such aspects, the method comprises a further step of isolation and/or purification of the PBC.

In **another aspect**, herein provided is a method of manufacturing a pharmaceutical composition comprising a PBC as described above, comprising formulating the PBC isolated by the methods described above into a pharmaceutically acceptable form.

In an alternative aspect, herein provided is a method of manufacturing a pharmaceutical composition comprising a NAC as described above, comprising formulating the NAC prepared by the methods described above into a pharmaceutically acceptable form.

According to some embodiments, the methods of manufacturing a pharmaceutical composition comprise a further step of combining said PBC and/or NAC with a pharmaceutically acceptable excipient or carrier.

Accordingly, in certain embodiments of such aspects, the PBC is a modified antibody and the method comprises a further step of addition of a functional moiety selected from a detectable labelling group or a cytotoxic moiety.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g., "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:
**Item 1:** A protein binding compound (PBC) specifically binding to the transpeptidase domain of penicillin binding protein 2 a (PBP2a), wherein the PBC when bound to the transpeptidase domain of PBP2A increases sensitivity of PBP2a towards a β-lactam antibiotic compared to when the PBC is not bound to the transpeptidase domain of PBP2a.
**Item 2:** The PBC of item 1, wherein the transpeptidase domain of PBP2a is located between amino acids 250 and 668 of *Staphylococcus aureus* PBP2a, or is located in a homologous region of a homologous PBP2a protein.
**Item 3:** The PBC of item 1 or 2, wherein the β-lactam antibiotic is a penicillin or penicillin derivative, such as methicillin, amoxicillin, amoxicillin/clavulanate, ampicillin, ampicillin/sulbactam, bacampicillin, carbenicillin, cloxacillin, dicloxacillin, methicillin, mezlocillin, nafcillin, oxacillin, penicillin G, penicillin V, piperacillin, piperacillin/tazobactam, ticarcillin, ticarcillin/clavulanate.
**Item 4:** The PBC of any one of items 1 to 3, wherein the binding between the PBC and the transpeptidase domain of PBP2a involves a covalent or non-covalent interaction between the PBC and one of the following amino acids of *Staphylococcus aureus* PBP2A: K334, N338, N339, K341, N377, T384, E385, D386, K387, K388, E389, E391, N415, N416, K417, G440, Y441, Y444, Y446, K477, K484, K485, Y499, Q502, N505, N510, I512, L513, D516, G522, E523, L525, W558,3 K559, K560, N561, K584, E585, D586, K589, E602, L603, K604, G611, R612, K634, K639, Y644, K651, D654, E655, E658, N659, and/or E668, or an PBP2a amino acid located in 5 or less amino acid positions in N- or C-terminal direction.
**Item 5:** The PBC according to any one of items 1 to 4, wherein the PBC when bound to PBP2a increases binding of the β-lactam antibiotic to the PBP2a.
**Item 6:** The PBC of any one of items 1 to 5, wherein the PBC is a compound selected from a polypeptide, peptide, glycoprotein, a peptidomimetic, ubiquitin-like protein, an antibody or antibody-like molecule; a nucleic acid such as a DNA or RNA, including variants or derivatives thereof such as a peptide nucleic acid (PNA); a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, small cell-permeable molecules, and peptidomimetic compounds.
**Item 7:** The PBC of any one of items 1 to 6, which is a protein or polypeptide, or which comprises a protein or polypeptide, and preferably is an antibody, or an antigen binding derivative or fragment thereof, or is a T-cell receptor (TCR), or an antigen binding derivative or fragment thereof, or is a small protein-protein interacting polypeptide, such polypeptide derived from gamma-B crystalline or ubiquitin (affilin).
**Item 8:** A protein binding compound (PBC), which is an isolated PBC comprising an amino acid sequence at least 80% identical to a sequence according to any one of SEQ ID NO: 1 to 392.
**Item 9:** The PBC of item 1 which is a PBC of any one of items 1 to 7.
**Item 10:** The PBC of item 8 or 9, comprising at least one Complementary Determining Region (CDR) 3 having an amino acid sequence with at least 80% sequence identity to an amino acid sequence selected from SEQ ID Nos 6, 14, 22, 30, 38, 46, 54, 62, 70, 78, 86, 94, 102, 110, 118, 126, 134, 142, 150, 158, 166, 174, 182, 190, 198, 206, 214, 222, 230, 238, 246, 254, 262, 270, 278, 286, 294, 302, 310, 318, 326, 334, 342, 350, 358, 366, 374, 382 and 390.
**Item 11:** The PBC of item 9, wherein said PBC further comprises at least one CDR1, and at least one CDR2.
**Item 12:** The PBC of any one of items 8 to 10, wherein the PBC is an antibody or an antigen binding fragment thereof.
**Item 13:** The isolated PBC of any one of items 8 to 12, which is comprising an antibody heavy chain, or an antigen binding fragment thereof, and/or an antibody light chain, or an antigen binding fragment thereof.
**Item 14:** The isolated PBC of any of items 8 to 13, which (i) comprises an antibody heavy chain variable region, or an antigen binding fragment thereof, and an antibody light chain variable region, or an antigen binding fragment thereof, or is (ii) a nanobody comprising an antibody heavy chain variable region, or an antigen binding fragment thereof, and not comprising an antibody light chain variable region, or an antigen binding fragment thereof.
**Item 15:** The isolated antigen binding construct according to any of items 24 to 27, comprising an antibody heavy chain variable region, or an antigen binding fragment thereof, and/or an antibody light chain variable region, or an antigen binding fragment thereof.
**Item 16:** The isolated PBC of item 15, wherein in (i) the isolated PBC comprises an antibody heavy chain variable region CDR1, CDR2, and CDR3, and an antibody light chain variable region CDR1, CDR2, and CDR3, or wherein in (ii) the isolated PBC comprises an antibody heavy chain variable region CDR1, CDR2, and CDR3
**Item** 17: The isolated PBC of item 15 or 16, wherein in (i) the isolated PBC comprises an antibody heavy chain sequence and/or an antibody light chain sequence, or an antigen binding fragment thereof; wherein the antibody heavy chain sequence, or the fragment thereof, comprises a CDR3 having at least 80% sequence identity to an amino acid sequence selected from SEQ ID Nos. 6, 22, 38, 54, 70, 86, 102, 118, 134, 150, 166, 182, 198, 214, 230, and 246, and/or wherein antibody light chain sequence, or the fragment thereof, comprises a CDR3 having at least 80% sequence identity to an amino acid sequence selected from SEQ ID Nos. 14, 30, 46, 62, 78, 94, 110, 126, 142, 158, 174, 190, 206, 222, 238, and 254.
**Item 18:** The isolated PBC of any one of items 15 to 17, wherein in (i) the isolated PBC comprises an antibody heavy chain sequence, or the fragment thereof, comprising a CDR1 having at least 80% sequence identity to an amino acid sequence selected from SEQ ID Nos. 2, 18, 34, 50, 66, 82, 98, 114, 130, 146, 162, 178, 194, 210, 226, and 242; and/or a CDR2 having at 80% sequence identity to an amino acid sequence selected from SEQ ID Nos. 4, 20, 36, 52, 68, 84, 100, 116, 132, 148, 164, 180, 196, 212, 228, and 244.
**Item 19:** The isolated PBC of any one of items 15 to 18, wherein in (i) the isolated PBC comprises an the antibody light chain sequence, or the fragment thereof, comprising a CDR1 having at least 80% sequence identity to an amino acid sequence selected from SEQ ID Nos. 10, 26, 42, 58, 74, 90, 106, 122, 138, 154, 170, 186, 202, 218, 234, and 250; and/or a CDR2 having at least 80% sequence identity to an amino acid sequence selected from SEQ ID Nos. 12, 28, 44, 60, 76, 92, 108, 124, 140, 156, 172, 188, 204, 220, 236, and 252.
**Item 20**: The isolated PBC of any one of the preceding items, wherein the PBC is an antibody, or an antigen binding fragment thereof, composed of at least one antibody heavy chain sequences, and at least one antibody light chain sequences, wherein at least one of said antibody heavy chain sequences and antibody light chain sequences comprise CDR1 to CDR3 sequences in the following combination:

| | **Heavy Chain CDR1 to CDR3 (SEQ ID NO)** | | | **Light Chain CDR1 to CDR3 (SEQ ID NO)** | | |
|---|---|---|---|---|---|---|
| **AB001** | 2 | 4 | 6 | 10 | 12 | 14 |
| **AB002** | 18 | 20 | 22 | 26 | 28 | 30 |
| **AB003** | 34 | 36 | 38 | 42 | 44 | 46 |
| **AB004** | 50 | 52 | 54 | 58 | 60 | 62 |
| **AB005** | 66 | 68 | 70 | 74 | 76 | 78 |
| **AB006** | 82 | 84 | 86 | 90 | 92 | 94 |
| **AB007** | 98 | 100 | 102 | 106 | 108 | 110 |
| **AB008** | 114 | 116 | 118 | 122 | 124 | 126 |
| **AB009** | 130 | 132 | 134 | 138 | 140 | 142 |
| **AB010** | 146 | 148 | 150 | 154 | 156 | 158 |
| **AB011** | 162 | 164 | 166 | 170 | 172 | 174 |
| **AB012** | 178 | 180 | 182 | 186 | 188 | 190 |
| **AB013** | 194 | 196 | 198 | 202 | 204 | 206 |
| **AB014** | 210 | 212 | 214 | 218 | 220 | 222 |
| **AB015** | 226 | 228 | 230 | 234 | 236 | 238 |
| **AB016** | 242 | 244 | 246 | 250 | 252 | 254 |

in each case independently, optionally with not more than three or two, preferably one, amino acid substitution(s), insertion(s) or deletion(s) compared to these sequences.
**Item 21:** The isolated PBC of any one of the preceding items, wherein the PBC is an antibody, or an antigen binding fragment thereof, composed of at least one antibody heavy chain sequence, and at least one antibody light chain sequence, wherein said antibody heavy chain sequence and the antibody light chain sequence comprises each a variable region sequences of the following combination:

| | **Heavy Chain Variable Region (SEQ ID NO)** | **Light Chain Variable Region (SEQ ID NO)** |
|---|---|---|
| **AB001** | 8 | 16 |
| **AB002** | 24 | 32 |
| **AB003** | 40 | 48 |
| **AB004** | 56 | 64 |
| **AB005** | 72 | 80 |
| **AB006** | 88 | 96 |
| **AB007** | 104 | 112 |
| **AB008** | 120 | 128 |
| **AB009** | 136 | 144 |
| **AB010** | 152 | 160 |
| **AB011** | 168 | 176 |
| **AB012** | 184 | 192 |
| **AB013** | 200 | 208 |
| **AB014** | 216 | 224 |
| **AB015** | 232 | 240 |
| **AB016** | 248 | 256 |

in each case independently, optionally with not more than three or two, preferably one, amino acid substitution(s), insertion(s) or deletion(s) compared to these sequences.
**Item 22:** The isolated PBC of item 15 or 16, wherein in (ii) the isolated PBC comprises a nanobody heavy chain sequence, or an antigen binding fragment thereof; wherein the nanobody heavy chain sequence, or the fragment thereof, comprises a CDR3 having at least 80% sequence identity to an amino acid sequence selected from SEQ ID Nos. 262, 270, 278, 286, 294, 302, 310, 318, 326, 334, 342, 350, 358, 366, 374, 382 and 390.
**Item 23:** The isolated PBC of item 22, further comprising a CDR1 having at least 80% sequence identity to an amino acid sequence selected from SEQ ID Nos. 258, 266, 274, 282, 290, 298, 306, 314, 322, 330, 338, 346, 354, 362, 370, 378 and 386; and/or a CDR2 having at least 80% sequence identity to an amino acid sequence selected from SEQ ID Nos. 260, 268, 276, 284, 292, 300, 308, 316, 324, 332, 340, 348, 356, 364, 372, 380 and 388.
**Item 24:** The isolated PBC of any one of items 21 to 23, wherein the PBC is a nanobody, or an antigen binding fragment thereof, composed of an antibody heavy chain sequence comprising CDR1 to CDR3 sequences in the following combination:

| | **Heavy Chain CDR1 to CDR3 (SEQ ID NO)** | | |
|---|---|---|---|
| **NB001** | 258 | 260 | 262 |
| **NB002** | 266 | 268 | 270 |
| **NB003** | 274 | 276 | 278 |
| **NB004** | 282 | 284 | 286 |
| **NB005** | 290 | 292 | 294 |
| **NB006** | 298 | 300 | 302 |
| **NB007** | 306 | 308 | 310 |
| **NB008** | 314 | 316 | 318 |
| **NB009** | 322 | 324 | 326 |
| **NB010** | 330 | 332 | 334 |
| **NB011** | 338 | 340 | 342 |
| **NB012** | 346 | 348 | 350 |
| **NBO13** | 354 | 356 | 358 |
| **NB014** | 362 | 364 | 366 |
| **NB015** | 370 | 372 | 374 |
| **NB016** | 378 | 380 | 382 |
| **NB017** | 386 | 388 | 390 |

in each case independently, optionally with not more than three or two, preferably one, amino acid substitution(s), insertion(s) or deletion(s) compared to these sequences.
**Item 25:** The isolated PBC of any one of items 21 to 24, wherein the PBC is a nanobody, or an antigen binding fragment thereof, composed of an antibody heavy chain sequence comprising any one of the sequences selected from SEQ ID Nos 264, 272, 280, 288, 296, 304, 312, 320, 328, 336, 344, 352, 360, 368, 376, 384 and 392, in each case independently, optionally with not more than ten, nine, eight, five, three or two, preferably one, amino acid substitution(s), insertion(s) or deletion(s) compared to these sequences.
**Item 26:** An isolated PBC, which competes with an PBC as recited in any one of items 10 to 25 for binding to the transpeptidase domain of PBP2a, preferably of *Staphylococcus aureus,* and is capable of increasing sensitivity of said PBP2a to a β-lactam antibiotic, preferably capable of increasing of the binding of the β-lactam antibiotic to the PBP2a.
**Item 27:** The PBC of any one of the preceding items, that enhances β-lactam antibiotic induced killing, lysing, or reducing the growth, of a bacterial cell expressing PBP2a.
**Item 28:** An isolated nucleic acid encoding for a PBC which is a protein or polypeptide according to any one of the preceding items.
**Item 29:** A recombinant host cell, comprising a PBC or an isolated nucleic acid according to any one of the preceding items.
**Item 30:** A pharmaceutical composition comprising a PBC recited in any of items 1 to 27, an isolated nucleic acid recited in item 28, a recombinant host cell recited in item 29, together with a pharmaceutically acceptable carrier and/or excipient.
**Item 31:** The pharmaceutical composition according to item 30, further comprising an additional active ingredient, preferably selected from a β-lactam antibiotic.
**Item 32:** The pharmaceutical composition of item 31, wherein the β-lactam antibiotic is a penicillin or penicillin derivative, such as methicillin, amoxicillin, amoxicillin/clavulanate, ampicillin, ampicillin/sulbactam, bacampicillin, carbenicillin, cloxacillin, dicloxacillin, methicillin, mezlocillin, nafcillin, oxacillin, penicillin G, penicillin V, piperacillin, piperacillin/tazobactam, ticarcillin, or ticarcillin/clavulanate.
**Item 33:** A product for use in medicine, wherein the product is selected from a PBC recited in any of items 1 to 27, an isolated nucleic acid recited in item 28, a recombinant host cell recited in item 29, and a pharmaceutical composition of any one of items 30 to 32.
**Item 34:** The product for use of item 33, wherein the product is for use in the treatment or prevention of an infectious disease, such as bacterial infection, most preferably of a gram positive bacterium such as *Staphylococcus aureus.*
**Item 35:** The product for use of item 34, wherein the infectious disease is methicillin resistant *Staphylococcus aureus* (MRSA).
**Item 36:** The product for use of item 33, wherein the product is for use in the prevention of a development of an antibiotic resistant bacterial disease, such as MRSA.
**Item 37:** The product for use of any one of items 33 to 35, wherein the use involves administration of a therapeutically effective or preventive amount of the product to a subject suffering from, or suspected to suffer from, the disease.
**Item 38:** An in-vitro method for identifying and/or characterising a compound suitable for the treatment of a disease, disorder or condition that is associated with, or caused by, a bacterial cell expressing PBP2a, or a variant thereof, and/or that is characterised by antibiotic resistance against a treatment with a β-lactam antibiotic, the method comprising the steps of:
(a) bringing into contact a candidate compound and a protein of PBP2a (or a variant thereof), or a protein-fragment of PBP2a comprising, or consisting essentially of, a transpeptidase domain of PBP2a (or of the variant thereof) an; and
(b) detecting and/or quantifying a binding of the candidate compound to the transpeptidase domain of the protein of PBP2a (or the variant thereof), or the protein-fragment of PBP2a (or of the variant thereof),
wherein a binding of the candidate compound to the transpeptidase domain of PBP2a (or the variant thereof) compared to a control indicates that the compound is suitable for the treatment of a disease, disorder or condition that is associated with, or caused by, a bacterial cell expressing PBP2a, or a variant thereof, and/or that is characterised by antibiotic resistance against a treatment with a β-lactam antibiotic.
**Item 39:** An in-vitro method for identifying and/or characterising a compound suitable for the treatment of a disease, disorder or condition that is associated with, or caused by, a bacterial cell expressing PBP2a, or a variant thereof, and/or that is characterised by antibiotic resistance against a treatment with a β-lactam antibiotic, the method comprising the steps of:
**(a)** bringing into contact a candidate compound and a bacterial cell expressing a protein of PBP2a (or a variant thereof), or a protein-fragment of PBP2a comprising, or consisting essentially of, a transpeptidase domain of PBP2a (or of the variant thereof) an; and
**(b)** determining the growth or viability of the bacterial cell of (a) in response to a treatment with a β-lactam antibiotic,
wherein a reduced growth or viability of the bacterial cell compared to a control indicates that the compound is suitable for the treatment of a disease, disorder or condition that is associated with, or caused by, a bacterial cell expressing PBP2a, or a variant thereof, and/or that is characterised by antibiotic resistance against a treatment with a β-lactam antibiotic.
**Item 40:** The method according to item 38 or 39, wherein the binding between the candidate compound and the transpeptidase domain of PBP2a involves a covalent or noncovalent interaction between the candidate compound and one of the following amino acids of *Staphylococcus aureus* PBP2a: K334, N338, N339, K341, N377, T384, E385, D386, K387, K388, E389, E391, N415, N416, K417, G440, Y441, Y444, Y446, K477, K484, K485, Y499, Q502, N505, N510, I512, L513, D516, G522, E523, L525, W558,3 K559, K560, N561, K584, E585, D586, K589, E602, L603, K604, G611, R612, K634, K639, Y644, K651, D654, E655, E658, N659, and/or E668, or an PBP2a amino acid located in 5 or less amino acid positions in N- or C-terminal direction.
**Item 41:** The method according to any one of items 38 to 40, wherein the candidate compound is selected from the group of compound classes consisting of a polypeptide, peptide, glycoprotein, a peptidomimetic, ubiquitin-like protein, an antibody or antibody-like molecule; a nucleic acid such as a DNA or RNA, including variants or derivatives thereof such as a peptide nucleic acid (PNA); a carbohydrate such as a polysaccharide or oligosaccharide and the like, including variants or derivatives thereof; a lipid such as a fatty acid and the like, including variants or derivatives thereof; or a small organic molecules including but not limited to small molecule ligands, small cell-permeable molecules, and peptidomimetic compounds.
**Item 42:** An in-vitro diagnostic method for determining whether a subject has, or is at risk of, developing a disease, disorder or condition that is associated with the undesired presence of PBP2a-positive cells (or cells positive for a variant of PBP2a) and/or that is characterized by a resistance to a treatment with a β-lactam antibiotic, the method comprising the step of:
**(i)** detecting PBP2a (or a variant thereof), in particular the presence (or an amount) of or expression and/or activity of PBP2a (or a variant thereof), in a biological sample of the subject, for example a sample suspected to comprising bacterial cells of the disease, with a PBC of any one of items 1 to 27,
wherein the detection of PBP2a (or the variant thereof) in the sample indicates such disease, disorder or condition, or a risk of developing such disease, disorder or condition, in the subject.

### BRIEF DESCRIPTION OF THE FIGURES, TABLES AND SEQUENCES

The figures show:

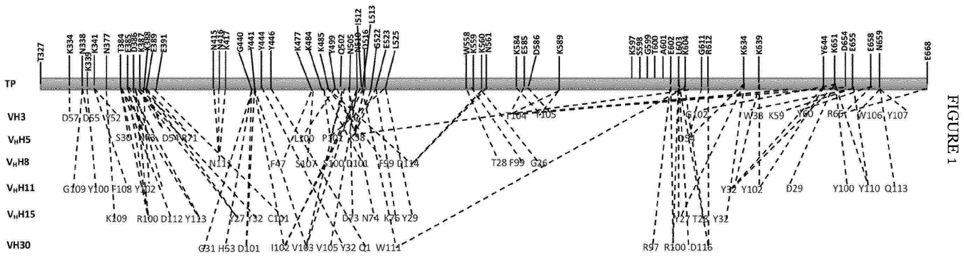

**Figure 1** shows the interaction residues between PBP2a-TP and nanobodies. Schematic overview of the interaction between PBP2a-TP and nanobodies. The amino acid residues of each nanobody (blue) interacted with their target amino acid residues of PBP2a-TP (black).

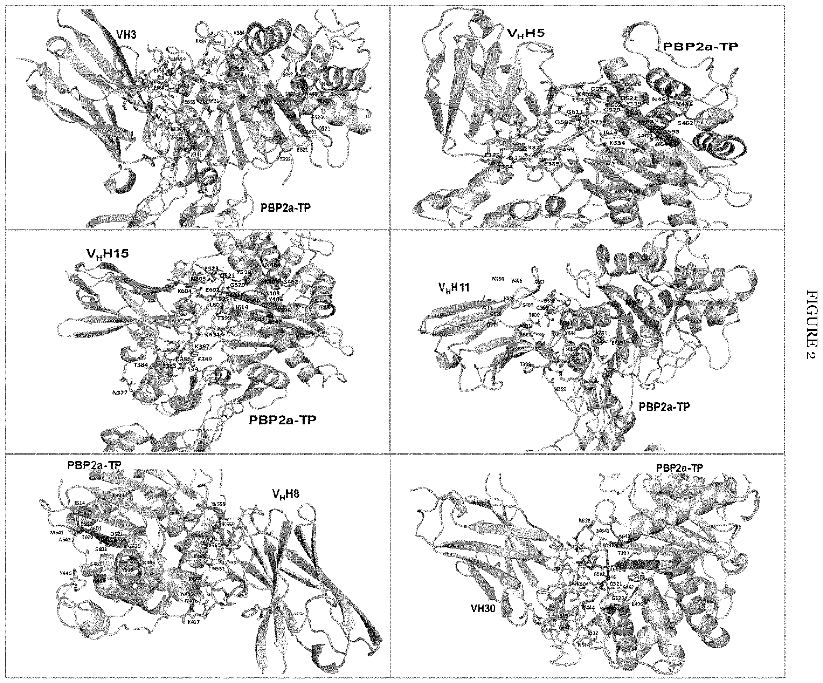

**Figure 2** shows the interactions between the PBP2a-TP and nanobodies. Details of the interactive amino acid residues the PBP2a-TP protein (pink) and the transpeptidase specific-nanobodies (green); i. e., VH3, V_{H}H5, V_{H}H8, V_{H}H11, V_{H}H15 and VH30 (see also Table 1-6).

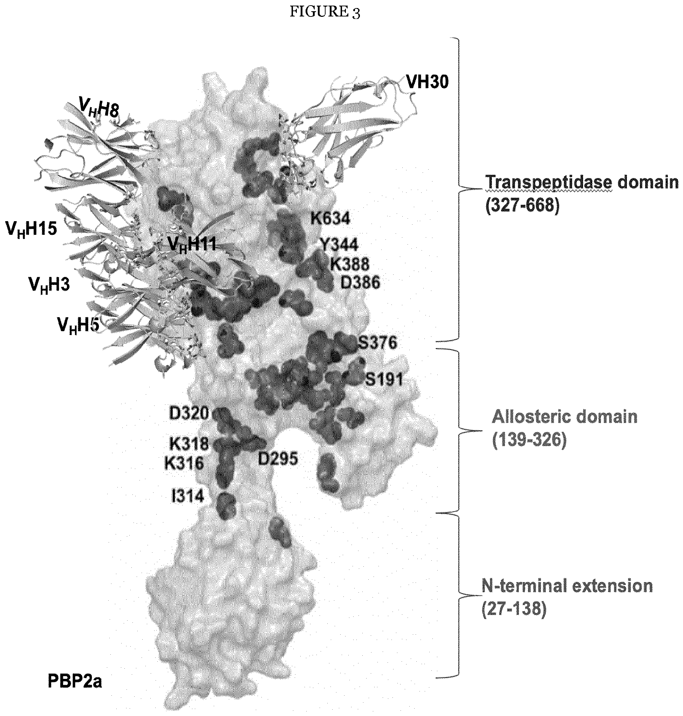

**Figure 3** shows the interaction complex of the PBP2a-TP and nanobodies. The PBP2a (translucent gray surface) in complex with nanobodies (green ribbons). Crystal structure of the PBP2a shows the predicted allosteric surface-critical (red sphere representation) and interior-critical residues (blue sphere representation) mapped on the PBP2a apo conformation (Mahasenan *et al.*, 2017).

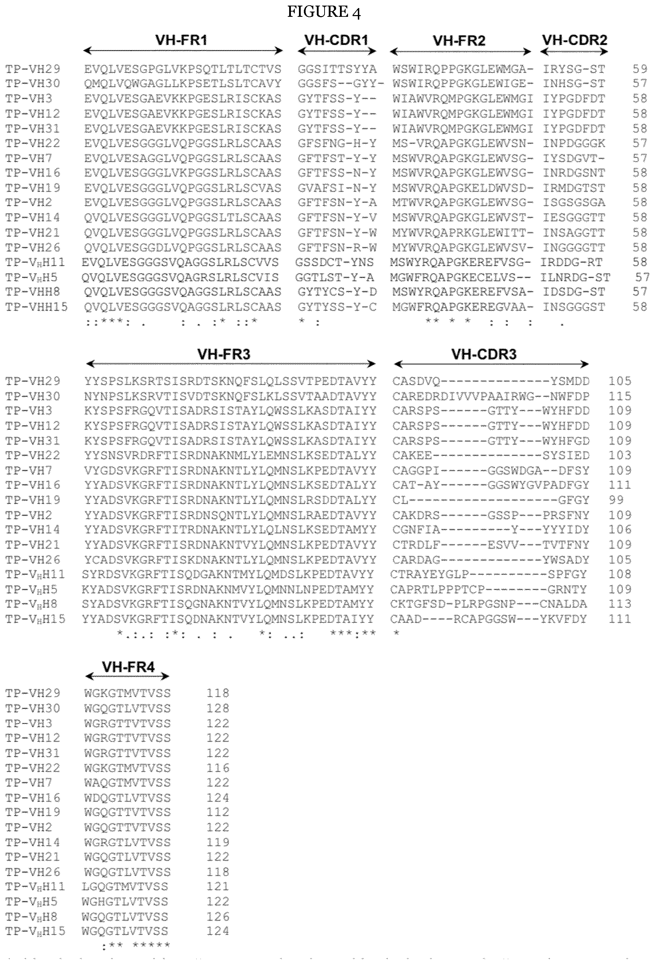

**Figure 4** shows the sequence domains and the alignment of amino acid sequences of the PBP2a-TP specific nanobodies. Deduced amino acid sequences of 17 PBP2a-TP specific nanobodies and their respective immunoglobulin frame works (FRs) and complementarity determining regions (CDRs). mark*, identical amino acids; ":", conserved amino acid substitution; and ".", semi-conserved amino acid substitution.

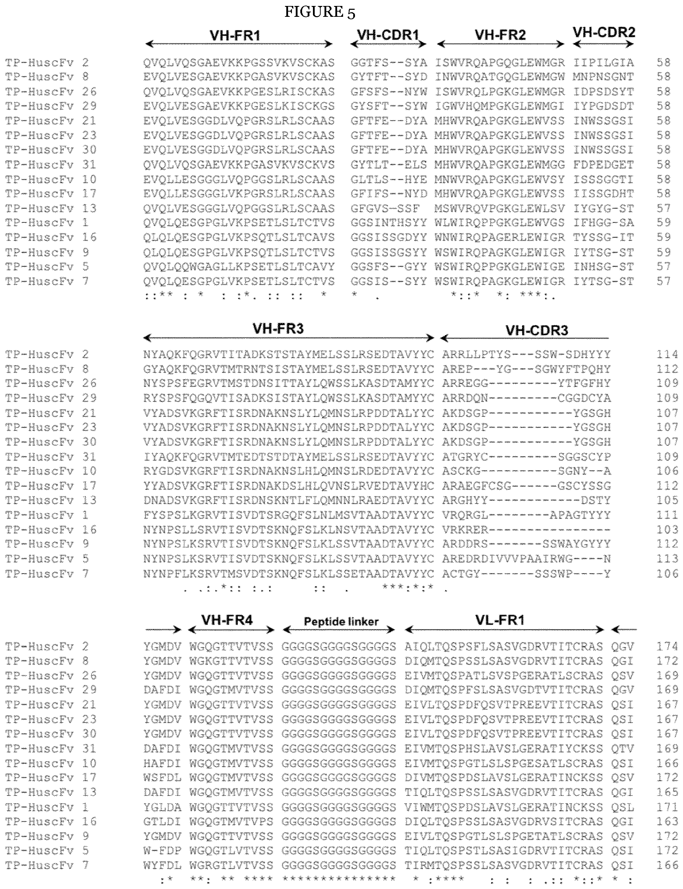

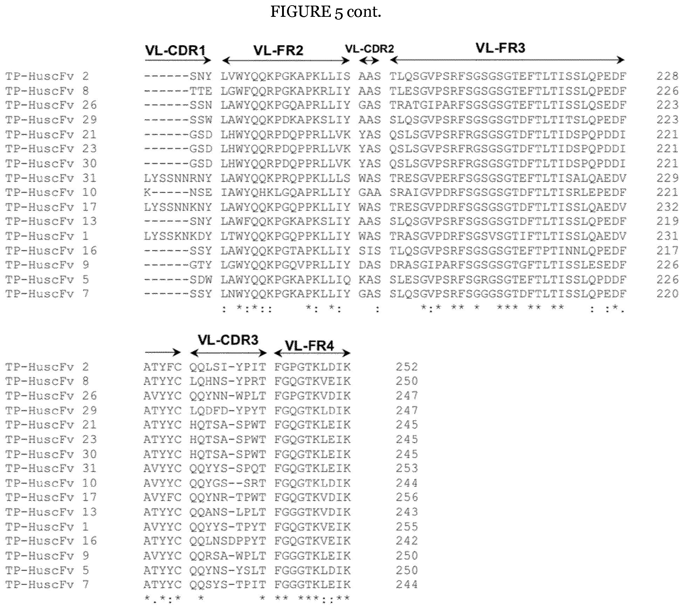

**Figure 5** shows the sequence domains and the alignment of amino acid sequences of the PBP2a-TP specific HuscFvs. Deduced amino acid sequences of 16 PBP2a-TP specific HuscFvs and their respective immunoglobulin frame works (FRs) and complementarity determining regions (CDRs). *, identical amino acids; ":", conserved amino acid substitution; and ".", semi-conserved amino acid substitution.

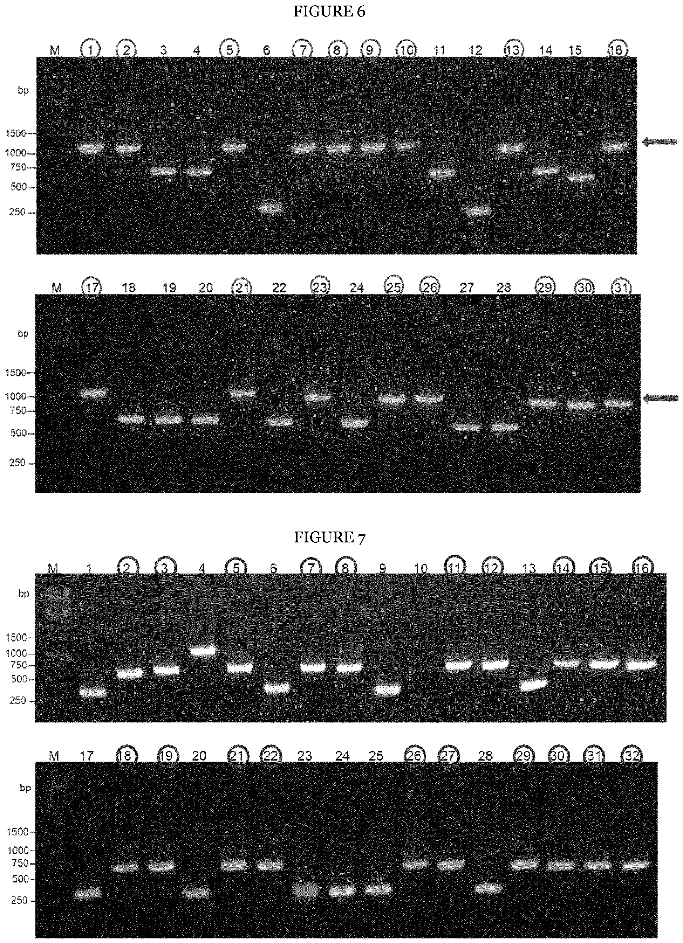

**Figure 6** shows the PCR amplicons of the randomly picked huscfv-phagemid transfom HB2151 *E. coli.* PCR screening of the presence of huscfv sequences (∼1000) in 31 randomly picked huscfv-phagemid transfomed *E. coli* HB2151 colonies. Lane M, GenRuler 1 kb DNA ladder. Lanes 1-31, PCR amplicons of huscfv-phagemid transfom HB2151 *E. coli* colonies 1-31. The PCP positive (red circles) were lanes 1, 2, 5, 7, 8, 9, 10, 13, 16, 17, 21, 23, 25, 26, 29, 30 and 31, respectively. They were named clone nos. 1, 2, 5, 7, 8, 9, 10, 13, 16, 17, 21, 23, 25, 26, 29, 30 and 31, respectively. Numbers at the left are DNA sizes in bp.
**Figure 7** shows the PCR amplicons of the randomly picked vh/vhh-phagemid transfom HB2151 *E. coli.* PCR screening of the presence of vh/vhh sequences (∼600) in 32 randomly picked vh/vhh-phagemid transfomed *E. coli* HB2151 colonies. Lane M, GenRuler TM 1 kb DNA ladder. Lanes 1-32, PCR amplicons of vh/vhh-phagemid transfom HB2151 *E. coli* colonies 1-32. The PCP positive (red circles) were lanes 2, 3, 5, 7, 8, 11, 12, 14, 15, 16, 18, 19, 21, 22, 26, 27, 29, 30, 31 and 32 respectively. They were named clone nos. 2, 3, 5, 7, 8, 11, 12, 14, 15, 16, 18, 19, 21, 22, 26, 27, 29, 30, 31 and 32 respectively. Numbers at the left are DNA sizes in bp.

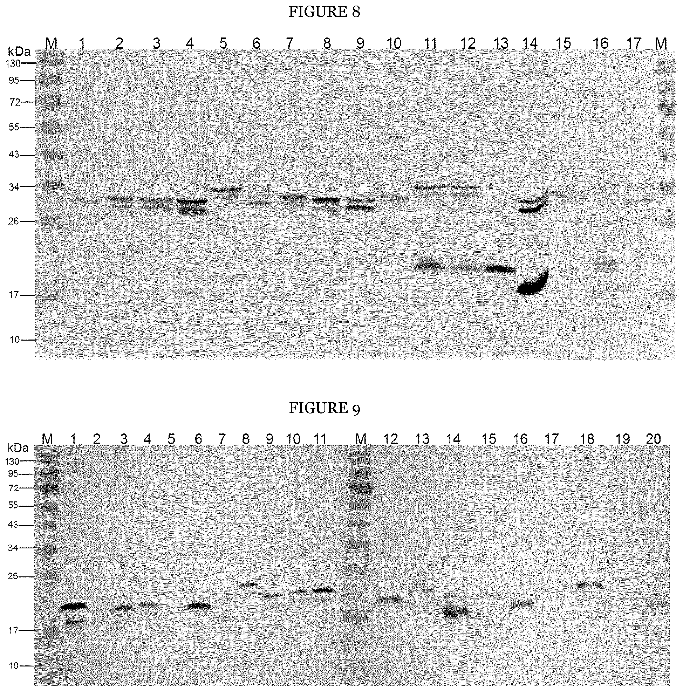

**Figure 8** shows the detection of HuscFvs in lysate of huscfv-phagemid transfomed *E*. *coli* HB2151 clones by Western Blot analysis. Western blot result for detecting the HuscFvs in lysate of huscfv-phagemid transfomed *E. coli* HB2151 clones. The protein was loaded 10 µl per well. Lane M: PageRulerTM Prestained Protein Ladder. Lanes1-17: HuscFvs (∼27-30 kDa) produced in lysates of E. coli clone nos.1, 2, 5, 7, 8, 9, 10, 13, 16, 17, 21, 23, 25, 26, 29, 30 and 31, respectively. Numbers at the left are molecular masses of standard proteins in kDa.
**Figure 9** shows the detection of VHs/VHHs in lysate of vh/vhh-phagemid transfomed *E. coli* HB2151 clones by Western Blot analysis. Western blot result for detecting the VHs/VHHs in lysate of vh/vhh-phagemid transfomed *E. coli* HB2151 clones. The protein was loaded 10 µl per well. Lane M: PageRulerTM Prestained Protein Ladder. Lanes1-20: VHs/VHHs (∼15-22 kDa) produced in lysates of *E. coli* clone nos. 2, 3, 5, 7, 8, 11, 12, 14-16, 18-19, 21-22, 26-27 and 29-32, respectively. Numbers at the left are molecular masses of standard proteins in kDa.

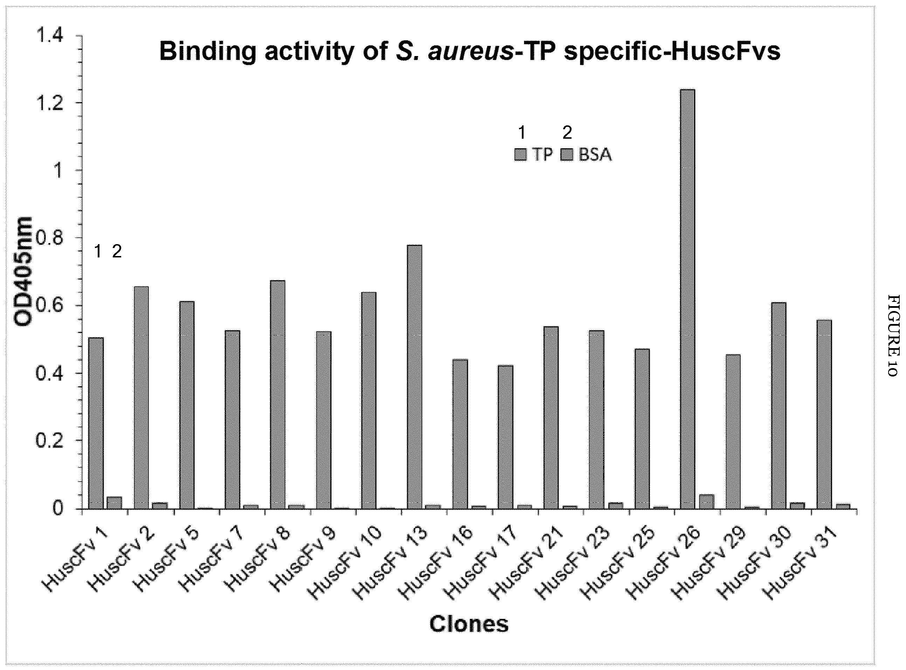

**Figure 10** shows ELISA result to determine the binding activity of HuscFvs to rPBP2a-TP. Indirect ELISA for determining the binding activity of HuscFvs to PBP2a-TP. The HuscFvs that gave the OD405nm values to rPBP2a-TP at least two times higher than to BSA control were rPBP2a-TP-specific VHs/V_{H}Hs. They were HuscFv nos. 1, 2, 5, 7, 8, 9, 10, 13, 16, 17, 21, 23, 25, 26, 29, 30, and 31, respectively.

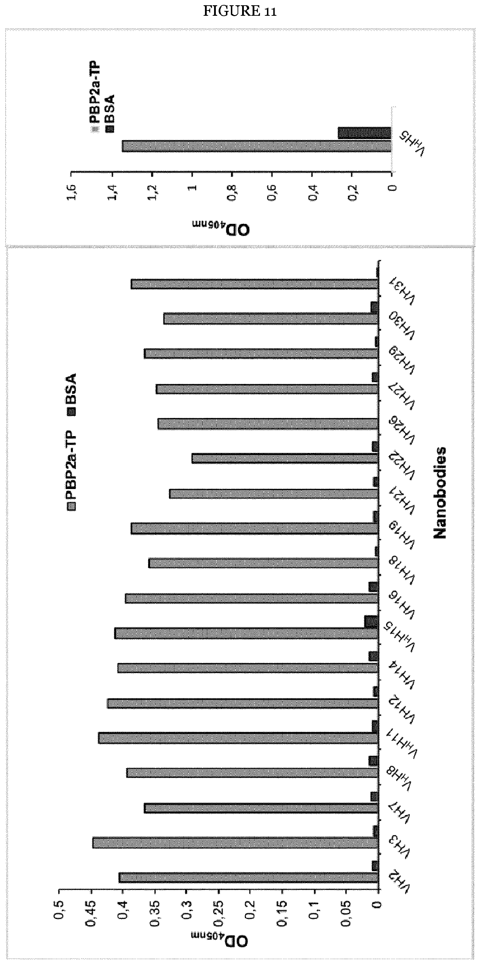

**Figure 11** shows ELISA result to determine the binding activity of VHs/V_{H}Hs to rPBP2a-TP. Indirect ELISA for determining the binding activity of VHs/V_{H}Hs to PBP2a-TP. The VHs/V_{H}Hs that gave the OD405nm values to rPBP2a-TP at least two times higher than to BSA control were rPBP2a-TP-specific VHs/V_{H}Hs. They were VH2, VH3, VH5, VH7, V_{H}H8, V_{H}H11, VH12, VH14, V_{H}H15, VH16, VH18, VH19, VH21, VH22, VH26, VH27, VH29, VH30, and VH31, respectively.

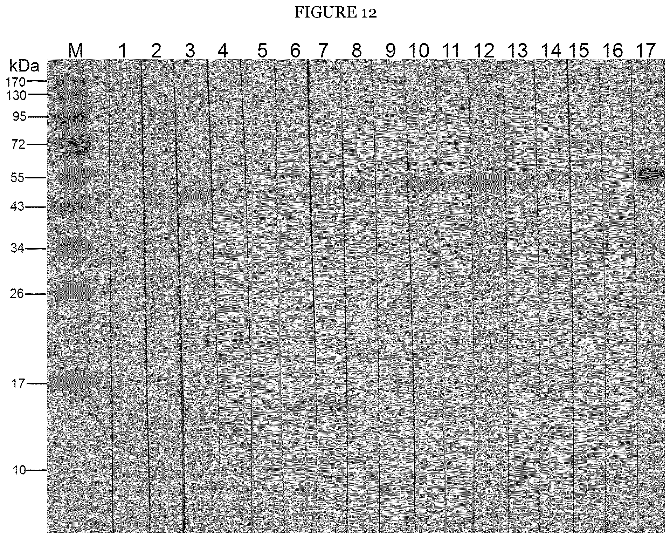

**Figure 12** shows the Binding activity of soluble HuscFvs to PBP2a-TP by Western Blot analysis. Western blotting results for determining the binding of HuscFvs to rPBP2a-TP. HuscFvs in lysates of the 17 ELISA-positive clones bound specifically to rPBP2a-TP blotted strips. Lane 1-15, rPBP2a-TP blotted strips probed with *E. coli* lysates containing the HuscFvs. Lane 16, rPBP2a-TP blotted strip probed lysate of original HB2151 *E. coli* (HuscFv negative control). Lane 14, rPBP2a-TP blotted strip probed with mouse anti-6x His Tag (positive binding control). Numbers at the left are molecular masses of standard proteins in kDa.

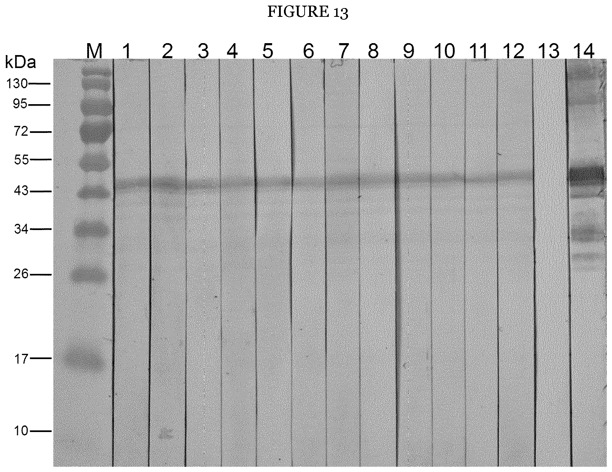

**Figure 13** shows the Binding activity of soluble VHs/VHHs to PBP2a-TP by Western Blot analysis. Western blotting results for determining the binding of VHs/VHHs to *S. aureus-*TP. VHs/VHHs in lysates of the 12 ELISA-positive clones bound specifically to *S. aureus*-TP blotted strips (lanes 1-12). Lane 1-12, rPBP2a -TP blotted strips probed with *E. coli* lysates containing the soluble VHs/VHHs. Lane 13, rPBP2a -TP blotted strip probed lysate of original HB2151 *E. coli* (VH/V_{H}H negative control). Lane 14, rPBP2a-TP blotted strip probed with mouse anti-6x His Tag (positive binding control). Numbers at the left are molecular masses of standard proteins in kDa.

### The sequences show:

SEQ ID NOs. 1 to 392 show the chain identities, sequence domains and amino acid sequences of the HuscFvs and nanobodies. The details of the sequences are as follows:
**Sequence No. 393:** a nucleotide sequence of PBP2a-Transpeptidase (5' to 3') 
gcacatacattaatagagaaaagaaaaaagatggcaaagatattcaactaactattgatgctaaagttcaaaagagtatttataacaacat
gaaaaatgattatggctcaggtactgctatccaccctcaaacaggtgaattattagcacttgtaagcacaccttcatatgacgtctatccattta
tgtatggcatgagtaacgaagaatataataaattaaccgaagataaaaaagaacctctgctcaacaagttccagattacaacttcaccaggt
tcaactcaaaaaatattaacagcaatgattgggttaaataacaaaacattagacgataaaacaagttataaaatcgatggtaaaggttggca
aaaagataaatcttggggtggttacaacgttacaagatatgaagtggtaaatggtaatatcgacttaaaacaagcaatagaatcatcagata
acattttctttgctagagtagcactcgaattaggcagtaagaaatttgaaaaaggcatgaaaaaactaggtgttggtgaagatataccaagtg
attatccattttataatgctcaaatttcaaacaaaaatttagataatgaaatattattagctgattcaggttacggacaaggtgaaatactgatt
aacccagtacagatcctttcaatctatagcgcattagaaaataatggcaatattaacgcacctcacttattaaaagacacgaaaaacaaagtt
tggaagaaaaatattatttccaaagaaaatatcaatctattaactgatggtatgcaacaagtcgtaaataaaacacataaagaagatatttat
agatcttatgcaaacttaattggcaaatccggtactgcagaactcaaaatgaaacaaggagaaactggcagacaaattgggtggtttatatc
atatgataaagataatccaaacatgatgatggctattaatgttaaagatgtacaagataaaggaatggctagctacaatgccaaaatctcag
gtaaagtgtatgatgagctatatgagaacggtaataaaaaatacgatatagatgaataa-3’
**Sequence no. 394:** an amino acid sequence of PBP2a-Transpeptidase (N-terminus to C-terminus)

AHTLIEKKKKDGKDIQLTIDAKVQKSIYNNMKNDYGSGTAIHPQTGELLALVSTPSYDVYPFMY
GMSNEEYNKLTEDKKEPLLNKFQITTSPGSTQKILTAMIGLNNKTLDDKTSYKIDGKGWQKDK
SWGGYNVTRYEVVNGNIDLKQAIESSDNIFFARVALELGSKKFEKGMKKLGVGEDIPSDYPFYN
AQISNKNLDNEILLADSGYGQGEILINPVQILSIYSALENNGNINAPHLLKDTKNKVWKKNIISK
ENINLLTDGMQQVVNKTHKEDIYRSYANLIGKSGTAELKMKQGETGRQIGWFISYDKDNPNM
MMAINVKDVQDKGMASYNAKISGKVYDELYENGNKKYDIDE

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples. Internal IDs of antibodies and nanobodies used in the following examples and the figures correspond to the molecules of the invention, and the sequences of the sequence listing as well as tables 11 and 12 according to table 13 below.

### The examples show:

### Example 1: Preparation of antibodies and nanobodies using the transpeptidase domain of PBP2a.

The phage clones displaying specific human scFv (HuscFvs) and VHs/V_{H}Hs that bound to the PBP2a-TP of *Staphylococcus aureus* USA300 (MRSA) were selected from previous HuscFv phage display library (Kulkeaw *et al.,* 2009) and a humanized-camel VH/V_{H}H phage display library (Thanongsaksrikul *et al.,* 2010) by using the purified rPBP2-TP as target antigen in the phage bio-panning process. Briefly, 5 µg of the purified rPBP2a-TP was coated onto an ELISA well surface and the HuscFv/VH/V_{H}H phage display library was added to the well. After an incubation, antigen unbound phages were removed by washing thoroughly and the bound phages were immediately supplemented with a log phase grown *E. coli* HB2151. The phagemid transformed *E. coli* clones were grown overnight on a selective agar plate (2×YT agar containing 100 µg/mL ampicillin and 2% glucose; 2×YT -AG). *E. coli* clones were randomly selected and screened for the presence of recombinant huscfv/*v*h/*v*hh-phagemids by PCR.

The PCR amplicons of the randomly picked huscfv-phagemid transfom HB2151 *E. coli* is shown in figure 6. In figure 7 the PCR amplicons of the randomly picked vh/vhh-phagemid transfom HB2151 *E. coli.*

The *E. coli* carrying *huscfv*/*vh*/*vhh-*phagemids were grown individually under 0.5 mM IPTG induction for production of soluble E-tag-HuscFv/VHs/V_{H}Hs. The presence of the HuscFvs and nanobodies in the bacterial lysates was detected by SDS-PAGE and Western blot analysis using rabbit anti-E Tag antibody as a detection reagent. Goat anti-rabbit immunoglobulin-alkaline phosphatase (AP) conjugate was used as an anti-isotype antibody and BCIP/NBT™ substrate was used as a chromogenic substrate.

Western blot shows the detection of scFv and VHH (Figures 8 and 9).

**Table 4: The residues of the PBP2a-TP bound by the residues of the PBP2a-TP specific VH3.**

| **PBP2a-TP** | **TP-VH3** | | **Intermolecular bonds** |
|---|---|---|---|
| **Residues** | **Amino acids** | **Domains** | |
| K334 | D57 | CDR2 | H-bond/ionic |
| N338 | D55 | CDR2 | H-bond |
| K341 | Y52 | FR2 | H-bond/cationic- |
| K584 | Y105 | CDR3 | H-bond |
| E585 | T104 | FR3 | H-bond |
| D586 | Y105 | CDR3 | H-bond |
| R589 | T104 | FR3 | H-bond |
| | Y105 | CDR3 | H-bond/cationic- |
| K651 | G102 | FR3 | H-bond |
| D654 | T104 | FR3 | H-bond |
| E655 | W33 | CDR1 | H-bond |
| | K59 | CDR2 | Ionic |
| E658 | Y60 | CDR2 | H-bond |
| | R65 | CDR2 | H-bond/ionic |
| N659 | W106 | CDR3 | H-bond |
| | Y107 | CDR3 | H-bond |
| E668 | R65 | CDR2 | H-bond/ionic |

**Table 5: The residues of the PBP2a-TP bound by the residues of the PBP2a-TP specific V_{H}H5.**

| **PBP2a-TP** | **TP-V_{H}H5** | | **Intermolecular bonds** |
|---|---|---|---|
| **Residue** | **Amino acid** | **Domain** | |
| **T384** | S30 | CDR1 | H-bond |
| **E385** | N73 | FR3 | H-bond |
| **D386** | N73 | FR3 | H-bond |
| **K387** | D54 | FR2 | H-bond |
| **E389** | D54 | FR2 | H-bond |
| | R71 | FR3 | Ionic |
| **Y499** | L100 | FR3 | Hydrophobic |
| **Q502** | P101 | FR3 | H-bond |
| **D516** | K58 | CDR2 | Ionic |
| **G522** | K58 | CDR2 | H-bond |
| **E523** | K58 | CDR2 | H-bond/ionic |
| **L525** | P101 | FR3 | Hydrophobic |
| **K604** | K58 | CDR2 | H-bond |
| **G611** | D54 | FR2 | H-bond |
| **K634** | D54 | FR2 | H-bond/ionic |

**Table 6: The residues of the PBP2a-TP bound by the residues of the PBP2a-TP specific V_{H}H8.**

| **PBP2a-TP** | **TP-V_{H}H8**^{a} | | **Intermolecular bond** |
|---|---|---|---|
| **Residue** | **Amino acid** | **Domain** | |
| N415 | N111 | CDR3 | H-bond |
| N416 | N111 | CDR3 | H-bond |
| K417 | N111 | CDR3 | H-bond |
| K477 | S107 | CDR3 | H-bond |
| | F47 | FR2 | Cationic- |
| K484 | S100 | FR3 | H-bond |
| | D101 | FR3 | H-bond |
| K485 | F99 | FR3 | H-bond |
| | D114 | CDR3 | H-bond/ionic |
| W558 | T28 | CDR1 | H-bond |
| K559 | F99 | FR3 | H-bond |
| K560 | G26 | CDR1 | H-bond |
| | D114 | CDR3 | H-bond |
| N561 | D114 | CDR3 | H-bond |

**Table 7: The residues of the PBP2a-TP bound by the residues of the PBP2a-TP specific V_{H}H11.**

| **PBP2a-TP** | **TP-V_{H}H11** | | **Intermolecular bonds** |
|---|---|---|---|
| **Residues** | **Amino acids** | **Domains** | |
| N338 | G109 | CDR3 | H-bond |
| N339 | Y100 | FR3 | H-bond |
| K341 | F108 | CDR3 | H-bond |
| K388 | Y102 | FR3 | H-bond/cationic- |
| K639 | Y32 | CDR1 | H-bond |
| | Y102 | FR3 | Cationic- |
| Y644 | Y32 | CDR1 | Hydrophobic/aromatic-aromatic |
| | Y102 | FR3 | Hydrophobic/H-bond/aromatic-aromatic |
| K651 | D29 | CDR1 | Ionic |
| | Y32 | CDR1 | Cationic- |
| | Y100 | FR3 | H-bond/cationic- |
| | Y110 | CDR3 | H-bond |
| E655 | Y110 | CDR3 | H-bond |
| N659 | Q113 | CDR3 | H-bond/ionic |

**Table 8: The residues of the PBP2a-TP bound by the residues of the PBP2a-TP specific V_{H}H15.**

| **PBP2a-TP** | **TP-V_{H}H15** | | **Intermolecular bonds** |
|---|---|---|---|
| **Residues** | **Amino acids** | **Domains** | |
| N377 | K109 | CDR3 | H-bond |
| T384 | R100 | FR3 | H-bond |
| | D112 | CDR3 | H-bond |
| | Y113 | CDR3 | H-bond |
| E385 | R100 | FR3 | H-bond |
| | Y113 | CDR3 | H-bond |
| D386 | R100 | FR3 | H-bond |
| K387 | Y27 | CDR1 | H-bond/cationic- |
| E389 | Y27 | CDR1 | H-bond |
| | Y32 | CDR1 | H-bond |
| L391 | C101 | FR3 | H-bond |
| N505 | D73 | FR3 | H-bond |
| | N74 | FR3 | H-bond |
| E523 | K76 | FR3 | H-bond/ionic |
| L525 | Y29 | CDR1 | Hydrophobic |
| L603 | Y27 | CDR1 | Hydrophobic |
| K604 | T28 | CDR1 | H-bond |
| K634 | Y27 | CDR1 | Cationic- |
| | Y32 | CDR1 | Cationic- |

**Table 9: The residues of the PBP2a-TP bound by the residues of the PBP2a-TP specific VH30.**

| **PBP2a-TP** | **TP-VH30** | | **Intermolecular bonds** |
|---|---|---|---|
| **Residues** | **Amino acids** | **Domains** | |
| **G440** | G31 | CDR1 | H-bond |
| | H53 | FR2 | H-bond |
| | D101 | FR3 | H-bond |
| **Y441** | D101 | FR3 | H-bond |
| | 1102 | FR3 | Hydrophobic |
| | V103 | FR3 | Hydrophobic |
| **T444** | Y32 | CDR1 | H-bond |
| **Y446** | Q1 | FR1 | H-bond |
| **N510** | V103 | FR3 | H-bond |
| | V105 | CDR3 | H-bond |
| **1512** | I102 | FR3 | Hydrophobic |
| | V103 | FR3 | Hydrophobic |
| **L513** | 1102 | FR3 | Hydrophobic |
| | W111 | CDR3 | Hydrophobic |
| **E602** | R97 | FR3 | H-bond/ionic |
| | R100 | FR3 | H-bond/ionic |
| **L603** | R100 | FR3 | H-bond |
| | D116 | CDR3 | H-bond |
| **K604** | W111 | CDR3 | Cationic- |
| **R612** | D116 | CDR3 | Ionic |

**Table 10: Amino acid residues of S. aureus USA300 (MRSA) PBP2a-TP Transpeptidase Domain) bound by various nanobodies.**

| **PBP2a-TP residues** | **Antibody clones (TP-bound residue(s))** | **PBP2a-TP residues** | **Antibody clones (TP-bound residue(s))** |
|---|---|---|---|
| **K334** | VH3 (D57) | **D516** | V_{H}H5 (K58) |
| **N338** | VH3 (D55), V_{H}H11 (G109) | **G522** | V_{H}H5 (K58) |
| **N339** | V_{H}H11 (Y100) | **E523** | V_{H}H5 (K58) |
| **K341** | VH3 (Y52), V_{H}H11 (F108) | **L525** | V_{H}H5 (P101) |
| **N377** | V_{H}H15 (K109) | **W558** | V_{H}H8 (T28) |
| **T384** | V_{H}H5 (S30), V_{H}H15 (R100, D112, Y113) | **K559** | V_{H}H8 (F99) |
| **E385** | V_{H}H5 (N73), V_{H}H15 (R100, Y113) | **K560** | V_{H}H8 (G26, D114) |
| **D386** | V_{H}H5 (N73), V_{H}H15 (R100) | **N561** | V_{H}H8 (D114) |
| **K387** | V_{H}H5 (D54), V_{H}H15 (Y27) | **K584** | VH3 (Y105) |
| **K388** | V_{H}H11 (Y102) | | |
| **E389** | V_{H}H5 (D54, R71), V_{H}H15 (Y27, Y32) | **E585** | VH3 (T104) |
| **E391** | V_{H}H15 (C101) | **D586** | VH3 (Y105) |
| **N415** | V_{H}H8 (N111) | **K589** | VH3 (T104, Y105) |
| **N416** | V_{H}H8 (N111) | **E602** | VH30 (R97, R100) |
| **K417** | V_{H}H8 (N111) | **L603** | VH30 (R100, D116), V_{H}H15 (Y27) |
| **G440** | VH30 (G31, H53, D101) | **K604** | VH30 (W111) |
| **Y441** | VH30 (D101, I102, V103) | **G611** | V_{H}H5 (D54) |
| **Y444** | VH30 (Y32) | **R612** | VH30 (D116) |
| **Y446** | VH30 (Q1) | **K634** | V_{H}H5 (D54), V_{H}H15 (Y27, Y32) |
| **K477** | V_{H}H8 (F47, S107) | **K639** | V_{H}H11 (Y32, Y102) |
| **K484** | V_{H}H8 (S100, D101) | **Y644** | V_{H}H11 (Y32, Y102) |
| **K485** | V_{H}H8 (F99, D114) | | |
| **Y499** | V_{H}H5 (L100) | **K651** | VH3 (G102), V_{H}H11 (D29, Y32, Y100, Y110) |
| **Q502** | V_{H}H5 (P101) | **L654** | VH3 (T104) |
| **N505** | V_{H}H15 (D73, N74) | **K655** | VH3 (W33, K59), V_{H}H11 (Y110) |
| **N510** | VH30 (V103, V105) | **K658** | VH3 (Y60, R65) |
| **1512** | VH30 (1102, V103) | **K659** | VH3 (W106, Y107), V_{H}H11 (Q113) |
| **L513** | VH30 (1102, W111) | **E668** | VH3 (R65) |

### Example 2: Antigen binding tests of HuscFvs/VHs/V_{H}Hs

For indirect ELISA, one µg aliquots of the purified rPBP2a-TP were immobilized in wells of an ELISA plate. Wells coated with 1 µg bovine serum albumin (BSA) (control antigen) and coating buffer only (blank) were included in the assay. After blocking the unoccupied spaces on the well surface with 5% skim milk in PBS, individual *E. coli* lysates containing standardized HuscFvs/VHs/V_{H}Hs and lysate of original *E. coli* HB2151 (negative antibody control) were added to appropriate wells and incubated at 37 °C for 1 h. Unbound materials were removed; rabbit anti-E Tag, goat anti-rabbit immunoglobulin- horseradish peroxidase (HRP) conjugate and ABTS substrate were added sequentially with washing between the steps. HuscFvs/VHs/V_{H}Hs in *E. coli* clones that revealed optical absorbance at 405 nm (OD405nm) to rPBP2a-TP at least two times higher than to BSA control were selected for further experiments (Figures 10 and 11).

For Western blot analysis, purified rPBP2a-TP was subjected to 12% SDS-PAGE and the separated components were blotted onto a nitrocellulose membrane (NC). The blotted NC was cut vertically into strips and blocked with 5% skim milk in Tris buffered saline (TBS) containing 0.05% Tween-20 (TBST). The strips were probed individually with *E. coli* lysates containing the HuscFvs/VHs/V_{H}Hs at 25 °C for 1 h. A lysate of original HB2151 *E. coli* was used as negative HuscFvs/VH/V_{H}H control. After washing, the antigen-antibody reactive band on each NC strip was detected by using rabbit anti-E Tag, goat anti-rabbit immunoglobulin-AP conjugate and BCIP/NBT™ substrate, respectively. The rPBP2a-TP blotted strip probed with the antibody diluent incubated with mouse anti- 6×His Tag, goat anti-mouse immunoglobulin-AP conjugate and BCIP/NBT substrate served as positive reaction control (Figure 12 and 13).

**Table 11: scFv antibodies of the invention**

| **SEQ ID NO:** | **Sequence-Domain:** | **Amino Acid Sequence:** |
|---|---|---|
| 1 | VH-FR1 | QVQLQESGPGLVKPSETLSLTCTVS |
| 2 | VH-CDR1 | GGSINTHSYY |
| 3 | VH-FR2 | WLWIRQPPGKGLEWVGS |
| 4 | VH-CDR2 | IFHGG-SA |
| 5 | VH-FR3 | FYSPSLKGRVTISVDTSRGQFSLNLMSVTAADTAVYY C |
| 6 | VH-CDR3 | VRQRGLAPAGTYYYYGLDA |
| 7 | VH-FR4 | WGQGTTVTVSS |
| 8 | Full Length VH | QVQLQESGPGLVKPSETLSLTCTVSGGSINTHSYYWL WIRQPPGKGLEWVGSIFHGGSAFYSPSLKGRVTISVD TSRGQFSLNLMSVTAADTAVYYCVRQRGLAPAGTYYY YGLDAWGQGTTVTVSS |
| 9 | VL-FR1 | VIWMTQSPDSLAVSLGERATINCKSS |
| 10 | VL-CDR1 | QSLLYSSKNKDY |
| 11 | VL-FR2 | LTWYQQKPGQPPKLLIY |
| 12 | VL-CDR2 | WAS |
| 13 | VL-FR3 | TRASGVPDRFSGSVSGTIFTLTISSLQAEDVAVYYC |
| 14 | VL-CDR3 | QQYYS-TPYT |
| 15 | VL-FR4 | FGQGTKVEIK |
| 16 | Full Length VL | VIWMTQSPDSLAVSLGERATINCKSSQSLLYSSKNKD YLTWYQQKPGQPPKLLIYWASTRASGVPDRFSGSVSG TIFTLTISSLQAEDVAVYYCQQYYSTPYTFGQGTKVEI K |
| 17 | VH-FR1 | QVQLVQSGAEVKKPGSSVKVSCKAS |
| 18 | VH-CDR1 | GGTFSSYA |
| 19 | VH-FR2 | ISWVRQAPGQGLEWMGR |
| 20 | VH-CDR2 | IIPILGIA |
| 21 | VH-FR3 | NYAQKFQGRVTITADKSTSTAYMELSSLRSEDTAVYY C |
| 22 | VH-CDR3 | ARRLLPTYSSSW-SDHYYYYGMDV |
| 23 | VH-FR4 | WGQGTTVTVSS |
| 24 | Full Length VH | QVQLVQSGAEVKKPGSSVKVSCKASGGTFSSYAISWV RQAPGQGLEWMGRIIPILGIANYAQKFQGRVTITADK STSTAYMELSSLRSEDTAVYYCARRLLPTYSSSWSDH YYYYGMDVWGQGTTVTVSS |
| 25 | VL-FR1 | AIQLTQSPSFLSASVGDRVTITCRAS |
| 26 | VL-CDR1 | QGVSNY |
| 27 | VL-FR2 | LVWYQQKPGKAPKLLIS |
| 28 | VL-CDR2 | AAS |
| 29 | VL-FR3 | TLQSGVPSRFSGSGSGTEFTLTISSLQPEDFATYFC |
| 30 | VL-CDR3 | QQLSIYPIT |
| 31 | VL-FR4 | FGPGTKLDIK |
| 32 | Full Length VL | AIQLTQSPSFLSASVGDRVTITCRASQGVSNYLVWYQ QKPGKAPKLLISAASTLQSGVPSRFSGSGSGTEFTLTIS SLQPEDFATYFCQQLSIYPITFGPGTKLDIK |
| 33 | VH-FR1 | QVQLQQWGAGLLKPSETLSLTCAVY |
| 34 | VH-CDR1 | GGSFSGYY |
| 35 | VH-FR2 | WSWIRQPPGKGLEWIGE |
| 36 | VH-CDR2 | INHSGST |
| 37 | VH-FR3 | NYNPSLKSRVTISVDTSKNQFSLKLSSVTAADTAVYYC |
| 38 | VH-CDR3 | AREDRDIVVVPAAIRWGNWFDP |
| 39 | VH-FR4 | WGQGTLVTVSS |
| 40 | Full Length VH | QVQLQQWGAGLLKPSETLSLTCAVYGGSFSGYYWSW IRQPPGKGLEWIGEINHSGSTNYNPSLKSRVTISVDTS KNQFSLKLSSVTAADTAVYYCAREDRDIVVVPAAIRW GNWFDPWGQGTLVTVSS |
| 41 | VL-FR1 | TIQLTQSPSTLSASIGDRVTITCRAS |
| 42 | VL-CDR1 | QSISDW |
| 43 | VL-FR2 | LAWYQQKPGKAPKLLIQ |
| 44 | VL-CDR2 | KAS |
| 45 | VL-FR3 | SLESGVPSRFSGRGSGTEFTLTISSLQPDDFATYYC |
| 46 | VL-CDR3 | QQYNSYSLT |
| 47 | VL-FR4 | FGGGTKLDIK |
| 48 | Full Length VL | TIQLTQSPSTLSASIGDRVTITCRASQSISDWLAWYQQ KPGKAPKLLIQKASSLESGVPSRFSGRGSGTEFTLTISS LQPDDFATYYCQQYNSYSLTFGGGTKLDIK |
| 49 | VH-FR1 | QVQLQESGPGLVKPSETLSLTCTVS |
| 50 | VH-CDR1 | GGSISSYY |
| 51 | VH-FR2 | WSWIRQPAGKGLEWIGR |
| 52 | VH-CDR2 | IYTSGST |
| 53 | VH-FR3 | NYNPFLKSRVTMSVDTSKNQFSLKLSSETAADTAVYY C |
| 54 | VH-CDR3 | ACTGYSSSWPYWYFDL |
| 55 | VH-FR4 | WGRGTLVTVSS |
| 56 | Full Length VH | QVQLQESGPGLVKPSETLSLTCTVSGGSISSYYWSWIR QPAGKGLEWIGRIYTSGSTNYNPFLKSRVTMSVDTSK NQFSLKLSSETAADTAVYYCACTGYSSSWPYWYFDL WGRGTLVTVSS |
| 57 | VL-FR1 | TIRMTQSPSSLSASVGDRVTITCRAS |
| 58 | VL-CDR1 | QSISSY |
| 59 | VL-FR2 | LNWYQQKPGKAPKLLIY |
| 60 | VL-CDR2 | GAS |
| 61 | VL-FR3 | SLQSGVPSRFSGGGSGTDFTLTISSLQPEDFATYYC |
| 62 | VL-CDR3 | QQSYS-TPIT |
| 63 | VL-FR4 | FGGGTKLEIK |
| 64 | Full Length VL | TIRMTQSPSSLSASVGDRVTITCRASQSISSYLNWYQQ KPGKAPKLLIYGASSLQSGVPSRFSGGGSGTDFTLTIS SLQPEDFATYYCQQSYSTPITFGGGTKLEIK |
| 65 | VH-FR1 | EVQLVESGAEVKKPGASVKVSCKAS |
| 66 | VH-CDR1 | GYTFTSYD |
| 67 | VH-FR2 | INWVRQATGQGLEWMGW |
| 68 | VH-CDR2 | MNPNSGNT |
| 69 | VH-FR3 | GYAQKFQGRVTMTRNTSISTAYMELSSLRSEDTAVYY C |
| 70 | VH-CDR3 | AREPYGSGWYFTPQHYYGMDV |
| 71 | VH-FR4 | WGKGTTVTVSS |
| 72 | Full Length VH | EVQLVESGAEVKKPGASVKVSCKASGYTFTSYDINWV RQATGQGLEWMGWMNPNSGNTGYAQKFQGRVTM TRNTSISTAYMELSSLRSEDTAVYYCAREPYGSGWYF TPQHYYGMDVWGKGTTVTVSS |
| 73 | VL-FR1 | DIQMTQSPSSLSASVGDRVTITCRAS |
| 74 | VL-CDR1 | QGITTE |
| 75 | VL-FR2 | LGWFQQRPGKAPKRLIY |
| 76 | VL-CDR2 | AAS |
| 77 | VL-FR3 | TLESGVPSRFSGSGSGTEFTLTISSLQPEDFATYYC |
| 78 | VL-CDR3 | LQHNSYPRT |
| 79 | VL-FR4 | FGQGTKVEIK |
| 80 | Full Length VL | DIQMTQSPSSLSASVGDRVTITCRASQGITTELGWFQ QRPGKAPKRLIYAASTLESGVPSRFSGSGSGTEFTLTIS SLQPEDFATYYCLQHNSYPRTFGQGTKVEIK |
| 81 | VH-FR1 | QLQLQESGPGLVKPSQTLSLTCTVS |
| 82 | VH-CDR1 | GGSISSGSYY |
| 83 | VH-FR2 | WSWIRQPAGKGLEWIGR |
| 84 | VH-CDR2 | IYTSG-ST |
| 85 | VH-FR3 | NYNPSLKSRVTISVDTSKNQFSLKLSSVTAADTAVYYC |
| 86 | VH-CDR3 | ARDDRSSSWAYGYYYYGMDV |
| 87 | VH-FR4 | WGQGTTVTVSS |
| 88 | Full Length VH | QLQLQESGPGLVKPSQTLSLTCTVSGGSISSGSYYWS WIRQPAGKGLEWIGRIYTSGSTNYNPSLKSRVTISVD TSKNQFSLKLSSVTAADTAVYYCARDDRSSSWAYGYY YYGMDVWGQGTTVTVSS |
| 89 | VL-FR1 | EIVLTQSPGTLSLSPGETATLSCRAS |
| 90 | VL-CDR1 | QSVGTY |
| 91 | VL-FR2 | LGWYQQKPGQVPRLLIY |
| 92 | VL-CDR2 | DAS |
| 93 | VL-FR3 | DRASGIPARFSGSGSGTGFTLTISSLESEDFAVYYC |
| 94 | VL-CDR3 | QQRSAWPLT |
| 95 | VL-FR4 | FGGGTKLEIK |
| 96 | Full Length VL | EIVLTQSPGTLSLSPGETATLSCRASQSVGTYLGWYQ QKPGQVPRLLIYDASDRASGIPARFSGSGSGTGFTLTI SSLESEDFAVYYCQQRSAWPLTFGGGTKLEIK |
| 97 | VH-FR1 | EVQLLESGGGLVQPGGSLRLSCAAS |
| 98 | VH-CDR1 | GLTLS--HYE |
| 99 | VH-FR2 | MNWVRQAPGKGLEWVSY |
| 100 | VH-CDR2 | ISSSGGTI |
| 101 | VH-FR3 | RYGDSVKGRFTISRDNAKNSLHLQMNSLRDEDTAVY YC |
| 102 | VH-CDR3 | ASCKGSGNYAHAFDI |
| 103 | VH-FR4 | WGQGTMVTVSS |
| 104 | Full Length VH | EVQLLESGGGLVQPGGSLRLSCAASGLTLSHYEMNW VRQAPGKGLEWVSYISSSGGTIRYGDSVKGRFTISRD NAKNSLHLQMNSLRDEDTAVYYCASCKGSGNYAHAF DIWGQGTMVTVSS |
| 105 | VL-FR1 | EIVMTQSPGTLSLSPGESATLSCRAS |
| 106 | VL-CDR1 | QSIKNSE |
| 107 | VL-FR2 | IAWYQHKLGQAPRLLIY |
| 108 | VL-CDR2 | GAA |
| 109 | VL-FR3 | SRAIGVPDRFSGSGSGTDFTLTISRLEPEDFAVYYC |
| 110 | VL-CDR3 | QQYGSSRT |
| 111 | VL-FR4 | FGQGTKLDIK |
| 112 | Full Length VL | EIVMTQSPGTLSLSPGESATLSCRASQSIKNSEIAWYQ HKLGQAPRLLIYGAASRAIGVPDRFSGSGSGTDFTLTI SRLEPEDFAVYYCQQYGSSRTFGQGTKLDIK |
| 113 | VH-FR1 | QVQLVESGGGLVQPGGSLRLSCAAS |
| 114 | VH-CDR1 | GFGVSSSF |
| 115 | VH-FR2 | MSWVRQVPGKGLEWLSV |
| 116 | VH-CDR2 | IYGYG-ST |
| 117 | VH-FR3 | DNADSVKGRFTISRDNSKNTLFLQMNNLRAEDTAVY YC |
| 118 | VH-CDR3 | ARGHYYDSTYDAFDI |
| 119 | VH-FR4 | WGQGTMVTVSS |
| 120 | Full Length VH | QVQLVESGGGLVQPGGSLRLSCAASGFGVSSSFMSW VRQVPGKGLEWLSVIYGYGSTDNADSVKGRFTISRD NSKNTLFLQMNNLRAEDTAVYYCARGHYYDSTYDAF DIWGQGTMVTVSS |
| 121 | VL-FR1 | TIQLTQSPSSLSASVGDRVTITCRAS |
| 122 | VL-CDR1 | QGISNY |
| 123 | VL-FR2 | LAWFQQKPGKAPKSLIY |
| 124 | VL-CDR2 | AAS |
| 125 | VL-FR3 | SLQSGVPSRFSGSGSGTDFTLTISSLQPEDFATYYC |
| 126 | VL-CDR3 | QQANS-LPLT |
| 127 | VL-FR4 | FGGGTKVDIK |
| 128 | Full Length VL | TIQLTQSPSSLSASVGDRVTITCRASQGISNYLAWFQQ KPGKAPKSLIYAASSLQSGVPSRFSGSGSGTDFTLTISS LQPEDFATYYCQQANSLPLTFGGGTKVDIK |
| 129 | VH-FR1 | QLQLQESGPGLVKPSQTLSLTCAVS |
| 130 | VH-CDR1 | GGSISSGDYY |
| 131 | VH-FR2 | WNWIRQPAGERLEWIGR |
| 132 | VH-CDR2 | TYSSGIT |
| 133 | VH-FR3 | NYNPSLLSRVTISVDTSKNQFSLKLNSVTAADTAVYYC |
| 134 | VH-CDR3 | VRKRERGTLDI |
| 135 | VH-FR4 | WGQGTMVTVPS |
| 136 | Full Length VH | QLQLQESGPGLVKPSQTLSLTCAVSGGSISSGDYYWN WIRQPAGERLEWIGRTYSSGITNYNPSLLSRVTISVDT SKNQFSLKLNSVTAADTAVYYCVRKRERGTLDIWGQ GTMVTVPS |
| 137 | VL-FR1 | DIQLTQSPSSLSASVGDRVSITCRAS |
| 138 | VL-CDR1 | QGISSY |
| 139 | VL-FR2 | LAWYQQKPGTAPKLLIY |
| 140 | VL-CDR2 | SIS |
| 141 | VL-FR3 | TLQSGVPSRFSGSGSGTEFTPTINNLQPEDFATYYC |
| 142 | VL-CDR3 | QQLNSDPPYT |
| 143 | VL-FR4 | FGQGTKVEIK |
| 144 | Full Length VL | DIQLTQSPSSLSASVGDRVSITCRASQGISSYLAWYQQ KPGTAPKLLIYSISTLQSGVPSRFSGSGSGTEFTPTINN LQPEDFATYYCQQLNSDPPYTFGQGTKVEIK |
| 145 | VH-FR1 | EVQLLESGGGLVKPGRSLRLSCAAS |
| 146 | VH-CDR1 | GFIFSNYD |
| 147 | VH-FR2 | MHWVRQAPGKGLEWVSS |
| 148 | VH-CDR2 | IISSGDHT |
| 149 | VH-FR3 | YYADSVKGRFTISRDNAKDSLHLQVNSLRVEDTAVY HC |
| 150 | VH-CDR3 | ARAEGFCSGGSCYSSGWSFDL |
| 151 | VH-FR4 | WGQGTMVTVSS |
| 152 | Full Length VH | EVQLLESGGGLVKPGRSLRLSCAASGFIFSNYDMHW VRQAPGKGLEWVSSIISSGDHTYYADSVKGRFTISRD NAKDSLHLQVNSLRVEDTAVYHCARAEGFCSGGSCY SSGWSFDLWGQGTMVTVSS |
| 153 | VL-FR1 | DIVMTQSPDSLAVSLGERATINCKSS |
| 154 | VL-CDR1 | QSVLYSSNNKNY |
| 155 | VL-FR2 | LAWYQQKPGQPPKLLIY |
| 156 | VL-CDR2 | WAS |
| 157 | VL-FR3 | TRESGVPDRFSGSGSGTDFTLTISSLQAEDVAVYFC |
| 158 | VL-CDR3 | QQYNRTPWT |
| 159 | VL-FR4 | FGQGTKVDIK |
| 160 | Full Length VL | DIVMTQSPDSLAVSLGERATINCKSSQSVLYSSNNKN YLAWYQQKPGQPPKLLIYWASTRESGVPDRFSGSGS GTDFTLTISSLQAEDVAVYFCQQYNRTPWTFGQGTK VDIK |
| 161 | VH-FR1 | EVQLVESGGDLVQPGRSLRLSCAAS |
| 162 | VH-CDR1 | GFTFEDYA |
| 163 | VH-FR2 | MHWVRQAPGKGLEWVSS |
| 164 | VH-CDR2 | INWSSGSI |
| 165 | VH-FR3 | VYADSVKGRFTISRDNAKNSLYLQMNSLRPDDTALYY C |
| 166 | VH-CDR3 | AKDSGPYGSGHYGMDV |
| 167 | VH-FR4 | WGQGTTVTVSS |
| 168 | Full Length VH | EVQLVESGGDLVQPGRSLRLSCAASGFTFEDYAMHW VRQAPGKGLEWVSSINWSSGSIVYADSVKGRFTI SRDNAKNSLYLQMNSLRPDDTALYYCAKDSGPYGSG HYGMDVWGQGTTVTVSS |
| 169 | VL-FR1 | EIVLTQSPDFQSVTPREEVTITCRAS |
| 170 | VL-CDR1 | QSILYSSNNKNY |
| 171 | VL-FR2 | LAWYQQKPGQPPKLLIY |
| 172 | VL-CDR2 | WAS |
| 173 | VL-FR3 | TRESGVPDRFSGSGSGTDFTLTISSLQAEDVAVYFC |
| 174 | VL-CDR3 | QQYNRTPWT |
| 175 | VL-FR4 | FGQGTKVDIK |
| 176 | Full Length VL | EIVLTQSPDFQSVTPREEVTITCRASQSIGSDLHWYQ QRPDQPPRLLVKYASQSLSGVPSRFRGSGSGTDFTLTI DSPQPDDIATYYCHQTSASPWTFGQGTKLEIK |
| 177 | VH-FR1 | EVQLVESGGDLVQPGRSLRLSCAAS |
| 178 | VH-CDR1 | GFTFEDYA |
| 179 | VH-FR2 | MHWVRQAPGKGLEWVSS |
| 180 | VH-CDR2 | INWSSGSI |
| 181 | VH-FR3 | VYADSVKGRFTISRDNAKNSLYLQMNSLRPDDTALYY C |
| 182 | VH-CDR3 | AKDSGPYGSGHYGMDV |
| 183 | VH-FR4 | WGQGTTVTVSS |
| 184 | Full Length VH | EVQLVESGGDLVQPGRSLRLSCAASGFTFEDYAMHW VRQAPGKGLEWVSSINWSSGSIVYADSVKGRFTISRD NAKNSLYLQMNSLRPDDTALYYCAKDSGPYGSGHYG MDVWGQGTTVTVSS |
| 185 | VL-FR1 | EIVLTQSPDFQSVTPREEVTITCRAS |
| 186 | VL-CDR1 | QSIGSD |
| 187 | VL-FR2 | LHWYQQRPDQPPRLLVK |
| 188 | VL-CDR2 | YAS |
| 189 | VL-FR3 | QSLSGVPSRFRGSGSGTDFTLTIDSPQPDDIATYYC |
| 190 | VL-CDR3 | HQTSA-SPWT |
| 191 | VL-FR4 | FGQGTKLEIK |
| 192 | Full Length VL | EIVLTQSPDFQSVTPREEVTITCRASQSIGSDLHWYQ QRPDQPPRLLVKYASQSLSGVPSRFRGSGSGTDFTLTI DSPQPDDIATYYCHQTSASPWTFGQGTKLEIK |
| 193 | VH-FR1 | QVQLVQSGAEVKKPGESLRISCKAS |
| 194 | VH-CDR1 | GFSFSNYW |
| 195 | VH-FR2 | ISWVRQLPGKGLEWMGR |
| 196 | VH-CDR2 | IDPSDSYT |
| 197 | VH-FR3 | NYSPSFEGRVTMSTDNSITTAYLQWSSLKASDTAMYY C |
| 198 | VH-CDR3 | ARREGGYTFGFHYYGMDV |
| 199 | VH-FR4 | WGQGTTVTVSS |
| 200 | Full Length VH | QVQLVQSGAEVKKPGESLRISCKASGFSFSNYWISWV RQLPGKGLEWMGRIDPSDSYTNYSPSFEGRVTMSTD NSITTAYLQWSSLKASDTAMYYCARREGGYTFGFHYY GMDVWGQGTTVTVSS |
| 201 | VL-FR1 | EIVMTQSPATLSVSPGERATLSCRAS |
| 202 | VL-CDR1 | QSVSSN |
| 203 | VL-FR2 | LAWYQQKPGQAPRLLIY |
| 204 | VL-CDR2 | GAS |
| 205 | VL-FR3 | TRATGIPARFSGSGSGTEFTLTISSLQSEDFAVYYC |
| 206 | VL-CDR3 | QQYNNWPLT |
| 207 | VL-FR4 | FGPGTKVDIK |
| 208 | Full Length VL | EIVMTQSPATLSVSPGERATLSCRASQSVSSNLAWYQ QKPGQAPRLLIYGASTRATGIPARFSGSGSGTEFTLTI SSLQSEDFAVYYCQQYNNWPLTFGPGTKVDIK |
| 209 | VH-FR1 | EVQLVESGAEVKKPGESLKISCKGS |
| 210 | VH-CDR1 | GYSFTSYW |
| 211 | VH-FR2 | IGWVHQMPGKGLEWMGI |
| 212 | VH-CDR2 | IYPGDSDT |
| 213 | VH-FR3 | RYSPSFQGQVTISADKSISTAYLQWSSLKASDTAMYYC |
| 214 | VH-CDR3 | ARRDQNCGGDCYADAFDI |
| 215 | VH-FR4 | WGQGTMVTVSS |
| 216 | Full Length VH | EVQLVESGAEVKKPGESLKISCKGSGYSFTSYWIGWV HQMPGKGLEWMGIIYPGDSDTRYSPSFQGQVTISAD KSISTAYLQWSSLKASDTAMYYCARRDQNCGGDCYA DAFDIWGQGTMVTVSS |
| 217 | VL-FR1 | DIQMTQSPFSLSASVGDTVTITCRAS |
| 218 | VL-CDR1 | QGVSSW |
| 219 | VL-FR2 | LAWYQQKPDKAPKSLIY |
| 220 | VL-CDR2 | AAS |
| 221 | VL-FR3 | SLQSGVPSRFSGSGSGTDFTLTITSLQPEDFATYYC |
| 222 | VL-CDR3 | LQDFDYPYT |
| 223 | VL-FR4 | FGQGTKLDIK |
| 224 | Full Length VL | DIQMTQSPFSLSASVGDTVTITCRASQGVSSWLAWYQ QKPDKAPKSLIYAASSLQSGVPSRFSGSGSGTDFTLTI TSLQPEDFATYYCLQDFDYPYTFGQGTKLDIK |
| 225 | VH-FR1 | EVQLVESGGDLVQPGRSLRLSCAAS |
| 226 | VH-CDR1 | GFTFEDYA |
| 227 | VH-FR2 | MHWVRQAPGKGLEWVSS |
| 228 | VH-CDR2 | INWSSGSI |
| 229 | VH-FR3 | VYADSVKGRFTISRDNAKNSLYLQMNSLRPDDTALYY C |
| 230 | VH-CDR3 | AKDSGPYGSGHYGMDV |
| 231 | VH-FR4 | WGQGTTVTVSS |
| 232 | Full Length VH | EVQLVESGGDLVQPGRSLRLSCAASGFTFEDYAMHW VRQAPGKGLEWVSSINWSSGSIVYADSVKGRFTISRD NAKNSLYLQMNSLRPDDTALYYCAKDSGPYGSGHYG MDVWGQGTTVTVSS |
| 233 | VL-FR1 | EIVLTQSPDFQSVTPREEVTITCRAS |
| 234 | VL-CDR1 | QSIGSD |
| 235 | VL-FR2 | LHWYQQRPDQPPRLLVK |
| 236 | VL-CDR2 | YAS |
| 237 | VL-FR3 | QSLSGVPSRFRGSGSGTDFTLTIDSPQPDDIATYYC |
| 238 | VL-CDR3 | HQTSASPWT |
| 239 | VL-FR4 | FGQGTKLEIK |
| 240 | Full Length VL | EIVLTQSPDFQSVTPREEVTITCRASQSIGSDLHWYQ QRPDQPPRLLVKYASQSLSGVPSRFRGSGSGTDFTLTI DSPQPDDIATYYCHQTSASPWTFGQGTKLEIK |
| 241 | VH-FR1 | QVQLVQSGAEVKKPGASVKVSCKVS |
| 242 | VH-CDR1 | GYTLTELS |
| 243 | VH-FR2 | MHWVRQAPGKGLEWMGG |
| 244 | VH-CDR2 | FDPEDGET |
| 245 | VH-FR3 | IYAQKFQGRVTMTEDTSTDTAYMELSSLRSEDTAVYY C |
| 246 | VH-CDR3 | ATGRYCSGGSCYPDAFDI |
| 247 | VH-FR4 | WGQGTMVTVSS |
| 248 | Full Length VH | QVQLVQSGAEVKKPGASVKVSCKVSGYTLTELSMHW VRQAPGKGLEWMGGFDPEDGETIYAQKFQGRVTMT EDTSTDTAYMELSSLRSEDTAVYYCATGRYCSGGSCY PDAFDIWGQGTMVTVSS |
| 249 | VL-FR1 | EIVMTQSPHSLAVSLGERATIYCKSS |
| 250 | VL-CDR1 | QTVLYSSNNRNY |
| 251 | VL-FR2 | LAWYQQKPRQPPKLLLS |
| 252 | VL-CDR2 | WAS |
| 253 | VL-FR3 | TRESGVPERFSGSGSGTEFTLTISALQAEDVAVYYC |
| 254 | VL-CDR3 | QQYYS-SPQT |
| 255 | VL-FR4 | FGQGTKLEIK |
| 256 | Full Length VL | EIVMTQSPHSLAVSLGERATIYCKSSQTVLYSSNNRNY LAWYQQKPRQPPKLLLSWASTRESGVPERFSGSGSG TEFTLTISALQAEDVAVYYCQQYYSSPQTFGQGTKLEI K |

**Table 12: Nanobodies of the invention**

| **SEQ ID NO:** | **Sequence-Domain** | **Amino Acid Sequence** |
|---|---|---|
| 257 | FR1 | EVQLVESGGGLVQPGGSLRLSCAAS |
| 258 | CDR1 | GFTFSNYA |
| 259 | FR2 | MTWVRQAPGKGLEWVSG |
| 260 | CDR2 | ISGSGSGA |
| 261 | FR3 | YYADSVKGRFTISRDNSQNTLYLQMNSLRAEDTAVYY |
| 262 | CDR3 | CAKDRSGSSPPRSFNY |
| 263 | FR4 | WGQGTTVTVSS |
| 264 | Full Length | EVQLVESGGGLVQPGGSLRLSCAASGFTFSNYAMTW VRQAPGKGLEWVSGISGSGSGAYYADSVKGRFTISRD NSQNTLYLQMNSLRAEDTAVYYCAKDRSGSSPPRSF NYWGQGTTVTVSS |
| 265 | FR1 | EVQLVESGAEVKKPGESLRISCKAS |
| 266 | CDR1 | GYTFSSY |
| 267 | FR2 | WIAWVRQMPGKGLEWMGI |
| 268 | CDR2 | IYPGDFDT |
| 269 | FR3 | KYSPSFRGQVTISADRSISTAYLQWSSLKASDTAIYY |
| 270 | CDR3 | CARSPSGTTYWYHFDD |
| 271 | FR4 | WGRGTTVTVSS |
| 272 | Full Length | EVQLVESGAEVKKPGESLRISCKASGYTFSSYWIAWV RQMPGKGLEWMGIIYPGDFDTKYSPSFRGQVTISAD RSISTAYLQWSSLKASDTAIYYCARSPSGTTYWYHFD DWGRGTTVTVSS |
| 273 | FR1 | QVQLVESGGGSVQAGRSLRLSCVIS |
| 274 | CDR1 | GGTLSTYA |
| 275 | FR2 | MGWFRQAPGKECELVS |
| 276 | CDR2 | ILNRDG-ST |
| 277 | FR3 | KYADSVKGRFTISRDNAKNMVYLQMNNLNPEDTAM YY |
| 278 | CDR3 | CAPRTLPPPTCPGRNTY |
| 279 | FR4 | WGHGTLVTVSS |
| 280 | Full Length | QVQLVESGGGSVQAGRSLRLSCVISGGTLSTYAMGW FRQAPGKECELVSILNRDGSTKYADSVKGRFTISRDN AKNMVYLQMNNLNPEDTAMYYCAPRTLPPPTCPGR NTYWGHGTLVTVSS |
| 281 | FR1 | EVQLVESAGGLVQPGGSLRLSCAAS |
| 282 | CDR1 | GFTFSTYY |
| 283 | FR2 | MSWVRQAPGKGLEWVSG |
| 284 | CDR2 | IYSDGVT |
| 285 | FR3 | VYGDSVKGRFTISRDNAKNTLYLQMNSLKPEDTAVY Y |
| 286 | CDR3 | CAGGPIGGSWDGADFSY |
| 287 | FR4 | WAQGTMVTVSS |
| 288 | Full Length | EVQLVESAGGLVQPGGSLRLSCAASGFTFSTYYMSWV RQAPGKGLEWVSGIYSDGVTVYGDSVKGRFTISRDN AKNTLYLQMNSLKPEDTAVYYCAGGPIGGSWDGADF SYWAQGTMVTVSS |
| 289 | FR1 | QVQLVESGGGSVQAGGSLRLSCAAS |
| 290 | CDR1 | GYTYCSYD |
| 291 | FR2 | MSWYRQAPGKEREFVSA |
| 292 | CDR2 | IDSDGST |
| 293 | FR3 | SYADSVKGRFTISQGNAKNTVYLQMNSLKPEDTAMY Y |
| 294 | CDR3 | CKTGFSD-PLRPGSNPCNALDA |
| 295 | FR4 | WGQGTLVTVSS |
| 296 | Full Length | QVQLVESGGGSVQAGGSLRLSCAASGYTYCSYDMSW YRQAPGKEREFVSAIDSDGSTSYADSVKGRFTISQGN AKNTVYLQMNSLKPEDTAMYYCKTGFSDPLRPGSNP CNALDAWGQGTLVTVSS |
| 297 | FR1 | EVQLVESGGGSVQAGGSLRLSCVVS |
| 298 | CDR1 | GSSDCTYNS |
| 299 | FR2 | MSWYRQAPGKEREFVSG |
| 300 | CDR2 | IRDDGRT |
| 301 | FR3 | SYRDSVKGRFTISQDGAKNTMYLQMDSLKPEDTAVY Y |
| 302 | CDR3 | CTRAYEYGLPSPFGY |
| 303 | FR4 | LGQGTMVTVSS |
| 304 | Full Length | EVQLVESGGGSVQAGGSLRLSCVVSGSSDCTYNSMS WYRQAPGKEREFVSGIRDDGRTSYRDSVKGRFTISQ DGAKNTMYLQMDSLKPEDTAVYYCTRAYEYGLPSPF GYLGQGTMVTVSS |
| 305 | FR1 | EVQLVESGAEVKKPGESLRISCKAS |
| 306 | CDR1 | GYTFSSY |
| 307 | FR2 | WIAWVRQMPGKGLEWMGI |
| 308 | CDR2 | IYPGDFDT |
| 309 | FR3 | KYSPSFRGQVTISADRSISTAYLQWSSLKASDTAIYY |
| 310 | CDR3 | CARSPSGTTYWYHFDD |
| 311 | FR4 | WGRGTTVTVSS |
| 312 | Full Length | EVQLVESGAEVKKPGESLRISCKASGYTFSSYWIAWV RQMPGKGLEWMGIIYPGDFDTKYSPSFRGQVTISAD RSISTAYLQWSSLKASDTAIYYCARSPSGTTYWYHFD DWGRGTTVTVSS |
| 313 | FR1 | QVQLVESGGGLVQPGGSLTLSCAAS |
| 314 | CDR1 | GFTFSNYV |
| 315 | FR2 | MSWVRQAPGKGLEWVST |
| 316 | CDR2 | IESGGGTT |
| 317 | FR3 | YYADSVKGRFTITRDNAKNTLYLQLNSLKSEDTAMYY |
| 318 | CDR3 | CGNFIAYYYYIDY |
| 319 | FR4 | WGRGTLVTVSS |
| 320 | Full Length | QVQLVESGGGLVQPGGSLTLSCAASGFTFSNYVMSW VRQAPGKGLEWVSTIESGGGTTYYADSVKGRFTITRD NAKNTLYLQLNSLKSEDTAMYYCGNFIAYYYYIDYWG RGTLVTVSS |
| 321 | FR1 | QVQLVESGGGSVQAGGSLRLSCAAS |
| 322 | CDR1 | GYTYSSYC |
| 323 | FR2 | MGWFRQAPGKEREGVAA |
| 324 | CDR2 | INSGGGST |
| 325 | FR3 | YYADSVKGRFTISQDNAKNTVYLQMNSLKPEDTAIYY |
| 326 | CDR3 | CAADRCAPGGSWYKVFDY |
| 327 | FR4 | WGQGTLVTVSS |
| 328 | Full Length | QVQLVESGGGSVQAGGSLRLSCAASGYTYSSYCMGW FRQAPGKEREGVAAINSGGGSTYYADSVKGRFTISQD NAKNTVYLQMNSLKPEDTAIYYCAADRCAPGGSWYK VFDYWGQGTLVTVSS |
| 329 | FR1 | EVQLVESGGGLVKPGGSLRLSCAAS |
| 330 | CDR1 | GFTFSSNY |
| 331 | FR2 | MSWVRQAPGKGLEWVSG |
| 332 | CDR2 | INRDGSNT |
| 333 | FR3 | YYADSVKGRFTISRDNAKNTLYLQMNSLKSEDTALYY |
| 334 | CDR3 | CATAYGGSWYGVPADFGY |
| 335 | FR4 | WDQGTLVTVSS |
| 336 | Full Length | EVQLVESGGGLVKPGGSLRLSCAASGFTFSSNYMSW VRQAPGKGLEWVSGINRDGSNTYYADSVKGRFTISR DNAKNTLYLQMNSLKSEDTALYYCATAYGGSWYGVP ADFGYWDQGTLVTVSS |
| 337 | FR1 | EVQLVESGGGLVQPGGSLRLSCVAS |
| 338 | CDR1 | GVAFSINY |
| 339 | FR2 | MSWVRQAPGKELDWVSD |
| 340 | CDR2 | IRMDGTST |
| 341 | FR3 | YYADSVKGRFTISRDNAKNTLYLQMNSLRSDDTALYY |
| 342 | CDR3 | CLGFGY |
| 343 | FR4 | WGQGTTVTVSS |
| 344 | Full Length | EVQLVESGGGLVQPGGSLRLSCVASGVAFSINYMSW VRQAPGKELDWVSDIRMDGTSTYYADSVKGRFTISR DNAKNTLYLQMNSLRSDDTALYYCLGFGYWGQGTT VTVSS |
| 345 | FR1 | QVQLVESGGGLVQPGGSLRLSCAAS |
| 346 | CDR1 | GFTFSNYW |
| 347 | FR2 | MYWVRQAPRKGLEWITT |
| 348 | CDR2 | INSAGGTT |
| 349 | FR3 | YYADSVKGRFTISRDNAKNTVYLQMNSLKPEDTAVY Y |
| 350 | CDR3 | CTRDLFESVVTVTFNY |
| 351 | FR4 | WGQGTLVTVSS |
| 352 | Full Length | QVQLVESGGGLVQPGGSLRLSCAASGFTFSNYWMYW VRQAPRKGLEWITTINSAGGTTYYADSVKGRFTISRD NAKNTVYLQMNSLKPEDTAVYYCTRDLFESVVTVTF NYWGQGTLVTVSS |
| 353 | FR1 | EVQLVESGGGLVQPGGSLRLSCAAS |
| 354 | CDR1 | GFSFNGHY |
| 355 | FR2 | MS-VRQAPGKGLEWVSN |
| 356 | CDR2 | INPDGGGK |
| 357 | FR3 | YYSNSVRDRFTISRDNAKNMLYLEMNSLKSEDTALY Y |
| 358 | CDR3 | CAKEESYSIED |
| 359 | FR4 | WGKGTMVTVSS |
| 360 | Full Length | EVQLVESGGGLVQPGGSLRLSCAASGFSFNGHYMSV RQAPGKGLEWVSNINPDGGGKYYSNSVRDRFTISRD NAKNMLYLEMNSLKSEDTALYYCAKEESYSIEDWGK GTMVTVSS |
| 361 | FR1 | QVQLVESGGDLVQPGGSLRLSCAAS |
| 362 | CDR1 | GFTFSNRW |
| 363 | FR2 | MYWVRQAPGKGLEWVSV |
| 364 | CDR2 | INGGGGTT |
| 365 | FR3 | YCADSVKGRFTISKDNAKNTLYLQMNSLKPEDTAVYY |
| 366 | CDR3 | CARDAGYWSADY |
| 367 | FR4 | WGQGTLVTVSS |
| 368 | Full Length | QVQLVESGGDLVQPGGSLRLSCAASGFTFSNRWMY WVRQAPGKGLEWVSVINGGGGTTYCADSVKGRFTIS KDNAKNTLYLQMNSLKPEDTAVYYCARDAGYWSAD YWGQGTLVTVSS |
| 369 | FR1 | EVQLVESGPGLVKPSQTLTLTCTVS |
| 370 | CDR1 | GGSITTSYYA |
| 371 | FR2 | WSWIRQPPGKGLEWMGA |
| 372 | CDR2 | IRYSGST |
| 373 | FR3 | YYSPSLKSRTSISRDTSKNQFSLQLSSVTPEDTAVYY |
| 374 | CDR3 | CASDVQYSMDD |
| 375 | FR4 | WGKGTMVTVSS |
| 376 | Full Length | EVQLVESGPGLVKPSQTLTLTCTVSGGSITTSYYAWS WIRQPPGKGLEWMGAIRYSGSTYYSPSLKSRTSISRD TSKNQFSLQLSSVTPEDTAVYYCASDVQYSMDDWGK GTMVTVSS |
| 377 | FR1 | QMQLVQWGAGLLKPSETLSLTCAVY |
| 378 | CDR1 | GGSFSGYY |
| 379 | FR2 | WSWIRQPPGKGLEWIGE |
| 380 | CDR2 | INHSGST |
| 381 | FR3 | NYNPSLKSRVTISVDTSKNQFSLKLSSVTAADTAVYY |
| 382 | CDR3 | CAREDRDIVVVPAAIRWGNWFDP |
| 383 | FR4 | WGQGTLVTVSS |
| 384 | Full Length | QMQLVQWGAGLLKPSETLSLTCAVYGGSFSGYYWS WIRQPPGKGLEWIGEINHSGSTNYNPSLKSRVTISVD TSKNQFSLKLSSVTAADTAVYYCAREDRDIVVVPAAIR WGNWFDPWGQGTLVTVSS |
| 385 | FR1 | EVQLVESGAEVKKPGESLRISCKAS |
| 386 | CDR1 | GYTFSSY |
| 387 | FR2 | WIAWVRQMPGKGLEWMGI |
| 388 | CDR2 | IYPGDFDT |
| 389 | FR3 | KYSPSFRGQVTISADRSISTAYLQWSSLKASDTAIYY |
| 390 | CDR3 | CARSPSGTTYWYHFGD |
| 391 | FR4 | WGRGTTVTVSS |
| 392 | Full Length | EVQLVESGAEVKKPGESLRISCKASGYTFSSYWIAWV RQMPGKGLEWMGIIYPGDFDTKYSPSFRGQVTISAD RSISTAYLQWSSLKASDTAIYYCARSPSGTTYWYHFG DWGRGTTVTVSS |

**Table 13: Correspondent IDs of Antibodies and Nanobodies of the Invention**

| **Internal ID:** | **Patent ID No:** | **Shown in SEQ ID Nos** |
|---|---|---|
| PBP2a-TP_HuscFv 1 | AB001 | 1-16 |
| PBP2a-TP_HuscFv 2 | AB002 | 17-32 |
| PBP2a-TP_HuscFv 5 | AB003 | 33-48 |
| PBP2a-TP_HuscFv 7 | AB004 | 49-64 |
| PBP2a-TP_HuscFv 8 | AB005 | 65-80 |
| PBP2a-TP_HuscFv 9 | AB006 | 81-96 |
| PBP2a-TP_HuscFv 10 | AB007 | 97-112 |
| PBP2a-TP_HuscFv 13 | AB008 | 113-128 |
| PBP2a-TP_HuscFv 16 | AB009 | 129-144 |
| PBP2a-TP_HuscFv 17 | AB010 | 145-160 |
| PBP2a-TP_HuscFv 21 | AB011 | 161-176 |
| PBP2a-TP_HuscFv 23 | AB012 | 177-192 |
| PBP2a-TP_HuscFv 26 | AB013 | 193-208 |
| PBP2a-TP_HuscFv 29 | AB014 | 209-224 |
| PBP2a-TP_HuscFv 30 | AB015 | 225-240 |
| PBP2a-TP_HuscFv 31 | AB016 | 241-256 |
| PBP2a-TP_VH2 | NB001 | 257-264 |
| PBP2a-TP_VH3 | NB002 | 265-272 |
| PBP2a-TP_V_{H}H5 | NB003 | 273-280 |
| PBP2a-TP_VH7 | NB004 | 281-288 |
| PBP2a-TP_V_{H}H8 | NB005 | 289-296 |
| PBP2a-TP_V_{H}H11 | NB006 | 297-304 |
| PBP2a-TP_VH12 | NB007 | 305-312 |
| PBP2a-TP_VH14 | NB008 | 313-320 |
| PBP2a-TP_V_{H}H15 | NB009 | 321-328 |
| PBP2a-TP_VH16 | NB010 | 329-336 |
| PBP2a-TP_VH19 | NB011 | 337-344 |
| PBP2a-TP_VH21 | NB012 | 345-352 |
| PBP2a-TP_VH22 | NB013 | 353-360 |
| PBP2a-TP_VH26 | NB014 | 361-368 |
| PBP2a-TP_VH29 | NB015 | 369-376 |
| PBP2a-TP_VH30 | NB016 | 377-384 |
| PBP2a-TP_VH31 | NB017 | 385-392 |

### REFERENCES

The references are:
Abhinandan, K.R. & A.C.R. Martin, (2008) Analysis and improvements to Kabat and structurally correct numbering of antibody variable domains. Mol Immunol 45: 3832-3839.
Borden, P. & E.A. Kabat, (1987) Nucleotide-Sequence of the Cdnas Encoding the Variable Region Heavy and Light-Chains of a Myeloma Protein-Specific for the Terminal Nonreducing End of Alpha-(1-]6)Dextran. P Natl Acad Sci USA 84: 2440-2443.
Carrillo, H. & D. Lipman, (1988) The Multiple Sequence Alignment Problem in Biology. Siam J Appl Math 48: 1073-1082.
Chothia, C. & A.M. Lesk, (1987) Canonical structures for the hypervariable regions of immunoglobulins. JMol Biol 196: 901-917.
Clackson, T., H.R. Hoogenboom, A.D. Griffiths & G. Winter, (1991) Making Antibody Fragments Using Phage Display Libraries. Nature 352: 624-628.
de Kruif, J., E. Boel & T. Logtenberg, (1995) Selection and application of human single chain Fv antibody fragments from a semi-synthetic phage antibody display library with designed CDR3 regions. J Mol Biol 248: 97-105.
Desmet, J., K. Verstraete, Y. Bloch, E. Lorent, Y. Wen, B. Devreese, K. Vandenbroucke, S. Loverix, T. Hettmann, S. Deroo, K. Somers, P. Henderikx, I. Lasters & S.N. Savvides, (2014) Structural basis of IL-23 antagonism by an Alphabody protein scaffold. Nat Commun 5: 5237.
Diem, M.D., L. Hyun, F. Yi, R. Hippensteel, E. Kuhar, C. Lowenstein, E.J. Swift, K.T. O'Neil & S.A. Jacobs, (2014) Selection of high-affinity Centyrin FN3 domains from a simple library diversified at a combination of strand and loop positions. Protein Eng Des Sel 27: 419-429.
Ebersbach, H., E. Fiedler, T. Scheuermann, M. Fiedler, M.T. Stubbs, C. Reimann, G. Proetzel, R. Rudolph & U. Fiedler, (2007) Affilin-novel binding molecules based on human gamma-B-crystallin, an all beta-sheet protein. J Mol Biol 372: 172-185.
Gebauer, M. & A. Skerra, (2009) Engineered protein scaffolds as next-generation antibody therapeutics. Curr Opin Chem Biol 13: 245-255.
Grabulovski, D., M. Kaspar & D. Neri, (2007) A novel, non-immunogenic Fyn SH3-derived binding protein with tumor vascular targeting properties. J Biol Chem 282: 3196-3204.
Gribskov, M. & J. Devereux, (1993) Sequence analysis primer. Oxford University press, Oxford.
Griffin, A.M. & H.G. Griffin, (1994) Computer Analysis of Sequence Data, Parts I and II. Humana Press, New Jersey.
Heijne, v., (1987) Sequence analysis in molecular biology: Treasure trove or trivial pursuit. Academic Press, New York.
Honegger, A. & A. Pluckthun, (2001) Yet another numbering scheme for immunoglobulin variable domains: an automatic modeling and analysis tool. J Mol Biol 309: 657-670.
Johnson, A., Q. Song, P. Ko Ferrigno, P.R. Bueno & J.J. Davis, (2012) Sensitive affimer and antibody based impedimetric label-free assays for C-reactive protein. Anal Chem 84: 6553-6560.
Kabat, E.A., T.T. Wu, H. Bilofsky, M. Reid-Miller & H. Perry, (1983) Sequences of Proteins of Immunological Interest. National Institutes of Health*.*
Kashmiri, S.V., R. De Pascalis, N.R. Gonzales & J. Schlom, (2005) SDR grafting--a new approach to antibody humanization. Methods 36: 25-34.
Kipriyanow & L. Gall, (2004) MOLECULAR BIOTECHNOLOGY 26: 39-60.
Knappik, A., L.M. Ge, A. Honegger, P. Pack, M. Fischer, G. Wellnhofer, A. Hoess, J. Wolle, A. Pluckthun & B. Virnekas, (2000) Fully synthetic human combinatorial antibody libraries (HuCAL) based on modular consensus frameworks and CDRs randomized with trinucleotides. Journal of Molecular Biology 296: 57-86.
Koide, A. & S. Koide, (2007) Monobodies: antibody mimics based on the scaffold of the fibronectin type III domain. Methods Mol Biol 352: 95-109.
Krehenbrink, M., M. Chami, I. Guilvout, P.M. Alzari, F. Pecorari & A.P. Pugsley, (2008) Artificial binding proteins (Affitins) as probes for conformational changes in secretin PulD. J Mol Biol 383: 1058-1068.
Kulkeaw, K., Y. Sakolvaree, P. Srimanote, P. Tongtawe, S. Maneewatch, N. Sookrung, A. Tunytrongchitr, P. Tapchaisri, H. Kurazono & W. Chaicumpa, (2009) Human monoclonal ScFv neutralize lethal Thai cobra, Naja kaouthia, neurotoxin. J Proteomics 72: 270-282.
Lefranc, M.P., (2003) IMGT, the international ImMunoGeneTics information system, Novartis Found Symp 254: 126-136; discussion 136-142, 216-122, 250-122.
Lesk, A.M. & A. Tramontano, (1989) The Computational Analysis of Protein Structures: Sources, Methods, Systems and Results. J Res Natl Inst Stand Technol 94: 85-92.
Mahasenan, K.V., M. Bastian, M. Gao, E. Frost, D.R. Ding, K. Zorina-Lichtenwalter, J. Jacobs, M.A. Suckow, V.A. Schroeder, W.R. Wolter, M. Chang & S. Mobashery, (2017) Exploitation of Conformational Dynamics in imparting Selective Inhibition for Related Matrix Metalloproteinases. Acs Med Chem Lett 8: 654-659.
Marks, J.D., H.R. Hoogenboom, T.P. Bonnert, J. Mccafferty, A.D. Griffiths & G. Winter, (1991) By-Passing Immunization - Human-Antibodies from V-Gene Libraries Displayed on Phage. Journal of Molecular Biology 222: 581-597.
Mendez, M.J., L.L. Green, J.R. Corvalan, X.C. Jia, C.E. Maynard-Currie, X.D. Yang, M.L. Gallo, D.M. Louie, D.V. Lee, K.L. Erickson, J. Luna, C.M. Roy, H. Abderrahim, F. Kirschenbaum, M. Noguchi, D.H. Smith, A. Fukushima, J.F. Hales, S. Klapholz, M.H. Finer, C.G. Davis, K.M. Zsebo & A. Jakobovits, (1997) Functional transplant of megabase human immunoglobulin loci recapitulates human antibody response in mice. Nat Genet 15: 146-156.
Mullinax, R.L., E.A. Gross, J.R. Amberg, B.N. Hay, H.H. Hogrefe, M.M. Kubitz, A. Greener, M. Altingmees, D. Ardourel, J.M. Short, J.A. Sorge & B. Shopes, (1990) Identification of Human-Antibody Fragment Clones Specific for Tetanus Toxoid in a Bacteriophage-Lambda Immunoexpression Library. P Natl Acad Sci USA 87: 8095-8099.
Nascimento, T.C., V.L. da Silva, A.B. Ferreira-Machado & C.G. Diniz, (2015) Potential spread of multidrug-resistant coagulase-negative staphylococci through healthcare waste. J Infect Dev Ctries 9: 29-34.
Nixon, A.E. & C.R. Wood, (2006) Engineered protein inhibitors of proteases. Curr Opin Drug Disc 9: 261-268.
Nygren, P.A., (2008) Alternative binding proteins: affibody binding proteins developed from a small three-helix bundle scaffold. FEBS J 275: 2668-2676.
Ohwada, A., M. Sekiya, H. Hanaki, K.K. Arai, I. Nagaoka, S. Hori, S. Tominaga, K. Hiramatsu & Y. Fukuchi, (1999) DNA vaccination by mecA sequence evokes an antibacterial immune response against methicillin-resistant Staphylococcus aureus. J Antimicrob Chemother 44: 767-774.
Petinaki, E., A. Arvaniti, G. Dimitracopoulos & I. Spiliopoulou, (2001) Detection of mecA, mecR1 and mecI genes among clinical isolates of methicillin-resistant staphylococci by combined polymerase chain reactions. J Antimicrob Chemother 47: 297-304.
Roth, D.M., J.P. Senna & D.C. Machado, (2006) Evaluation of the humoral immune response in BALB/c mice immunized with a naked DNA vaccine anti-methicillin-resistant Staphylococcus aureus. Genet Mol Res 5: 503-512.
Senna, J.P., D.M. Roth, J.S. Oliveira, D.C. Machado & D.S. Santos, (2003) Protective immune response against methicillin resistant Staphylococcus aureus in a murine model using a DNA vaccine approach. Vaccine 21: 2661-2666.
Silverman, J., Q. Liu, A. Bakker, W. To, A. Duguay, B.M. Alba, R. Smith, A. Rivas, P. Li, H. Le, E. Whitehorn, K.W. Moore, C. Swimmer, V. Perlroth, M. Vogt, J. Kolkman & W.P. Stemmer, (2005) Multivalent avimer proteins evolved by exon shuffling of a family of human receptor domains. Nat Biotechnol 23: 1556-1561.
Skerra, A., (2008) Alternative binding proteins: anticalins - harnessing the structural plasticity of the lipocalin ligand pocket to engineer novel binding activities. FEBS J 275: 2677-2683.
Smith, D.W., (1994) Biocomputing: Informatics and Genome Projects. Academic Press Inc, New York.
Stumpp, M.T., H.K. Binz & P. Amstutz, (2008) DARPins: a new generation of protein therapeutics. Drug Discov Today 13: 695-701.
Thanongsaksrikul, J., P. Srimanote, S. Maneewatch, K. Choowongkomon, P. Tapchaisri, S. Makino, H. Kurazono & W. Chaicumpa, (2010) A VHH That Neutralizes the Zinc Metalloproteinase Activity of Botulinum Neurotoxin Type A. J Biol Chem 285: 9657-9666.
Weidle, U.H., D. Maisel, U. Brinkmann & G. Tiefenthaler, (2013) The Translational Potential for Target Validation and Therapy Using Intracellular Antibodies in Oncology. Cancer Genom Proteom 10: 239-250.

## Claims

1. **A protein binding compound (PBC)** specifically binding to the transpeptidase domain of penicillin binding protein 2 a (PBP2a), wherein the PBC when bound to the transpeptidase domain of PBP2A increases sensitivity of PBP2a towards a β-lactam antibiotic compared to when the PBC is not bound to the transpeptidase domain of PBP2a.

2. The PBC of claim 1, wherein the transpeptidase domain of PBP2a is located between amino acids 250 and 668 of *Staphylococcus aureus* PBP2a, or is located in a homologous region of a homologous PBP2a protein.

3. The PBC of claim 1 or 2, wherein the β-lactam antibiotic is a penicillin or penicillin derivative, such as methicillin, amoxicillin, amoxicillin/clavulanate, ampicillin, ampicillin/sulbactam, bacampicillin, carbenicillin, cloxacillin, dicloxacillin, methicillin, mezlocillin, nafcillin, oxacillin, penicillin G, penicillin V, piperacillin, piperacillin/tazobactam, ticarcillin, ticarcillin/clavulanate.

4. The PBC of any one of claims 1 to 3, wherein the binding between the PBC and the transpeptidase domain of PBP2a involves a covalent or non-covalent interaction between the PBC and one of the following amino acids of *Staphylococcus aureus* PBP2A: K334, N338, N339, K341, N377, T384, E385, D386, K387, K388, E389, E391, N415, N416, K417, G440, Y441, Y444, Y446, K477, K484, K485, Y499, Q502, N505, N510, I512, L513, D516, G522, E523, L525, W558,3 K559, K560, N561, K584, E585, D586, K589, E602, L603, K604, G611, R612, K634, K639, Y644, K651, D654, E655, E658, N659, and/or E668, or an PBP2a amino acid located in 5 or less amino acid positions in N- or C-terminal direction.

5. **A protein binding compound (PBC),** which is an isolated PBC comprising at least one Complementary Determining Region (CDR) 3 having an amino acid sequence with at least 80% sequence identity to an amino acid sequence selected from SEQ ID Nos 6, 14, 22, 30, 38, 46, 54, 62, 70, 78, 86, 94,102, 110, 118, 126, 134, 142, 150, 158, 166, 174, 182, 190, 198, 206, 214, 222, 230, 238, 246, 254, 262, 270, 278, 286, 294, 302, 310, 318, 326, 334, 342, 350, 358, 366, 374, 382 and 390.

6. The isolated PBC of claim 5, which (i) comprises an antibody heavy chain variable region, or an antigen binding fragment thereof, and an antibody light chain variable region, or an antigen binding fragment thereof, or is (ii) a nanobody comprising an antibody heavy chain variable region, or an antigen binding fragment thereof, and not comprising an antibody light chain variable region, or an antigen binding fragment thereof.

7. The isolated PBC of any one of the preceding claims, wherein in (i) the PBC is an antibody, or an antigen binding fragment thereof, composed of at least one antibody heavy chain sequences, and at least one antibody light chain sequences, wherein at least one of said antibody heavy chain sequences and antibody light chain sequences comprise CDR1 to CDR3 sequences in the following combination:
| | **Heavy Chain CDR1 to CDR3 (SEQ ID NO)** | | | **Light Chain CDR1 to CDR3 (SEQ ID NO)** | | |
|---|---|---|---|---|---|---|
| **AB001** | 2 | 4 | 6 | 10 | 12 | 14 |
| **AB002** | 18 | 20 | 22 | 26 | 28 | 30 |
| **AB003** | 34 | 36 | 38 | 42 | 44 | 46 |
| **AB004** | 50 | 52 | 54 | 58 | 60 | 62 |
| **AB005** | 66 | 68 | 70 | 74 | 76 | 78 |
| **AB006** | 82 | 84 | 86 | 90 | 92 | 94 |
| **AB007** | 98 | 100 | 102 | 106 | 108 | 110 |
| **AB008** | 114 | 116 | 118 | 122 | 124 | 126 |
| **AB009** | 130 | 132 | 134 | 138 | 140 | 142 |
| **AB010** | 146 | 148 | 150 | 154 | 156 | 158 |
| **AB011** | 162 | 164 | 166 | 170 | 172 | 174 |
| **AB012** | 178 | 180 | 182 | 186 | 188 | 190 |
| **AB013** | 194 | 196 | 198 | 202 | 204 | 206 |
| **AB014** | 210 | 212 | 214 | 218 | 220 | 222 |
| **AB015** | 226 | 228 | 230 | 234 | 236 | 238 |
| **AB016** | 242 | 244 | 246 | 250 | 252 | 254 |
in each case independently, optionally with not more than three or two, preferably one, amino acid substitution(s), insertion(s) or deletion(s) compared to these sequences.

8. The isolated PBC of claim 6 or 7, wherein in (ii) the isolated PBC comprises a nanobody heavy chain sequence, or an antigen binding fragment thereof; wherein the nanobody heavy chain sequence, or the fragment thereof, comprises a CDR3 having at least 80% sequence identity to an amino acid sequence selected from SEQ ID Nos. 262, 270, 278, 286, 294, 302, 310, 318, 326, 334, 342, 350, 358, 366, 374, 382, and 390.

9. The isolated PBC of claim 8, wherein the PBC is a nanobody, or an antigen binding fragment thereof, composed of an antibody heavy chain sequence comprising CDR1 to CDR3 sequences in the following combination:
| | **Heavy Chain CDR1 to CDR3 (SEQ ID NO)** | | |
|---|---|---|---|
| **NB001** | 258 | 260 | 262 |
| **NB002** | 266 | 268 | 270 |
| **NB003** | 274 | 276 | 278 |
| **NB004** | 282 | 284 | 286 |
| **NB005** | 290 | 292 | 294 |
| **NB006** | 298 | 300 | 302 |
| **NB007** | 306 | 308 | 310 |
| **NB008** | 314 | 316 | 318 |
| **NB009** | 322 | 324 | 326 |
| **NB010** | 330 | 332 | 334 |
| **NB011** | 338 | 340 | 342 |
| **NB012** | 346 | 348 | 350 |
| **NB013** | 354 | 356 | 358 |
| **NB014** | 362 | 364 | 366 |
| **NB015** | 370 | 372 | 374 |
| **NB016** | 378 | 380 | 382 |
| **NB017** | 386 | 388 | 390 |
in each case independently, optionally with not more than three or two, preferably one, amino acid substitution(s), insertion(s) or deletion(s) compared to these sequences.

10. **An isolated PBC,** which competes with an PBC as recited in any one of claims 5 to 9 for binding to the transpeptidase domain of PBP2a, preferably of *Staphylococcus aureus,* and is capable of increasing sensitivity of said PBP2a to a β-lactam antibiotic, preferably capable of increasing of the binding of the β-lactam antibiotic to the PBP2a.

11. The PBC of any one of the preceding claims, that enhances β-lactam antibiotic induced killing, lysing, or reducing the growth, of a bacterial cell expressing PBP2a.

12. **An isolated nucleic acid** encoding for a PBC which is a protein or polypeptide according to any one of the preceding claims.

13. **A recombinant host cell,** comprising a PBC or an isolated nucleic acid according to any one of the preceding claims.

14. **A pharmaceutical composition** comprising a PBC recited in any of claims 1 to 11, an isolated nucleic acid recited in claim 12, a recombinant host cell recited in claim 13, together with a pharmaceutically acceptable carrier and/or excipient.

15. **A product for use in medicine,** wherein the product is selected from a PBC recited in any of claims 1 to 11, an isolated nucleic acid recited in claim 12, a recombinant host cell recited in claim 13, and a pharmaceutical composition of claim 14.

16. **An in-vitro method for identifying and/or characterising a compound suitable for the treatment of a disease,** disorder or condition that is associated with, or caused by, a bacterial cell expressing PBP2a, or a variant thereof, and/or that is **characterised by** antibiotic resistance against a treatment with a β-lactam antibiotic, the method comprising the steps of:
**(a)** bringing into contact a candidate compound and a protein of PBP2a (or a variant thereof), or a protein-fragment of PBP2a comprising, or consisting essentially of, a transpeptidase domain of PBP2a (or of the variant thereof); and
**(b)** detecting and/or quantifying a binding of the candidate compound to the transpeptidase domain of the protein of PBP2a (or the variant thereof), or the protein-fragment of PBP2a (or of the variant thereof),
wherein a binding of the candidate compound to the transpeptidase domain of PBP2a (or the variant thereof) compared to a control indicates that the compound is suitable for the treatment of a disease, disorder or condition that is associated with, or caused by, a bacterial cell expressing PBP2a, or a variant thereof, and/or that is **characterised by** antibiotic resistance against a treatment with a β-lactam antibiotic.

17. **An in-vitro method for identifying and/or characterising a compound suitable for the treatment of a disease**, disorder or condition that is associated with, or caused by, a bacterial cell expressing PBP2a, or a variant thereof, and/or that is **characterised by** antibiotic resistance against a treatment with a β-lactam antibiotic, the method comprising the steps of:
**(a)** bringing into contact a candidate compound and a bacterial cell expressing a protein of PBP2a (or a variant thereof), or a protein-fragment of PBP2a comprising, or consisting essentially of, a transpeptidase domain of PBP2a (or of the variant thereof) an; and
**(b)** determining the growth or viability of the bacterial cell of (a) in response to a treatment with a β-lactam antibiotic,
wherein a reduced growth or viability of the bacterial cell compared to a control indicates that the compound is suitable for the treatment of a disease, disorder or condition that is associated with, or caused by, a bacterial cell expressing PBP2a, or a variant thereof, and/or that is **characterised by** antibiotic resistance against a treatment with a β-lactam antibiotic.

18. **An *in-vitro* diagnostic method for determining** whether a subject has, or is at risk of, developing a disease, disorder or condition that is associated with the undesired presence of PBP2a-positive cells (or cells positive for a variant of PBP2a) and/or that is **characterized by** a resistance to a treatment with a β-lactam antibiotic, the method comprising the step of:
**(i)** detecting PBP2a (or a variant thereof), in particular the presence (or an amount) of or expression and/or activity of PBP2a (or a variant thereof), in a biological sample of the subject, for example a sample suspected to comprising bacterial cells of the disease, with a PBC of any one of claims 1 to 11,
wherein the detection of PBP2a (or the variant thereof) in the sample indicates such disease, disorder or condition, or a risk of developing such disease, disorder or condition, in the subject.
